(19) Europäisches Patentamt
European Patent Office
Office européen des brevets

(11) **EP 3 455 633 B1**

(12) **FASCICULE DE BREVET EUROPEEN**

(45) Date de publication et mention
de la délivrance du brevet:
**12.08.2020 Bulletin 2020/33**

(51) Int Cl.:
***G01N 33/574*** *(2006.01)* ***C12M 3/00*** *(2006.01)*
***C12N 5/00*** *(2006.01)*

(21) Numéro de dépôt: **17727302.6**

(22) Date de dépôt: **11.05.2017**

(86) Numéro de dépôt international:
**PCT/FR2017/051149**

(87) Numéro de publication internationale:
**WO 2017/194895 (16.11.2017 Gazette 2017/46)**

(54) **METHODE DE DIAGNOSTIC DE CANCERS UROLOGIQUES**

VERFAHREN ZUR DIAGNOSE VON UROGENITALKREBS

METHOD FOR DIAGNOSING GENITOURINARY CANCERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **11.05.2016 FR 1654215**

(43) Date de publication de la demande:
**20.03.2019 Bulletin 2019/12**

(73) Titulaires:
• **Université Grenoble Alpes
38401 Saint-Martin-d'Hères (FR)**
• **Commissariat à l'Énergie Atomique
et aux Énergies Alternatives
75015 Paris (FR)**

(72) Inventeurs:
• **MARTIN, Donald, Keith
38610 Gieres (FR)**
• **PICOLLET-D'HAHAN, Nathalie
38580 La Ferrière (FR)**

(74) Mandataire: **Bronchart, Quentin
Cabinet Hautier
20, rue de la Liberté
06000 Nice (FR)**

(56) Documents cités:
• **R T BRYAN ET AL: "Protein shedding in urothelial bladder cancer: prognostic implications of soluble urinary EGFR and EpCAM", BRITISH JOURNAL OF CANCER, vol. 112, no. 6, 17 mars 2015 (2015-03-17) , pages 1052-1058, XP055323353, GB ISSN: 0007-0920, DOI: 10.1038/bjc.2015.21**
• **MANOUSOS MAKRIDAKIS ET AL: "Analysis of Secreted Proteins for the Study of Bladder Cancer Cell Aggressiveness", JOURNAL OF PROTEOME RESEARCH., vol. 9, no. 6, 4 juin 2010 (2010-06-04), pages 3243-3259, XP055323354, US ISSN: 1535-3893, DOI: 10.1021/pr100189d**
• **N J SHIMWELL ET AL: "Combined proteome and transcriptome analyses for the discovery of urinary biomarkers for urothelial carcinoma", BRITISH JOURNAL OF CANCER, vol. 108, no. 9, 14 mars 2013 (2013-03-14) , pages 1854-1861, XP055248692, GB ISSN: 0007-0920, DOI: 10.1038/bjc.2013.157**
• **ELIZABETH KAVALER ET AL: "Detecting human bladder carcinoma cells in voided urine samples by assaying for the presence of telomerase activity", CANCER., vol. 82, no. 4, 15 février 1998 (1998-02-15), pages 708-714, XP055323358, US ISSN: 0008-543X, DOI: 10.1002/(SICI)1097-0142(19980215)82:4<708: :AID-CNCR14>3.0.CO;2-1**
• **PICOLLET-D'HAHAN NATHALIE ET AL: "A 3D Toolbox to Enhance Physiological Relevance of Human Tissue Models", TRENDS IN BIOTECHNOLOGY, ELSEVIER PUBLICATIONS, CAMBRIDGE, GB, vol. 34, no. 9, 4 août 2016 (2016-08-04), pages 757-769, XP029689296, ISSN: 0167-7799, DOI: 10.1016/J.TIBTECH.2016.06.012**

**Description**

La présente invention concerne une méthode de diagnostic *in vitro* d'un cancer urologique à partir de l'analyse du sécrétome provenant de cellules isolées de l'urine du patient.

De nombreux dispositifs actuels développés dans le but de mimer des organes sur une puce, sont constitués de membranes en 2D.

Dans le brevet US 8 647 861 B2 de Ingber et al., déposé le 13 Octobre 2011, les auteurs ont mis au point un dispositif organomimétique permettant une co-culture de cellules adhérentes de part et d'autre d'une membrane, doté de moyens permettent de créer une pression différentielle aux abords de la membrane provoquant ainsi l'expansion ou la rétraction de celle-ci, afin de reproduire les contraintes mécaniques existant in vivo à une interface tissu-tissu. Ce dispositif, basé sur un système microfluidique comprenant une membrane poreuse flexible en polydiméthylsiloxane (PDMS), à l'interface de deux canaux microfluidiques permettant la circulation de fluides le long de ladite membrane, permet notamment de mimer les alvéoles pulmonaires pendant la respiration.

Dans la demande US 2014/0038279 A1 de Ingber et al., déposée le 28 février 2012, les auteurs proposent un système de co-culture cellulaire permettant reproduire les structures épithéliales intestinales naturelles et mimer leur comportement. Ce dispositif organomimétique, basé sur un système microfluidique, comporte une membrane poreuse en PDMS, positionnée à l'interface de deux canaux microfluidiques permettant la circulation de fluides le long de ladite membrane, et sur laquelle est cultivée une couche de cellules épithéliales intestinales sur au moins l'un des côtés. Le couplage de la membrane à des éléments de support entraine un mouvement d'étirement de la membrane dans au moins une dimension, et permet ainsi de reproduire le mouvement des tissus *in vivo.*

Même si ces dispositifs permettent de recréer des interfaces tissu-tissu ainsi que les contraintes mécaniques existant *in vivo*, pour mimer la microarchitecture des organes, la co-culture cellulaire demeure sur un milieu en 2D, et ne permettent pas de reproduire la topographie de certains organes.

En 2013, Kim et al., dans leur publication intitulée « Gut-on-a-Chip microenvironment induces human intestinal cells to undergo villus différenciation », Integr. Biol., 5, 1130, proposent une puce microfluidique organomimétiques permettant ainsi de reproduire les villosités intestinales en 3D, à l'image de celles formées *in vivo* dans l'intestin. Cette puce, basée sur un système microfluidique, comporte une membrane poreuse en PDMS, positionnée à l'interface de deux canaux microfluidiques permettant la circulation de fluides le long de ladite membrane, et sur laquelle sont cultivées des cellules épithéliales intestinales Caco-2 qui forment spontanément des villosités au-delà de deux jours de culture en présence d'un flux constant de milieu de 30 µL/h.

Tous ces dispositifs proposent cependant une co-culture cellulaire sur une membrane poreuse en PDMS, qui n'est pas optimale en termes de biocompatibilité (inertie, porosité). Le PDMS peut absorber des petits composants organiques, aussi des drogues et a une perméabilité aux gaz pouvant gêner certaines applications. Par ailleurs, les membranes en PDMS peuvent avoir des propriétés de transport, mécaniques et structurales intrinsèques qui sont différentes de celles la membrane basale naturelle du tissu. En recréant ce type d'interfaces, les auteurs privilégient la reproduction de la microarchitecture de l'organe.

En 2014, March et al., dans leur publication intitulé « Differentiating Intestinal Stem Cells in a 3D Niche », proposent un modèle d'intestin *in vitro* utilisant des structures en acide glycolique poly-lactique (PLGA) ayant la morphologie 3D des microvillosités et aptes à supporter une co-culture de différents types de cellules épithéliales à leur surface, telles qu'une co-culture de cellules épithéliales intestinales Caco-2 et HT29-MTX. Ce modèle permet d'obtenir une différenciation cellulaire à la surface des microvillosités d'une manière similaire aux intestins in vivo.

Ainsi, même si certains dispositifs actuels présentent des structures, en PDMS ou en PGLA, permettant de mimer les microvillosités des organes, ces structures sont pleines, c'est-à-dire non évidées, et ne permettent donc pas la collecte d'effluents ou de biofluides sécrétés par les cellules en co-culture sur ces structures. Dans le cas de ces structures pleines, la détection de marqueurs connus se fait par immunofluorescence après fixation des cellules et marquage à l'aide d'un anticorps, ce qui implique donc la lyse des cellules pour l'analyse.

De nombreuses publications font état que des cellules en culture sur des structures 3D n'ont pas les mêmes propriétés que les mêmes cellules cultivées en 2D ; des différences sont en effet observées dans le profil d'expression de gènes et de protéines, l'adhésion des cellules, la vitesse de prolifération et la différenciation cellulaire. De plus, la réponse métabolique aux drogues d'un type cellulaire est différente selon que les cellules sont cultivées en 2D et en 3D, comme l'ont montré Kim et al. en 2013 dans leur publication intitulée « Gut-on-a-Chip microenvironment induces human intestinal cells to undergo villus différenciation », Integr. Biol., 5, 1130, où ils réalisent une comparaison, entre le modèle de culture 2D Transwell® et un modèle de culture comportant des structures 3D mimant les microvillosités, de l'activité de l'enzyme cytochrome P450 métabolisant les drogues, et montrent qu'en 2D l'activité reste inchangée au cours de la culture, tandis qu'en 3D cette activité augmente lors de la formation des microvillosités puis se stabilise.

La topographie du milieu cellulaire ayant un impact non négligeable sur le comportement des cellules, il est donc important de reproduire le plus précisément possible les conditions in vivo des cellules, afin de mettre au point des modèles fiables de culture cellulaire.

Le document R. T. Bryan, et al., Protein shedding in urothelial bladder cancer: prognostic implications of soluble urinary EGFR and EpCAM, Br J Cancer, 2015, 112(6), 1052-1058, divulgue une méthode de diagnostic de cancer de la vessie, dans laquelle des biomarqueurs sont identifiés par une étude protéomique de sécrétions de lignées cellulaires caractéristique du carcinome urothélial de la vessie. La présence de ces biomarqueurs est ensuite testée dans des échantillons d'urine de patients à l'aide d'un anticorps, par test ELISA.

La présente invention concerne une méthode de diagnostic *in vitro* d'un cancer urologique comprenant :

- la récupération des sécrétions produites par les cellules isolées à partir d'un échantillon d'urine d'un patient à diagnostiquer,
- l'analyse des composants desdites sécrétions, dit sécrétome, produites par lesdites cellules isolées à partir d'un échantillon d'urine dudit patient à diagnostiquer ;
- la comparaison d'un sécrétome de cellules isolées à partir d'un échantillon d'urine d'un patient à diagnostiquer par rapport :

  - soit à un sécrétome de référence obtenu à partir de sécrétions de cellules saines isolées d'un échantillon d'urine d'une personne saine,

  - soit à un sécrétome de référence obtenu à partir de sécrétions de cellules saines dérivées de cultures de lignées cellulaires standard caractéristiques d'un organe urologique déterminé,

  ledit sécrétome et ledit sécrétome de référence étant constitués de l'ensemble des composants formant leurs sécrétions respectives,
- la détermination de la présence d'un cancer urologique lorsqu'au moins une différence d'un composant est déterminée entre ledit sécrétome de cellules isolées à partir d'un échantillon d'urine dudit patient à diagnostiquer et ledit sécrétome de référence.

Selon un mode de réalisation particulier, l'invention concerne une méthode de diagnostic *in vitro* d'un cancer urologique, dans laquelle la comparaison d'un sécrétome de cellules isolées à partir d'un échantillon d'urine d'un patient à diagnostiquer est réalisée par rapport à un sécrétome de référence obtenu à partir de sécrétions de cellules saines isolées d'un échantillon d'urine d'une personne saine, ledit sécrétome et ledit sécrétome de référence étant constitués de l'ensemble des composants formant leurs sécrétions respectives.

Selon un mode de réalisation particulier, l'invention concerne une méthode de diagnostic *in vitro* d'un cancer urologique, dans laquelle la comparaison d'un sécrétome de cellules isolées à partir d'un échantillon d'urine d'un patient à diagnostiquer est réalisée par rapport à un ensemble de trois sécrétomes de référence obtenus respectivement à partir des sécrétions de lignées cellulaires standard de vessie, prostate et reins.

Selon un mode de réalisation particulier, l'invention concerne méthode de diagnostic *in vitro* d'un cancer urologique, dans laquelle la comparaison d'un sécrétome de cellules isolées à partir d'un échantillon d'urine d'un patient à diagnostiquer est réalisée par rapport :

- à un sécrétome de référence obtenu à partir de sécrétions de cellules saines isolées d'un échantillon d'urine d'une personne saine, et

- à un ensemble de trois sécrétomes de référence obtenus respectivement à partir des sécrétions de lignées cellulaires standard de vessie, prostate et reins,

ledit sécrétome et ledit sécrétome de référence étant constitués de l'ensemble des composants formant leurs sécrétions respectives.

Le sécrétome correspond à l'ensemble des composants présents dans les sécrétions tels que les peptides, les protéines, les acides aminés, les miARN, l'ADN et l'ARN. Ce terme englobe à la fois l'aspect qualitatif et quantitatif des composants.

Les cellules permettant d'obtenir un sécrétome de référence peuvent être des lignées cellulaires standard dérivées de chacun des organes urologiques tels que les reins, la prostate et la vessie.

Il est également possible d'utiliser des cellules isolées de l'urine d'un patient sain.

De manière générale, les cellules isolées à partir de l'urine d'un patient, sain ou pas, sont des cellules uroépithéliales (ou urothéliales) exfoliées, incluant à la fois les cellules épithéliales de vessie, de prostate et de rein.

Ainsi, le sécrétome de référence obtenu à partir de cellules isolées de l'urine de patient sain contient des sécrétions provenant à la fois des reins, de la prostate et de la vessie. Par conséquent, un tel sécrétome est une combinaison des sécrétomes de chacun de ces types cellulaires présent dans l'urine.

Autrement dit, il résulte de l'utilisation de cellules isolées de l'urine d'un patient sain pour l'obtention du sécrétome, que le sécrétome de référence obtenu est une combinaison des sécrétomes individuels du rein, de la prostate et de la vessie.

Dans un mode de réalisation particulier, le sécrétome de référence est obtenu préférablement à partir de lignées cellulaires de reins, prostate ou vessie, soit de lignées cellulaires dont l'organe de provenance et les caractéristiques sont bien connues afin de diagnostiquer un éventuel cancer urologique et de pouvoir identifier l'organe atteint.

Le sécrétome du patient à diagnostiquer étant obtenu à partir de cellules isolées d'un échantillon d'urine dudit patient, il en résulte que le sécrétome obtenu pour les cellules du patient à diagnostiquer est une combinaison des sécrétomes individuels du rein, de la prostate et de la vessie ; les cellules isolées à partir de l'urine incluant à la fois les cellules épithéliales de vessie, de prostate et de rein.

Le profil des patients sains pour l'obtention des sécrétomes de référence sont avantageusement des jeunes gens d'environ 20 ans n'ayant aucun antécédent familial de cancer urothélial.

Deux groupes distincts doivent être fait entre les hommes et les femmes.

Il est possible soit de combiner plusieurs échantillons d'urine provenant de patients sains afin d'obtenir un sécrétome moyen de référence, soit d'obtenir des sécrétomes de référence à partir d'échantillons d'urine provenant respectivement de patients sains individuels.

L'avantage de cette dernière méthode est d'obtenir des statistiques concernant la variation de du sécrétome à partir d'une population de gens sains.

Selon un mode de réalisation avantageux, les cellules permettant d'obtenir un sécrétome de référence sont des lignées cellulaires standard dérivées de chacun des organes urologiques tels que les reins, la prostate et la vessie.

Selon un mode de réalisation particulier, l'invention concerne une méthode de diagnostic *in vitro* d'un cancer urologique, dans laquelle la comparaison d'un sécrétome de cellules isolées à partir d'un échantillon d'urine d'un patient à diagnostiquer par rapport à un sécrétome de référence, est réalisée sans séparation préalable des composants desdits sécrétomes.

Les cellules isolées à partir d'un échantillon d'urine du patient à diagnostiquer ne sont pas préalablement séparées par type cellulaire (vessie, prostate ou rein). Ainsi, le sécrétome obtenu à partir des cellules du patient à diagnostiquer correspond à la combinaison des sécrétomes de chacun de ces types cellulaires présent dans l'urine.

L'invention concerne une méthode de diagnostic *in vitro* d'un cancer urologique, dans laquelle la détermination d'au moins une différence d'un composant entre le sécrétome de cellules isolées est déterminée à partir d'un échantillon d'urine d'un patient à diagnostiquer et le sécrétome de référence.

Selon un mode de réalisation particulier, l'invention concerne une méthode de diagnostic *in vitro* d'un cancer urologique, dans laquelle ladite au moins une différence d'un composant déterminée entre le sécrétome de cellules isolées à partir d'un échantillon d'urine du patient à diagnostiquer et le sécrétome de référence, correspond

- soit la présence d'un composant en plus forte ou en plus faible concentration,
- soit à l'absence d'un composant, dans le sécrétome de cellules isolées à partir d'un échantillon d'urine dudit patient à diagnostiquer, par rapport audit composant dans le sécrétome de référence ;
- soit à la présence d'un composant dans le sécrétome de cellules isolées à partir d'un échantillon d'urine dudit patient à diagnostiquer, lequel est absent dans le sécrétome de référence.

Le diagnostic du cancer est établi lorsque le sécrétome du patient est différent de celui du sécrétome de référence.

La présence de cancer entraine une variation de la quantité des composants présents dans le sécrétome caractéristique d'une personne saine

Cependant il est également possible que des cellules cancéreuses sécrètent des molécules qui sont des produits de dégradations de composants présents dans le sécrétome caractéristique d'une personne saine.

Dans chacun des cas, le profil du sécrétome (le profil des masses des molécules présentes dans un spectre) donne l'empreinte permettant de distinguer entre le sécrétome d'un patient sain en comparaison avec un patient atteint d'un cancer.

Selon un mode de réalisation particulier, le diagnostic ne nécessite que l'utilisation de l'empreinte globale obtenue à partir de l'analyse du sécrétome.

Il est à noter qu'en ayant recours à certains types d'analyse (spectroscopie de masse) on dispose d'un ensemble de pics correspondant à des molécules spécifiques et qu'il est par conséquent possible d'avoir recours à l'approche classique de la spectroscopie de masse pour analyser les molécules.

L'invention concerne une méthode de diagnostic *in vitro* d'un cancer urologique, dans laquelle ledit patient à diagnostiquer est diagnostiqué comme présentant un cancer urologique lorsqu'au moins une différence d'un composant est observée entre le sécrétome de cellules isolées à partir d'un échantillon d'urine dudit patient à diagnostiquer et le sécrétome de référence.

Selon un mode de réalisation particulier, l'invention concerne une méthode de diagnostic *in vitro* d'un cancer urologique, dans laquelle la sévérité dudit cancer urologique est déterminée par l'amplitude de la au moins une différence observée d'un composant, entre le sécrétome de cellules isolées à partir d'un échantillon d'urine du patient à diagnostiquer et le sécrétome de référence.

L'amplitude de la au moins une différence observée signifie que la quantification de cette différence observée est effectuée.

Selon un mode de réalisation particulier, l'invention concerne une méthode de diagnostic *in vitro* d'un cancer urologique, dans laquelle la localisation dudit cancer urologique est effectuée dans les reins, la vessie ou la prostate par la comparaison du sécrétome de cellules isolées à partir d'un échantillon d'urine du patient à diagnostiquer par rapport aux sécrétomes de référence obtenu séparément à partir de cellules saines de reins, de vessie et de prostate, isolées d'un échantillon d'urine d'une personne saine,

et la détermination d'au moins une différence d'un composant entre le sécrétome de cellules isolées à partir d'un échantillon d'urine du patient à diagnostiquer et au moins un desdits sécrétomes de référence de reins, de vessie ou de prostate.

Selon un mode de réalisation particulier, l'invention concerne une méthode de diagnostic *in vitro* d'un cancer urologique, dans laquelle le sécrétome de référence analysé est obtenu :

- soit à partir de cellules saines isolées d'un échantillon d'urine d'une personne saine,
- soit à partir de lignées cellulaires standard dérivées de reins, de vessie, de prostate,

la méthode de diagnostic comprenant en outre une étape de détermination de la localisation dudit cancer urologique dans la vessie, les reins ou la prostate, selon que la au moins une différence d'un composant est observée par rapport à un sécrétome de référence obtenu pour la vessie, les reins et/ou la prostate.

Il est possible d'observer des différences entre le sécrétome des cellules isolées de l'urine du patient et plusieurs sécrétomes de référence obtenus respectivement à partir de lignées cellulaires standard dérivées de reins, de prostate et de vessie.

Cette information est dérivée de la procédure de l'analyse du signal pour faire une déconvolution des signaux combinés des cellules dérivées du patient en composants séparés relatifs respectivement au rein à la prostate et à la vessie. Les procédures de traitement du signal peuvent fournir des informations quantifiables relatives à la localisation individuelle (rein, prostate ou vessie) qui combinés fournissent le profil du sécrétome dérivé du patient.

Selon un mode de réalisation particulier, l'invention concerne une méthode de diagnostic *in vitro* d'un cancer urologique, dans laquelle l'étape de récupération du sécrétome desdites cellules isolées à partir de l'urine du patient à diagnostiquer comporte les étapes suivantes :

- l'isolement des cellules d'un échantillon d'urine du patient à diagnostiquer par centrifugation ; suivi de
- la culture *in vitro* desdites cellules ; suivi de
- la récupération des sécrétions produites par lesdites cellules.

Selon un mode de réalisation particulier, l'invention concerne une méthode de diagnostic *in vitro* d'un cancer urologique, dans laquelle les cellules isolées sont cultivées sur un milieu de culture en 2D comportant un tapis cellulaire de cellules épithéliales confluentes surmonté d'un tapis cellulaire de fibroblastes confluents.

Le terme « tapis cellulaire » indique que la culture cellulaire est au stade de la confluence et que l'ensemble des cellules forme une couche cellulaire.

Selon un mode de réalisation particulier, l'invention concerne une méthode de diagnostic *in vitro* d'un cancer urologique, dans laquelle ledit milieu de culture en 2D présente au moins un repli de manière à former un milieu de culture en 3D, mimant la structure d'au moins une glande exocrine composée de structure acinaire/canaliculaire et comportant ledit tapis cellulaire de cellules épithéliales surmonté dudit tapis cellulaire de fibroblastes.

Selon un mode de réalisation particulier, l'invention concerne une méthode de diagnostic *in vitro* d'un cancer urologique, dans laquelle lesdites cellules isolées sont cultivées dans une puce microfluidique de culture cellulaire décrite ci-après, permettant de collecter les sécrétions desdites cellules.

Selon un mode de réalisation particulier de l'invention, lorsque le dispositif utilisé pour obtenir le sécrétome est constitué par ladite puce microfluidique de culture cellulaire décrite ci-après, celui-ci est pré-ensemencé avec une culture de cellules de lignées cellulaires sur les faces externes et internes des protubérances, avant l'addition aux cellules du patient provenant d'un échantillon d'urine.

Les cellules isolées de l'urine du patient sont introduites en mélange dans le dispositif, il n'est pas nécessaire de trier les cellules selon leur type cellulaire avant l'introduction dans puce microfluidique de culture cellulaire décrite ci-après.

Le tri est obtenu à partir des procédures d'analyse du signal provenant du profil du sécrétome combiné obtenu de la suspension de cellules complète de cellules dérivées de patients.

Les procédures de gestion de données pour analyser le sécrétome d'un patient peuvent impliquer des techniques de corrélation, des techniques de convolutions et d'autres analyses de signaux pour séparer les signaux composant le sécrétome « de combinaison » obtenu du patient. De telles techniques d'analyse de signaux sont bien connues dans le cadre de signaux complexes.

Selon un mode de réalisation particulier, l'invention concerne une méthode de diagnostic *in vitro* d'un cancer urologique, dans laquelle ladite analyse du sécrétome est réalisée par spectrométrie de masse sans séparation préalable des composants dudit sécrétome, pour obtenir un spectre de masse spécifique du sécrétome des cellules isolées à partir de l'échantillon.

Selon un mode de réalisation particulier, l'invention concerne une méthode de diagnostic *in vitro* d'un cancer urologique, comprenant la comparaison du spectre de masse d'un sécrétome de cellules isolées à partir d'un échantillon d'urine du patient à diagnostiquer par rapport à un spectre de masse d'un sécrétome de référence obtenu à partir de cellules saines isolées d'un échantillon d'urine d'une personne saine.

Selon un mode de réalisation particulier, la méthode de spectrométrie de masse utilisé est le MALDI-TOF.

Selon un mode de réalisation particulier, l'invention concerne une méthode de diagnostic *in vitro* d'un cancer urologique, comprenant la détermination d'au moins une différence d'un composant entre le spectre de masse obtenu pour le sécrétome de cellules isolées à partir d'un échantillon d'urine du patient à diagnostiquer et le spectre de masse du sécrétome de référence.

Selon un mode de réalisation particulier, l'invention concerne une méthode de diagnostic *in vitro* d'un cancer urologique, dans laquelle ledit patient à diagnostiquer est diagnostiquée comme présentant un cancer urologique lorsqu'au moins une différence d'un composant est observée entre le spectre de masse du sécrétome de cellules isolées à partir d'un échantillon d'urine de la personne à diagnostiquer et le spectre de masse du sécrétome de référence.

Selon un autre mode de réalisation de l'invention, il est possible d'observer des différences par rapport à plusieurs spectres de référence obtenus pour chacun des trois organes et ainsi diagnostiquer par exemple un cancer de la vessie, un cancer de la prostate et/ou un cancer des reins.

Selon un mode de réalisation particulier, l'invention concerne une méthode de diagnostic *in vitro* d'un cancer urologique, dans laquelle la sévérité dudit cancer urologique est déterminée par l'amplitude de la au moins une différence observée entre le spectre de masse du sécrétome de cellules isolées à partir d'un échantillon d'urine de la personne à diagnostiquer et le spectre de masse du sécrétome de référence.

Selon un mode de réalisation particulier, l'invention concerne une méthode de diagnostic *in vitro* d'un cancer urologique, dans laquelle la localisation dudit cancer urologique est effectuée dans les reins, la vessie ou la prostate par la comparaison du spectre de masse du sécrétome de cellules isolées à partir d'un échantillon d'urine du patient à diagnostiquer par rapport aux spectres de masse des sécrétomes de référence obtenu séparément à partir de cellules saines de reins, de vessie et de prostate, isolées d'un échantillon d'urine d'une personne saine,

et la détermination d'au moins une différence entre le spectre de masse du sécrétome de cellules isolées à partir d'un échantillon d'urine du patient à diagnostiquer et au moins un desdits sécrétomes de référence de reins, de vessie ou de prostate.

Selon un mode de réalisation particulier, l'invention concerne une méthode de diagnostic *in vitro* d'un cancer urologique, dans laquelle les composants présents dans les sécrétions constituant le sécrétome sont des protéines, des peptides, des acides aminés et des biomarqueurs nucléiques (ADN, ARN, miARN et ARNi).

Selon un mode de réalisation particulier, l'invention concerne une méthode de diagnostic *in vitro* d'un cancer urologique, dans laquelle les composants constituant le sécrétome comprennent les protéines suivantes : PSA, PCA3, KLK15, SPINK1, PRSS3, cathepsine D, Apolipoprotéine A-I, PLK2.

Selon un mode de réalisation particulier, l'invention concerne une méthode de diagnostic *in vitro* d'un cancer urologique, dans laquelle les composants constituant le sécrétome comprennent les biomarqueurs nucléiques suivants : miR-141, miR-375, transcrits de fusion TMPRSS2-ERG, les gènes codant pour SFPR1 et BNC1, les gènes codant pour l'hydrolase Gamma-glutamyl (*GGH*), l'inhibiteur de liaison au diazépam (*DBI*), et le facteur de transcription E2F3.

Selon un mode de réalisation particulier, l'invention concerne une méthode de diagnostic *in vitro* d'un cancer urologique, dans laquelle les composants constituant le sécrétome de cellules saines de prostate isolées à partir d'un échantillon d'urine d'une personne saine, comprennent les protéines suivantes : PSA, PCA3, KLK15, SPINK1 et PRSS3.

Selon un mode de réalisation particulier, l'invention concerne une méthode de diagnostic *in vitro* d'un cancer urologique, dans laquelle les composants constituant le sécrétome de cellules saines de reins isolées à partir d'un échantillon d'urine d'une personne saine, comprennent la protéine cathepsine D.

Selon un mode de réalisation particulier, l'invention concerne une méthode de diagnostic *in vitro* d'un cancer urologique, dans laquelle les composants constituant le sécrétome de cellules saines de vessie isolées à partir d'un échantillon d'urine d'une personne saine, comprennent les protéines suivantes : Apolipoprotéine A-I et PLK2.

Selon un mode de réalisation particulier, l'invention concerne une méthode de diagnostic *in vitro* d'un cancer urologique, dans laquelle les composants constituant le sécrétome de cellules saines de prostate isolées à partir d'un échantillon d'urine d'une personne saine, comprennent l'ARN de fusion TMPRSS2-ERG et les miARN miR-141 et miR-375.

Selon un mode de réalisation particulier, l'invention concerne une méthode de diagnostic *in vitro* d'un cancer urologique, dans laquelle les composants constituant le sécrétome de cellules saines de reins isolées à partir d'un échantillon d'urine d'une personne saine, comprennent les gènes SFPR1 et de BNC1.

Selon un mode de réalisation particulier, l'invention concerne une méthode de diagnostic *in vitro* d'un cancer urologique, dans laquelle les composants constituant le sécrétome de cellules saines de vessie isolées à partir d'un échantillon d'urine d'une personne saine, comprennent les biomarqueurs nucléiques suivants : gènes codant pour l'hydrolase Gamma-glutamyl (*GGH*), l'inhibiteur de liaison au diazépam (*DBI*), et le facteur de transcription E2F3.

Les Inventeurs ont également mis au point une puce microfluidique de culture cellulaire, mimant les microvillosités d'organes avec une structure acinaire/tubullaire et le microenvironnement luminal.

La puce microfluidique comprend une membrane en biomatériaux poreux qui comporte des replis formant ainsi des protubérances creuses en 3D sur lesquelles sont cultivées des cellules adhérentes de part et d'autre de ladite membrane au niveau des protubérances. Le couplage de cette membrane à un système microfluidique permet de récupérer les sécrétions cellulaires au cours de leur culture pour en analyser la composition.

Ainsi, contrairement à l'art antérieur, où les marqueurs sont détectés par un immunomarquage sur cellules fixées impliquant donc la mort des cellules (Kim *et al.,* 2013), la puce permet de réaliser ces détections dans les sécrétions des cellules cultivées sur la protubérance, qui sont récupérées par le système microfluidique.

La puce permet ainsi de collecter des sécrétions sur cellules vivantes et par conséquent de collecter les sécrétions à des temps différents au cours de la culture, voire sur plusieurs jours pour la réalisation d'études cinétiques, tout en maintenant la viabilité et la fonctionnalité des cellules au cours du temps.

Ces sécrétions sont constituées de l'ensemble des molécules pouvant être sécrétées dans le milieu de culture cellulaire tel que les peptides, les protéines, des acides aminés, des miARN, ADN, ARN

Le profil d'analyse de ces sécrétions permet d'obtenir le sécrétome, c'est-à-dire le profil qualitatif et quantitatif des composants des sécrétions.

La méthode de diagnostic *in vitro* d'un cancer urologique peut plus particulièrement être mise en œuvre avec une puce microfluidique de culture cellulaire qui contient un module central (104) comprenant :

- une unité centrale (105), laquelle contient

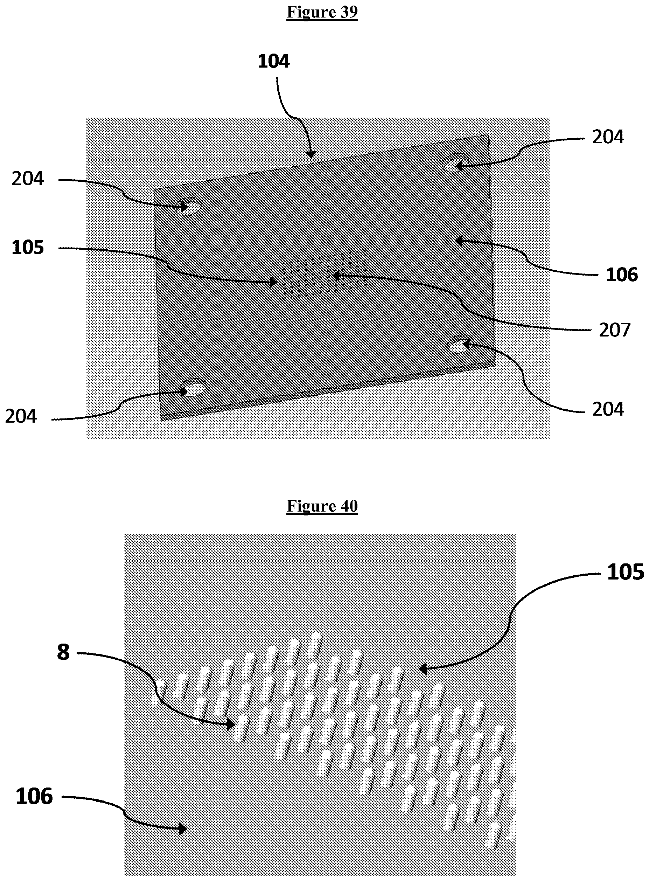

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5) **(Figure 40)**,

  ladite face inférieure (7) de ladite membrane poreuse nanostructurée en 3D (5) étant positionnée de façon solidaire sur ladite face supérieure (2) dudit support (1),
  ou ladite face supérieure (6) de la membrane nanostructurée en 3D (5) étant positionnée de façon solidaire sur ladite face inférieure (3) dudit support (1), et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106) **(Figures 39 et 40).**

L'expression « **membrane non résorbable** » signifie que la membrane ne peut être éliminée ni par un procédé physique ni par un procédé chimique dans un solvant aqueux.

L'expression « **perforation** » qui se rapporte au support, désigne un évidement traversant le support de part en part, c'est-à-dire une ouverture à travers le support entre la face inférieure et la face supérieure, permettant ainsi la communication entre ces deux faces.

Cette perforation dudit support crée un espace vide à travers ledit support, et est caractérisée par une section au niveau de la face supérieure dudit support et une section au niveau de la face inférieure dudit support.

L'expression « **protubérance** » qui se rapporte à la membrane poreuse nanostructurée en 3D désigne une saillie proéminente formant une structure en relief, du côté de la face supérieure de ladite membrane poreuse nanostructurée en 3D.

L'expression « **poreuse** » qui se rapporte à la membrane poreuse nanostructurée en 3D signifie que ladite membrane comporte des pores continus interconnectés entre eux, d'un diamètre compris entre 2 et 10 nm, s'étendant de la face supérieure à la face inférieure de ladite membrane. Ces pores permettant les échanges de gaz à travers la membrane, ainsi que de petites molécules contenues dans le milieu de culture (facteurs de croissance, protéines sériques, éléments nutritifs assurant la viabilité des cellules teks que les ions $Ca^{2+}$, $K^+$, $Na^+$...). Ces pores permettent également de laisser passer des molécules pharmacologiques telles que des inhibiteurs ou des anti-cancéreux, des petits ARN (par exemple des ARN interférents), des hormones (par exemple la dihydrotestostérone).

L'expression « **nanostructurée en 3D »** qui se rapporte à la membrane poreuse désigne la présence d'au moins une structure en relief, soit en 3 dimensions, sur la face supérieure de ladite membrane poreuse, l'échelle de ladite structure étant de l'ordre du nanomètre.

L'expression « **de façon solidaire** » qui désigne la nature de la liaison entre ledit support et ladite membrane, signifie que des liaisons chimiques ainsi que des interactions hydrophobes et électrostatiques sont établies entre ledit support et ladite membrane afin de lier ladite membrane et ledit support de manière cohésive par une connexion étanche.

Le terme **« socle »** désigne la partie solide dans laquelle est intégrée ladite unité.

L'expression « **formant un tout »** utilisée pour caractériser l'agencement entre le socle et l'unité, signifie que le socle et l'unité forme ensemble une pièce d'un seul tenant, et ne sont pas dissociables l'un de l'autre.

Selon un mode de réalisation particulier, la puce microfluidique de culture cellulaire contient un module central (104) comprenant :

- une unité centrale (105), laquelle contient

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5) **(Figure 40)**,

  ladite face inférieure (7) de ladite membrane poreuse nanostructurée en 3D (5) étant positionnée de façon solidaire sur ladite face supérieure (2) dudit support (1),
  et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106) **(Figures 39 et 40).**

Selon un mode de réalisation particulier, la puce microfluidique de culture cellulaire contient un module central (104) comprenant :

- une unité centrale (105), laquelle contient

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5),

  ladite face supérieure (6) de la membrane nanostructurée en 3D (5) étant positionnée de façon solidaire sur ladite face inférieure (3) dudit support (1),
  et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106).

Selon un mode de réalisation particulier, ladite au moins protubérance (8) de la membrane poreuse nanostructurée de ladite puce microfluidique de culture cellulaire, est en forme de dôme creux et présente une base circulaire (13).

L'expression « **en forme de dôme**» qui se rapporte à la forme de la protubérance signifie que ladite protubérance a une structure arrondie en forme de voûte avec une base circulaire, formant une convexité du côté de la face supérieure dudit support.

Dans un autre mode de réalisation, ladite protubérance peut avoir une structure arrondie en forme de voûte avec une base ovale, formant une convexité du côté de la face supérieure dudit support.

L'expression « **en forme de dôme creux**» signifie que la face interne de ladite protubérance délimite un volume vide car ladite protubérance correspond à un renfoncement de ladite membrane poreuse nanostructurée en 3D du côté de la face inférieure de celle-ci, ledit renfoncement formant ainsi une concavité du côté de la face inférieure de ladite membrane, et donc un volume vide.

L'expression « **base circulaire** » qui se rapporte au dôme creux, désigne la surface inférieure vide sur laquelle repose la protubérance en forme de dôme et délimitée par la face interne de ladite protubérance.

Selon un mode de réalisation particulier, ladite au moins une perforation du support est caractérisée par une section circulaire au niveau de la face supérieure dudit support et une section circulaire au niveau de la face inférieure dudit support.

Selon un mode de réalisation particulier, ladite au moins une perforation (4) du support (1) présente

une section (11) au niveau de la face supérieure (2) du support (1) (section supérieure de la perforation) présentant un axe (x) passant par le centre de ladite section supérieure (11) de la perforation (4) perpendiculaire audit support (1),

et une section (12) au niveau de la face inférieure (3) du support (1) (section inférieure de la perforation) présentant un axe (w) passant par le centre de ladite section inférieure (12) de la perforation (4) et perpendiculaire audit support (1),

et dans laquelle lesdits axes (x) et (w) sont confondus.

Selon un mode de réalisation particulier, le diamètre d2 de la section circulaire de ladite perforation au niveau de la face inférieure du support, est supérieure ou égal au diamètre d1 de la section circulaire de ladite perforation au niveau de la face supérieure du support.

Selon un mode de réalisation particulier, ladite au moins une perforation (4) du support (1) présente

une section (11) de forme circulaire au niveau de la face supérieure du support (1) (section supérieure de la perforation) présentant un diamètre d1 (diamètre supérieur d1 de la perforation) et un axe (x) passant par le centre de ladite section supérieure (11) de la perforation (4) et perpendiculaire audit support (1),

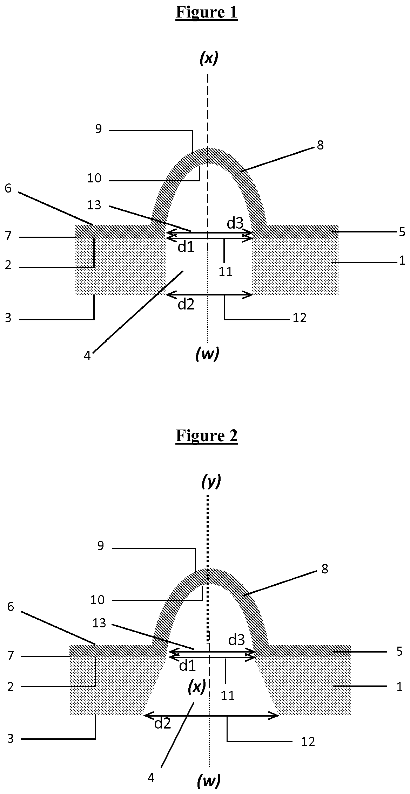

et une section (12) de forme circulaire au niveau de la face inférieure (3) du support (1) (section inférieure de la perforation) présentant un diamètre d2 (diamètre inférieur d2 de la perforation) et un axe (w) passant par le centre de ladite section inférieure de la perforation et perpendiculaire audit support (1),

le diamètre d1 étant d'une valeur supérieure ou égale à 10 $\mu$m à une valeur inférieure ou égale à 500 $\mu$m, préférablement d'une valeur de 150 $\mu$m, et le diamètre d2 étant d'une valeur supérieure ou égale à 10 $\mu$m à une valeur inférieure ou égale à 500 $\mu$m, préférablement d'une valeur de 150 $\mu$m,

tel que la valeur du diamètre d2 est supérieure ou égale à la valeur du diamètre d1,

et dans laquelle lesdits axes (x) et (w) sont confondus **(Figures 1 et 2)**.

Selon un mode de réalisation particulier, le diamètre d2 de la section circulaire de ladite perforation au niveau de la face inférieure du support, est supérieur au diamètre d1 de la section circulaire de ladite perforation au niveau de la face supérieure du support.

Selon un mode de réalisation particulier, le diamètre d2 de la section circulaire de ladite perforation au niveau de la face inférieure du support, est supérieur au diamètre d1 de la section circulaire de ladite perforation au niveau de la face supérieure du support, tel que le ratio (valeur de d1/ valeur de d2) est compris d'une valeur de 0.3 à une valeur inférieure à 1.

Selon un mode de réalisation particulier, e diamètre d2 de la section circulaire de ladite perforation au niveau de la face inférieure du support, est supérieur au diamètre d1 de la section circulaire de ladite perforation au niveau de la face supérieure du support, et ladite perforation dudit support est en forme de cône tronqué.

Selon un mode de réalisation particulier, la valeur du diamètre supérieur d1 de ladite au moins une perforation (4) est inférieure à la valeur du diamètre inférieur d2 de ladite au moins une perforation (4), et ladite perforation (4) est en forme de cône tronqué (Figure 2).

Selon un mode de réalisation particulier, le diamètre d2 de la section circulaire de ladite perforation au niveau de la face inférieure du support, est égal au diamètre d1 de la section circulaire de ladite perforation au niveau de la face supérieure du support.

Selon un mode de réalisation particulier, le diamètre d2 de la section circulaire de ladite perforation au niveau de la face inférieure du support, est égal au diamètre d1 de la section circulaire de ladite perforation au niveau de la face supérieure du support, et ladite perforation dudit support est de forme cylindrique.

Dans ce mode de réalisation, la perforation dudit support est donc de forme régulière c'est-à-dire que les diamètres de l'ensemble de ses sections sont de longueurs égales, et que lesdites sections sont de forme circulaire.

Selon un mode de réalisation particulier, la valeur du diamètre supérieur d1 de ladite au moins une perforation (4) est égale à la valeur du diamètre inférieur d2 de ladite au moins une perforation (4), et ladite perforation (4) est de forme cylindrique **(Figure 1)**.

Selon un mode de réalisation particulier, ladite protubérance (8) présente une base circulaire (13) présente de diamètre d3 (diamètre d3 de la base circulaire de la protubérance), ledit diamètre d3 de la protubérance (8) étant d'une valeur de 10 $\mu$m à une valeur de 500 $\mu$m.

Selon un mode de réalisation particulier, la valeur du diamètre d3 de ladite au moins une protubérance (8) est égale à la valeur du diamètre supérieur d1 de ladite au moins une perforation (4) **(Figures 1 et 2)**.

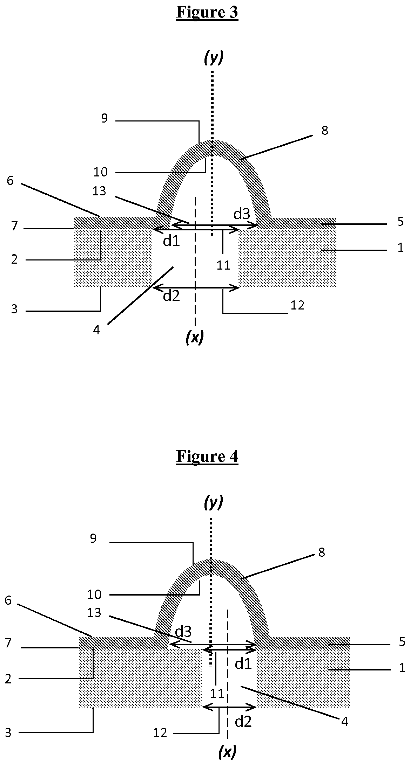

Selon un mode de réalisation particulier, la valeur du diamètre d3 de ladite au moins une protubérance (8) est égale à la valeur du diamètre supérieur d1 de ladite au moins une perforation et est égale à la valeur du diamètre inférieur d2 de ladite au moins une perforation (4) **(Figures 1 et 3)**.

Selon un mode de réalisation particulier, la valeur du diamètre d3 de ladite au moins une protubérance (8) est supérieure à la valeur du diamètre supérieur d1 de ladite au moins une perforation (4) **(Figures 4, 5 et 6)**.

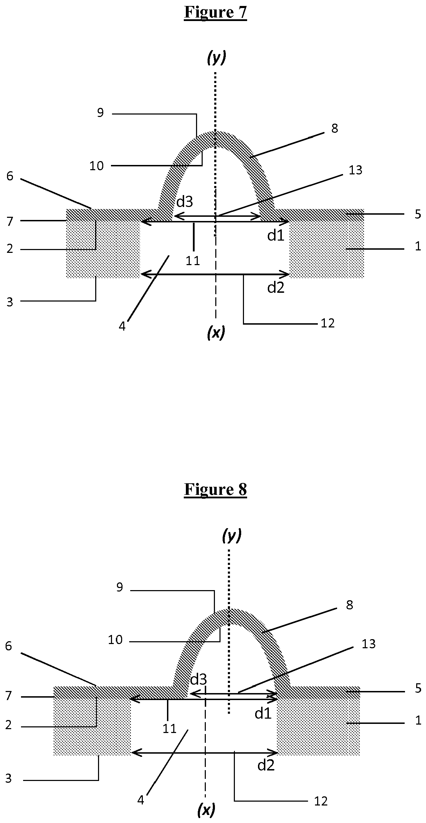

Selon un mode de réalisation particulier, la valeur du diamètre d3 de ladite au moins une protubérance (8) est inférieure à la valeur du diamètre supérieur d1 de ladite au moins une perforation (4) **(Figures 7, 8, 9, 10 et 11)**.

Selon un mode de réalisation particulier, ladite face interne (10) de ladite au moins une protubérance (8) est en totalité ou en partie en regard de ladite au moins une perforation (4) dudit support (1).

La membrane nanostructurée en 3D comportant au moins une protubérance et le support perforé par au moins une perforation sont positionnés l'un par rapport de façon à ce que la face interne de la dite protubérance soit en totalité ou en partie en regard de la section circulaire de ladite perforation au niveau de la face supérieure du support.

Selon un mode de réalisation particulier, ladite face interne (10) de ladite au moins une protubérance (8) est **en totalité en regard de ladite au moins une perforation** (4) dudit support (1) :

- lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus**

**(Figures 1, 2, 7 et 9)**; ou

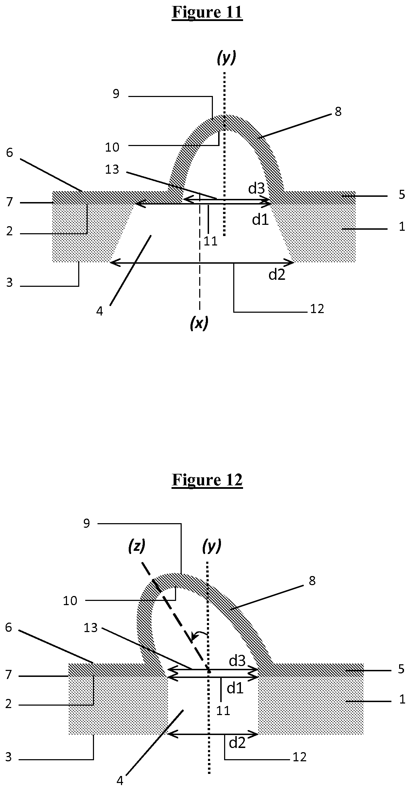

- lorsque la valeur du diamètre supérieur d1 de ladite au moins une perforation (4) est **supérieure à** la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de dl - valeur de d3)/2] **(Figures 8 et 11).**

Le terme « **confondus** » utilisé pour caractériser les axes (x) et (y) signifie que la distance entre ces deux axes est égale à zéro.

Le terme « **distincts** » utilisé pour caractériser les axes (x) et (y) signifie que la distance entre ces deux axes est strictement supérieure à zéro.

Selon un mode de réalisation particulier, ladite face interne (10) de ladite au moins une protubérance (8) est **en partie en regard de ladite au moins une perforation (4)** dudit support (1) :

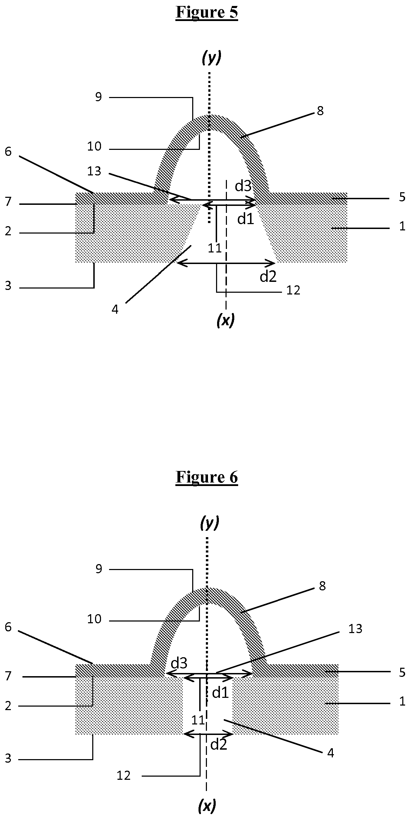

- lorsque la valeur du diamètre supérieur d1 de ladite au moins une perforation (4) est **inférieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus (Figure 6)**; ou

- lorsque la valeur du diamètre supérieur d1 de ladite perforation (4) est **égale ou inférieure à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure à la valeur de d3 **(Figures 3, 4 et 5)**; ou

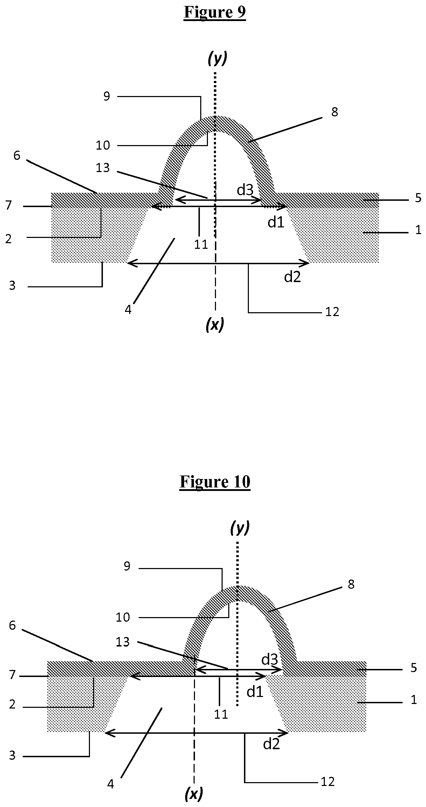

- lorsque la valeur du diamètre supérieur d1 de ladite perforation (4) est **supérieure** à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur supérieure à [(valeur de d1 - valeur de d3)/2] à une valeur inférieure à [[valeur de d1 + valeur de d3] /2] **(Figure 10).**

Selon un mode de réalisation particulier, la protubérance (4) présente une déformation oblique, ladite déformation oblique étant définie par l'angle α formé entre

- l'axe (z) passant par le centre de la base circulaire (13) de diamètre d3, délimitée par ladite protubérance (8) au niveau de la face supérieure (2) dudit support (1), et par le sommet de ladite protubérance (8),
- et l'axe (y) passant par le centre de ladite base circulaire (13) et qui est perpendiculaire audit support (1) **(Figure 12).**

Selon un mode de réalisation particulier, l'angle α formé entre l'axe (y) et l'axe (z) est compris entre 0 et 45°.

Selon un mode de réalisation particulier, l'angle α formé entre l'axe (y) et l'axe (z) est compris entre 0 et 60°.

Si l'angle α formé entre l'axe (y) et l'axe (z) est supérieur à 45°, l'inclinaison de la protubérance bloque partiellement la récupération des sécrétions. La valeur maximale théorique que peut prendre l'angle α est de 90°, mais dans ce mode de réalisation, la protubérance est complètement rabattue le long de la face supérieure de ladite membrane nanostructurée en 3D, et la récupération des sécrétions est bloquée.

Selon un mode de réalisation particulier, la face externe (9) de ladite protubérance (8) supporte au moins une cellule adhérente à ladite face externe, et dans laquelle la face interne (10) de ladite protubérance (8) supporte au moins une cellule adhérente à ladite face interne (10), les au moins deux cellules appartenant à deux types de cellules distincts.

Le terme de « **cellules adhérentes** » désigne tout type de cellules dont la croissance nécessite une adhésion à un support et pour lesquelles le détachement audit support nécessite un traitement mécanique ou enzymatique (par exemple avec de la trypsine).

Le terme de « **types de cellules distincts** » est utilisé pour désigner des types de cellules de nature, de fonction différentes ou provenant de tissus différents.

Selon un mode de réalisation particulier, ladite cellule adhérente à ladite face externe est une cellule stromale, et ladite cellule adhérente à ladite face interne est une cellule épithéliale.

Selon un mode de réalisation particulier, ladite au moins une cellule adhérente à ladite face externe (9) est une cellule stromale, plus particulièrement un fibroblaste, et ladite au moins une cellule adhérente à ladite face interne (10) est une cellule épithéliale.

Selon un mode de réalisation particulier, ladite cellule adhérente à ladite face externe est une cellule épithéliale, et ladite cellule adhérente à ladite face interne est une cellule stromale.

Les cellules épithéliales peuvent être des lignées cellulaires commerciales non tumorigènes de prostate, vessie ou rein, ou des cultures primaires commerciales.

Les cellules stromales peuvent être des fibroblastes (cultures primaires commerciales ou lignées), des cellules mésenchymateuses (cultures commerciales ou lignées), ou d'autres cellules stromales telles que les cellules endothéliales.

Les deux types de cellules cultivés sur les faces internes et externes de ladite protubérance, sont dites « neutres » ou saines, c'est-à-dire qu'elles sont non tumorigènes.

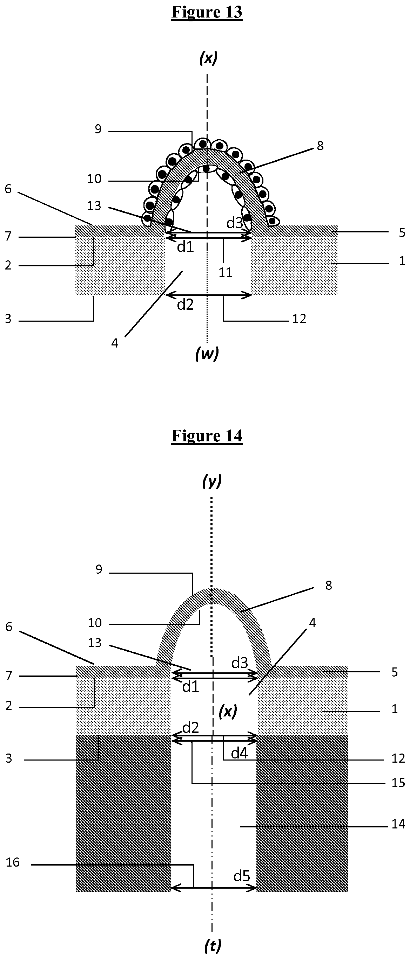

Selon un mode de réalisation particulier, la face externe (9) de ladite protubérance (8) supporte un premier ensemble de cellules adhérentes au stade de la confluence, et dans laquelle la face interne (10) de ladite protubérance (8) supporte un deuxième ensemble de cellules adhérentes au stade de la confluence, les cellules du premier et du deuxième ensemble de cellules adhérentes appartenant à deux types cellulaires distincts **(Figure 13)**.

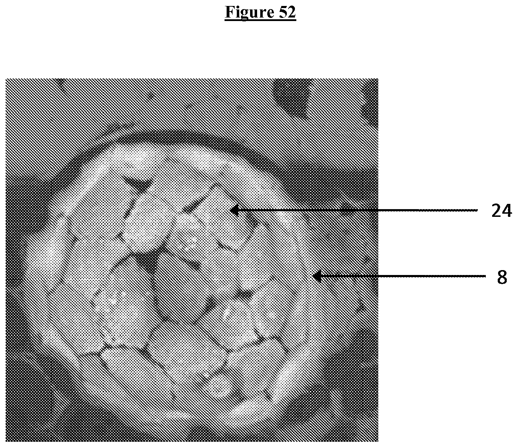

Selon un mode de réalisation particulier, la face externe (9) de ladite protubérance (8) supporte un premier ensemble de cellules adhérentes avantageusement au stade de la confluence, ledit premier ensemble étant un ensemble de cellules stromales, plus particulièrement de fibroblastes, et dans laquelle la face interne (10) de ladite protubérance (8) supporte un deuxième ensemble de cellules adhérentes au stade de la confluence, ledit deuxième ensemble étant un ensemble de cellules épithéliales **(Figure 52)**.

Selon un mode de réalisation particulier, la face externe (9) de ladite protubérance (8) supporte un premier ensemble de cellules adhérentes, ledit premier ensemble étant un ensemble de cellules stromales, plus particulièrement de fibroblastes, et dans laquelle la face interne (10) de ladite protubérance (8) supporte un deuxième ensemble de cellules adhérentes au stade de la confluence, ledit deuxième ensemble étant un ensemble de cellules épithéliales.

Selon un mode de réalisation particulier, la face externe et la face interne des protubérances, sont recouvertes d'une préparation de matrice extracellulaire (ECM) (Matrigel®, collagène, fibronectine, acide hyaluronique), avant l'introduction des cellules sur ces surfaces, soit avant la culture cellulaire. Plus particulièrement, ladite préparation ECM est composée de Matrigel® ou d'un mélange Matrigel® /collagène.

Selon un mode de réalisation particulier, ladite membrane poreuse nanostructurée en 3D a une épaisseur de 2 à 300 nm, plus particulièrement de 30 nm.

Selon un mode de réalisation particulier, ladite membrane poreuse nanostructurée en 3D a une épaisseur de 2 à 300 nm.

Selon un mode de réalisation particulier, ladite membrane poreuse nanostructurée en 3D a une épaisseur de 30 nm.

La protubérance possédant une section à sa base de forme circulaire, la surface de cette section peut être calculée en fonction du rayon.

Selon un mode de réalisation particulier, ladite protubérance en forme de dôme creux a une hauteur de 1 à 600 $\mu$m, plus particulièrement de 1 à 300 $\mu$m, plus particulièrement de 50 à 300 $\mu$m, plus particulièrement de 50 $\mu$m et une surface délimitée par la base du dôme de 78,5 $\mu$m$^2$ à 200 000 $\mu$m$^2$, plus particulièrement de 2 000 à 70 000 $\mu$m$^2$, encore plus particulièrement de 17 500 $\mu$m$^2$, et encore plus particulièrement de 7850 $\mu$m$^2$.

Le terme « **hauteur** » désigne la distance entre le centre du sommet de la protubérance et le centre de l'ouverture de diamètre d3.

Selon un mode de réalisation particulier, ledit support comporte au moins deux perforations de forme et de dimensions identiques, et ladite membrane poreuse nanostructurée en 3D comporte au moins deux protubérances de forme et de dimensions identiques, le nombre de perforations étant égal au nombre de protubérances.

Lorsque que ladite membrane comporte au moins deux protubérances, il convient de définir la distance entre ces au moins deux protubérances.

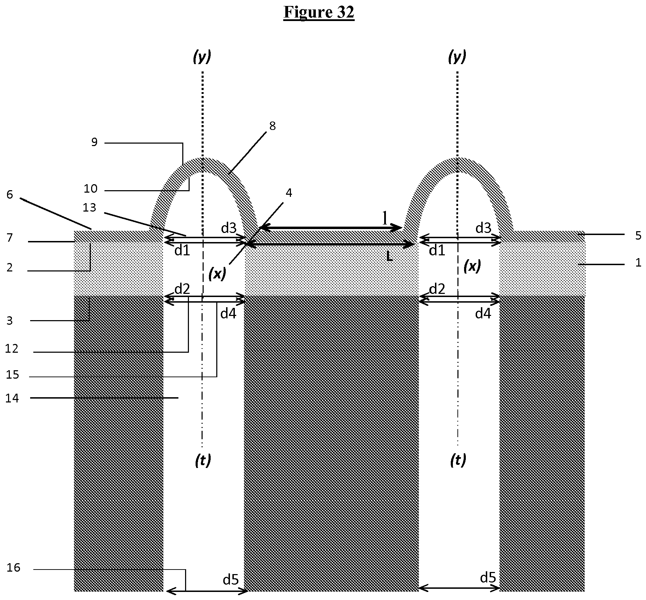

La définition de l'expression « **distance entre deux protubérances contiguës** » doit être comprise comme étant la distance entre deux points, situés respectivement sur la circonférence formée par l'intersection de la face externe de ladite protubérance et de la face supérieure de ladite membrane nanostructurée en 3D de deux protubérances contiguës, et formant la distance la plus courte parmi l'ensemble des distances possibles entre les couples de points situés respectivement sur lesdites circonférences de deux protubérances contiguës **(Figure 32)**.

Lorsque que ladite membrane comporte au moins deux protubérances, celles-ci doivent être séparées par une distance appropriée.

Selon un mode de réalisation particulier, **la distance entre deux protubérances contiguës** est d'une valeur 1, ladite valeur 1 étant de 10 à 100 $\mu$m.

Selon un mode de réalisation particulier, la **distance entre deux protubérances contiguës** est d'une valeur 1, ladite valeur 1 étant de 10 à 100 $\mu$m, plus particulièrement de 50 à 100 $\mu$m.

Selon un mode de réalisation particulier, la **distance entre deux protubérances contiguës** est d'une valeur 1, ladite valeur 1 étant de 50 à 100 $\mu$m.

Dans chacun des paragraphes ci-dessous, l'expression « **distance entre deux protubérances contiguës** » peut être remplacée par la définition donnée ci-dessus.

Selon un mode de réalisation particulier, ledit support comporte au moins deux perforations, et ladite membrane poreuse nanostructurée comporte au moins deux protubérances

tel que le ratio $\frac{d1}{d2}$ varie de 1 à $\frac{d1}{(d1+l)}$, 1 étant la valeur de la distance deux protubérances contiguës, où le diamètre d1 est d'une valeur supérieure ou égale à 10 $\mu$m à une valeur inférieure ou égale à 500 $\mu$m, et

le diamètre d2 est d'une valeur supérieure ou égale à 10 $\mu$m et inférieure ou égale à 500 $\mu$m.

Ainsi, le diamètre inférieur d2 de ladite perforation au niveau de la face inférieure du support, est toujours supérieur ou égal au diamètre supérieur d1 de ladite perforation au niveau de la face supérieure du support.

Selon un mode de réalisation particulier, ladite membrane poreuse nanostructurée en 3D comporte 1 à 100 protubérances pour une surface de l'unité centrale de 1 cm$^2$.

Selon un mode de réalisation particulier, ladite membrane poreuse nanostructurée en 3D comporte un nombre maximal de protubérances pour une surface de l'unité centrale de 1 cm$^2$, égal à la formule

$$\frac{\pi \, (d3/2)^2}{(l + d3) - \pi \, (d3/2)^2}$$

où 1 est la distance entre deux protubérances contiguës, ladite valeur 1 étant de 10 à 100 $\mu$m, plus particulièrement de 50 à 100 $\mu$m,

et où d3 est le diamètre de ladite protubérance.

La membrane poreuse nanostructurée en 3D est composée d'un nombre de couches successives de polyélectrolytes de 1 à 150, étant entendu que la successivité des couches s'applique à partir d'au moins deux couches.

Le nombre de couches permet de faire varier la tenue mécanique de la protubérance. En effet, plus le nombre de couches sera élevé et plus la résistance mécanique de la protubérance sera élevée afin qu'elle puisse conserver sa forme tout en soutenant la culture cellulaire sur ces faces externes et internes et en résistant au flux (par exemple de milieu de culture) de part et d'autre de celle-ci.

Selon un mode de réalisation particulier le nombre de couches successives de polyélectrolytes est de 1 à 150, en particulier de 15.

L'utilisation de polyélectrolyte pour constituer la membrane poreuse nanostructurée en 3D fournit un substitut biocompatible et fonctionnel pour mimer la lame basale sur laquelle viennent se fixer les cellules adhérentes dans les tissus.

La membrane poreuse nanostructurée en 3D peut être constituée d'une couche d'un polyélectolyte.

Selon un mode de réalisation particulier, la membrane poreuse nanostructurée en 3D est constituée d'une couche d'un polyélectolyte choisi parmi le poly(sodium 4-styrènesulphonate) (PSS), le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

La membrane poreuse nanostructurée en 3D, peut être constituée d'au moins deux couches successives d'un polyélectolyte.

Lorsque ladite membrane comprend au moins deux couches, la construction de cette membrane couche par couche est indispensable.

La au moins une protubérance étant une partie intégrante de ladite la membrane poreuse nanostructurée en 3D, la composition de ladite la membrane poreuse nanostructurée en 3D sera identique à la composition de ladite au moins une protubérance.

Selon un mode de réalisation particulier, la membrane poreuse nanostructurée en 3D est constituée de couches successives d'un polyélectrolyte choisi parmi le poly(sodium 4-styrènesulphonate) (PSS), le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

Lorsque ladite membrane comprend au moins deux couches successives, lesdites couches peuvent correspondre à au moins deux polyélectrolytes de nature différente : au moins un polyélectrolyte chargé négativement et au moins un polyélectrolyte chargé positivement.

Dans ce cas, la construction couche par couche de cette membrane doit être effectuée de telle manière qu'une couche de polyélectrolyte chargé positivement soit en alternance avec une couche de polyélectrolyte chargé négativement.

La membrane est ainsi fortement cohésive en raison des interactions électrostatiques entre les couches de polyélectrolytes chargées positivement et les couches de polyélectrolytes chargées négativement. Cela permet d'obtenir une membrane ayant un module de Young de l'ordre du kiloPascal.

Il est à noter que la couche inférieure et la couche supérieure de la membrane poreuse, peuvent être constituées, indépendamment l'une de l'autre, de l'un quelconque des polyélectrolytes.

Cependant, la charge du polyélectrolyte constituant la dernière couche, et constituant donc la face supérieure de ladite membrane, a un impact sur l'hydrophobicité de la membrane. Ainsi, lorsque la dernière couche est constituée par un polyélectrolyte chargée négativement, la face supérieure de la membrane est de nature relativement hydrophile en comparaison à une membrane dont la face supérieure est chargée positivement. A l'inverse, lorsque la dernière couche est constituée par un polyélectrolyte chargée positivement, la face supérieure de la membrane est de nature relativement hydrophobique en comparaison à une membrane dont la face supérieure est chargée négativement.

La rugosité de la face supérieure de la membrane est régie par les groupements portés par sur le polyélectrolyte constituant la dernière couche de la membrane, soit la couche constituant la face supérieure de ladite membrane. Par exemple, s'il s'agit d'une couche de PSS, ce polyélectrolyte possède un noyau benzène pendant attaché et un groupement sulfate lié, qui fournissent ainsi une surface plus rugueuse qu'une couche de PAH, électrolyte qui ne possède pas de noyau benzène attaché.

La rugosité de la membrane poreuse nanostructurée en 3 D est de l'ordre du nanomètre.

Selon un mode de réalisation particulier, le nombre de couches successives est de 1 à 150, en particulier de 15, d'un polyélectrolyte chargé positivement et d'un polyélectrolyte chargé négativement, ladite couche de polyélectrolyte chargé négativement et ladite couche de polyélectrolyte chargé positivement étant en alternance l'une par rapport à l'autre.

Selon un mode de réalisation particulier, la membrane poreuse nanostructurée en 3D est constituée de couches successives d'au moins deux polyélectrolytes, dont au moins un polyélectrolyte est chargé positivement et au moins un polyélectrolyte est chargé négativement,

ledit polyélectrolyte chargé positivement étant choisi parmi le poly(sodium 4-styrènesulphonate), le poly(éthylèneimine), le poly(chlorure de diallyldimethylammonium), le poly(chlorure de acrylamide-*co*-diallyidimethylammonium), le chlorure de diallyldiméthylammonium,

et ledit polyélectrolyte chargé négativement étant choisi parmi le poly(hydrochlorure d'allylamine), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

ladite couche de polyélectrolyte chargé négativement et ladite couche de polyélectrolyte chargé positivement étant en alternance l'une par rapport à l'autre.

Selon un mode de réalisation particulier, la membrane poreuse nanostructurée en 3D est constituée de couches successives d'un polyélectrolyte chargé positivement et d'un polyélectrolyte chargé négativement, ledit polyélectrolyte chargé positivement étant le poly(sodium 4- styrènesulphonate) (PSS) et ledit polyélectrolyte chargé négativement étant le poly(hydrochlorure d'allylamine) (PAH), ladite couche de polyélectrolyte chargé négativement et ladite couche de polyélectrolyte chargé positivement étant en alternance l'une par rapport à l'autre.

Selon un mode de réalisation particulier, le nombre de couches successives de poly(sodium 4-styrenesulphonate) (PSS) et/ou de poly(allylamine hydrochloride) (PAH), est de 1 à 150 , en particulier de 15.

Selon un mode de réalisation particulier, chaque couche de polyélectrolyte a une épaisseur de 2 à 300 nm, plus particulièrement d'environ 2 nm.

Selon un mode de réalisation particulier, le nombre de couches successives est de 15, d'un polyélectrolyte chargé positivement et d'un polyélectrolyte chargé négativement, ladite couche de polyélectrolyte chargé négativement et ladite couche de polyélectrolyte chargé positivement étant en alternance l'une par rapport à l'autre, chaque couche ayant une épaisseur de 2 nm.

Selon un mode de réalisation particulier, la valeur de l'épaisseur de la totalité des couches successives de polyélectrolytes est comprise de la valeur de l'épaisseur d'une couche à une valeur inférieure à la moitié du diamètre d3 de ladite protubérance.

Selon un mode de réalisation particulier, la membrane poreuse nanostructurée en 3D (5) est constituée d'au moins une couche d'un polyélectolyte choisi parmi le poly(sodium 4-styrènesulphonate) (PSS), le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté,

et dans laquelle lorsque ladite membrane poreuse nanostructurée en 3D (5) est constituée d'au moins deux couches successives, une couche constituée d'un polyélectrolyte chargé positivement est en alternance d'une couche constituée d'un polyélectrolyte chargé négativement,

ladite membrane poreuse nanostructurée en 3D (5) étant en particulier constituée 1 à 150 couches successives de polyélectrolyte,

et plus particulièrement ladite membrane poreuse nanostructurée en 3D (5) étant constituée de 15 couches successives de poly(sodium 4-styrènesulphonate) (PSS) et/ou de poly(allylamine hydrochloride) (PAH), en alternance l'une de l'autre.

La membrane poreuse nanostructurée en 3D est donc composée de couches successives de polyélectrolytes et comporte ainsi comme paramètres variables :

- le nombre de couches,

- l'épaisseur de chacune des couches,

- la charge du ou des polyélectrolytes utilisés.

En faisant varier le nombre de couches, la rugosité, l'épaisseur et la rigidité de ladite membrane, et donc de ladite protubérance peuvent être modifiées.

En faisant varier le nombre de couches ou le type de charge des polyélectrolytes utilisés, l'hydrophobicité de ladite membrane et de ladite protubérance peut également être modifiée.

Selon un mode de réalisation particulier, ledit support a une épaisseur de 2 $\mu$m à 1000 $\mu$m plus particulièrement de 20 $\mu$m.

Selon un mode de réalisation particulier, ladite perforation a une surface délimitée par ladite membrane non résorbable de 78,5 $\mu$m$^2$ à 200 000 $\mu$m$^2$, plus particulièrement de 2 000 à 70 000 $\mu$m$^2$, encore plus particulièrement de 17 500 $\mu$m$^2$, et encore plus particulièrement de 7850 $\mu$m$^2$.

Le support comporte au moins une perforation. Lorsque que ledit support comporte au moins deux perforations, il convient de définir la distance entre ces au moins deux perforations.

La « **distance entre deux perforations contigües** » doit être comprise comme étant la distance entre deux points situés respectivement sur la circonférence de chacune des sections circulaires supérieures desdites perforations contiguës et formant la distance la plus courte, parmi l'ensemble des distances possibles entre les couples de points situés respectivement la circonférence de chacune des sections circulaires supérieures desdites perforations contiguës.

Lorsque que ledit support comporte au moins deux perforations, celles-ci doivent être séparées par une distance appropriée.

Selon un mode de réalisation particulier, ladite distance entre deux perforations contiguës est d'une valeur L, ladite valeur L étant de 10 à 100 $\mu$m, plus particulièrement de 50 à 100 $\mu$m **(Figure 32).**

Selon un mode de réalisation particulier, ledit support comporte 1 à 100 perforations pour une surface de l'unité centrale de 1 cm$^2$.

Selon un mode de réalisation particulier, le support est composé de plastique bioimprimé, de polycarbonate, de plastique de culture tissulaire, de verre ou de résine SU-8.

La résine SU-8 est une résine polymérique photosensible négative à base d'époxyde novolac possédant une fonctionnalité moyenne en groupes époxyde d'environ 8. La résine SU-8 est bien connue de l'homme du métier et couramment utilisée dans la fabrication de micro-système. Le terme négatif signifie que les parties exposées aux UV deviennent réticulées, tandis que le reste du film reste soluble et peut être éliminé par lavage.

Les plastiques bioimprimés correspondent aux plastiques connus de l'homme de métier qui sont adaptés et compatibles aux équipements d'impression 3D (extrusion, contraintes de température).

Les plastiques de culture tissulaire correspondent aux plastiques, constituant les boites de Pétri, qui comportent un traitement en fond (traitement effectué par décharge électrique ou plasma pour rendre la surface hydrophile c'est-à-dire avec une charge nette négative) et permettent la fixation et la croissance de cellules adhérentes eucaryotes (à la différence des boites de culture plastiques non traitées pour la bactériologie).

Selon un mode de réalisation particulier, le support est composé de plastique bioimprimé, de polycarbonate, de plastique de culture tissulaire, de verre ou de résine SU-8, et ledit support est transparent.

## MODULE INFERIEUR

Selon un mode de réalisation particulier, le module inférieur (107) solide, comporte :

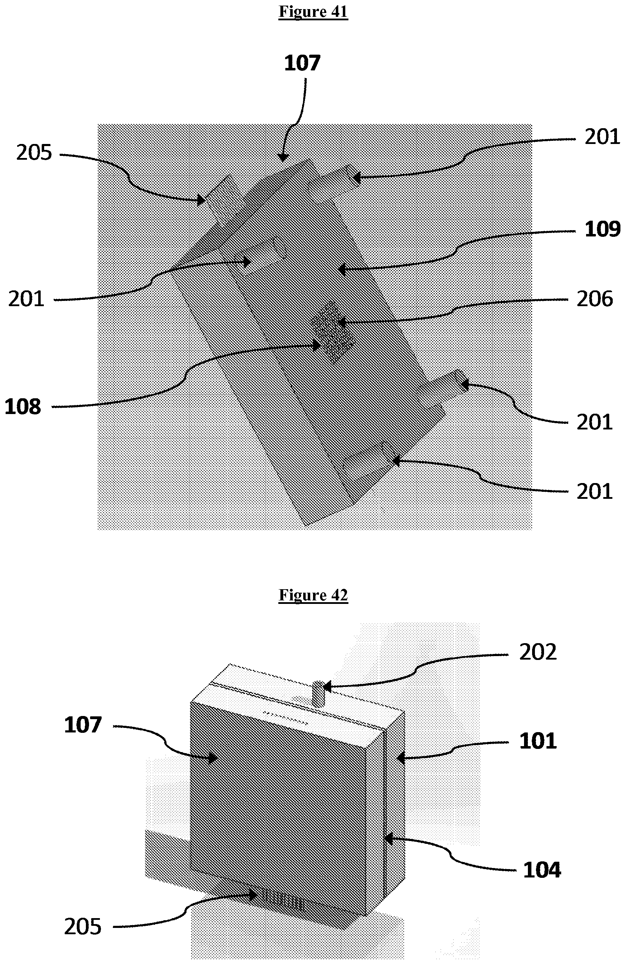

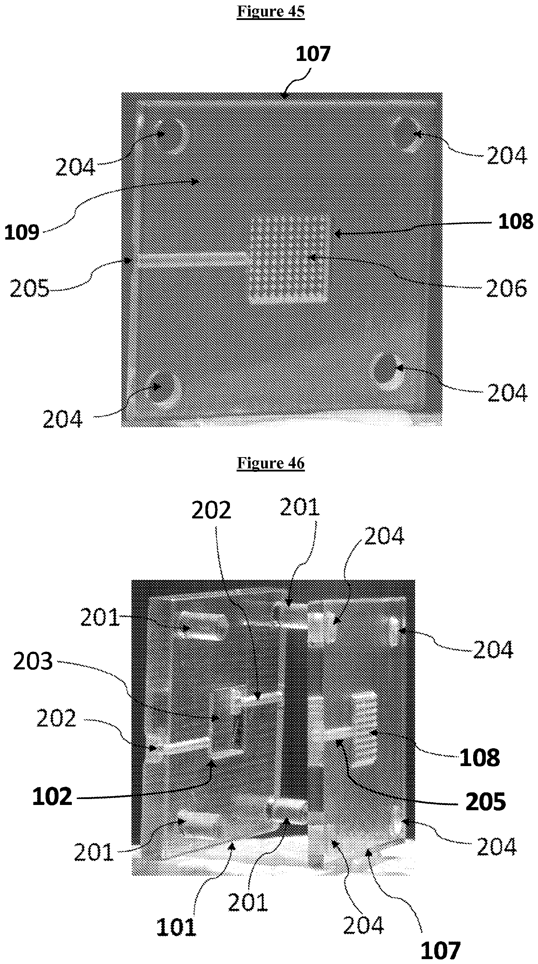

- une unité inférieure (108) laquelle contient une face supérieure, une face inférieure et au moins une face latérale, et comprenant au moins un canal (14) de forme tubulaire comportant un orifice supérieur (15) et un orifice inférieur (16),
- un socle (109),

ladite unité inférieure (108) étant intégrée dans ledit socle (109), et formant un tout avec ledit socle (109), et ladite face supérieure de ladite unité inférieure comportant ledit orifice supérieur (15) du au moins un canal, et ledit orifice inférieur (16) débouchant, lui-même ou par des moyens intermédiaires (17), à l'extérieur dudit socle (109) **(Figures 41 et 45).**

L'orifice supérieur du au moins un canal est situé au sein de l'unité inférieure, le au moins un canal s'étend à travers l'unité inférieure puis à travers le socle du module inférieur, ladite unité et ledit socle formant une pièce d'un seul tenant, de telle sorte que l'orifice inférieur du au moins un canal débouche à l'extérieur dudit socle.

L'orifice inférieur du au moins un canal débouche soit lui-même à l'extérieur dudit socle, soit débouche dans un moyen intermédiaire tel qu'un réservoir, qui lui débouche à l'extérieur dudit socle.

Selon un mode de réalisation particulier, ledit module inférieur (107) et ledit module central (104) sont assemblés tel que l'orifice supérieur (15) du au moins un canal (14), de ladite face supérieure de ladite unité inférieure (108), s'ouvre sur au moins une desdites perforations (4) dudit support (1)de ladite unité centrale (105), et l'orifice inférieur (16) du au moins un canal (14), débouche sur l'extérieur de la puce, ladite face supérieure de ladite unité inférieure (108) et ladite face inférieure dudit support (3) de ladite unité centrale (105) ayant une forme et une surface identiques entre elles.

L'expression **« à l'extérieur de la puce»** doit s'interpréter comme **« à l'extérieur du socle »**, et vice-versa.

Lorsque le module inférieur est assemblé au module central, l'orifice supérieur (15) du au moins un canal (14), de ladite face supérieure de ladite unité inférieure (108), s'ouvre sur au moins une desdites perforations (4) dudit support (1). Ce au moins un canal permet de récupérer les sécrétions des cellules cultivées sur la face interne de la au moins une protubérance.

Ce au moins canal permet également d'introduire du milieu de culture et éventuellement les cellules qui sont cultivées sur la face interne de ladite protubérance.

Selon un mode de réalisation particulier, ledit module inférieur (107) et ledit module central (104) sont assemblés tel que l'orifice supérieur (15) du au moins un canal (14), de ladite face supérieure de ladite unité inférieure (108), s'ouvre sur au moins une desdites perforations (4) dudit support (1) de ladite unité centrale (105), et l'orifice inférieur (16) du au moins un canal (14), débouche sur l'extérieur de la puce, via un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107), ladite face supérieure de ladite unité inférieure (108) et ladite face inférieure dudit support de ladite unité centrale (105) ayant une forme et une surface identique entre elles.

Selon un mode de réalisation particulier, ledit orifice inférieur (16) du au moins un canal (14), débouche à l'extérieur de la puce sur un dispositif permettant l'analyse de composés en solution.

Ce dispositif d'analyse peut être constitué par tout dispositif analytique connu de l'Homme du métier tel qu'un spectromètre de masse, un dispositif RMN, un dispositif de chromatographie (en phase liquide ou gazeuse), un dispositif basé sur les interactions immunologiques (ELISA, immunoprécipitation), un dispositif PCR ou RT-PCR.

En effet, les sécrétions des cellules sont composées à la fois de protéines et peptides mais également d'ADN et d'ARN. Ainsi, le ou les dispositifs choisis d'analyse doivent permettre d'analyser l'ensemble de ces molécules.

Selon un mode de réalisation particulier ledit orifice inférieur (16) du au moins un canal (14), débouche à l'extérieur de la puce sur un dispositif permettant l'analyse de composés en solution, ledit dispositif étant un spectromètre de masse.

La puce microfluidique peut être couplée directement à une puce intégrant un spectromètre de masse afin de réaliser une analyse en temps réel et en continu des sécrétions.

Selon un mode de réalisation particulier, ladite unité inférieure (108) et ladite unité centrale (105) sont assemblées par des éléments de fixation (204) situés respectivement sur chacun des socles du module inférieur (109) et du module central (106), de façon à assembler de manière étanche ladite face supérieure de ladite unité inférieure (108) et ladite face inférieure dudit support (3) de ladite unité centrale (105).

Selon un mode de réalisation particulier, la valeur du diamètre d4 dudit orifice supérieur (15) dudit canal (14) est supérieure à la valeur du diamètre d5 dudit orifice inférieur (16) dudit canal (14).

Selon un mode de réalisation particulier, la valeur du diamètre d4 dudit orifice supérieur (15) dudit canal (14) est égale à la valeur du diamètre d5 dudit orifice inférieur (16) dudit canal (14), ledit au moins un canal étant de forme cylindrique.

Selon un mode de réalisation particulier, ledit orifice supérieur (15) dudit au moins un canal (14) présente un diamètre d4 et un axe (t) passant par le centre dudit orifice supérieur (15) et perpendiculaire audit support (1),

tel que la valeur du diamètre d4 est **supérieure ou égale** à la valeur du diamètre inférieur d2 de ladite au moins une perforation (4).

Selon un mode de réalisation particulier, ledit orifice supérieur (15) dudit au moins un **canal (14)** débouche sur **une seule perforation** (4) dudit support (1).

Selon un mode de réalisation particulier, ladite au moins une perforation (4) dudit support (1) est en **totalité en regard** dudit orifice supérieur (15) dudit au moins un canal (14) :

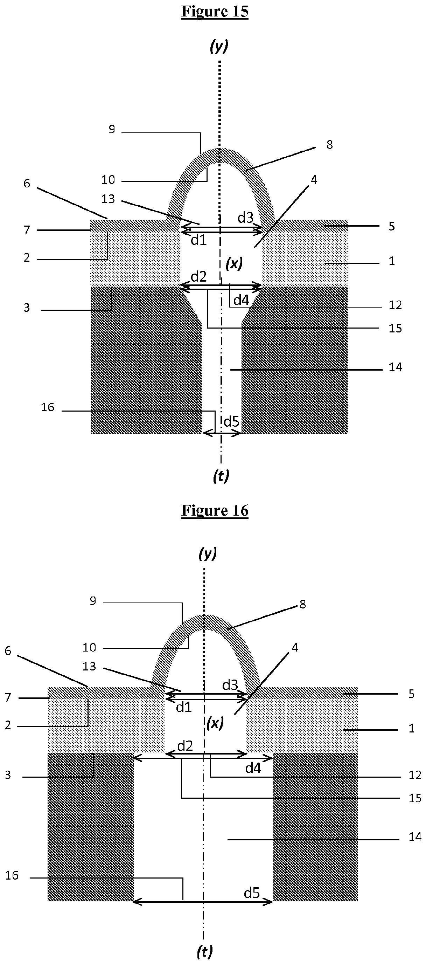

- lorsque la valeur du diamètre inférieure d2 de ladite perforation (4) est égale à la valeur du diamètre d4 dudit orifice supérieur (15) dudit canal (14), et que l'axe (x), passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont confondus **(Figures 14 et 15),** ou

- lorsque la valeur du diamètre inférieure d2 de ladite perforation (4) est **inférieure** à la valeur du diamètre d4 dudit orifice supérieur (15) dudit canal (14), et que l'axe (x), passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **confondus, ou distincts l'un par rapport à l'autre** d'une distance d'une valeur inférieure ou égale à [(valeur de d4 - valeur de d2) /2] (Figures 16, 17 et 18).

Selon un mode de réalisation particulier, ladite au moins une perforation (4) dudit support (1) est **en partie en regard** dudit orifice supérieur (15) dudit au moins un canal (14) :

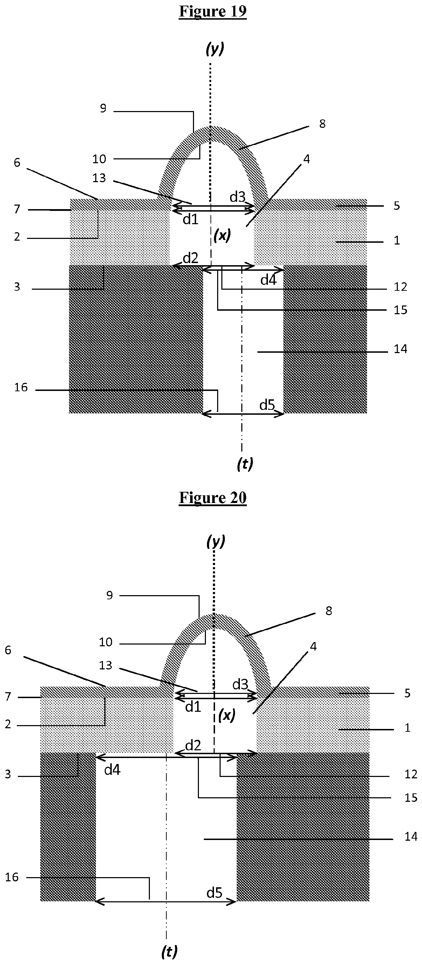

- lorsque la valeur du diamètre inférieure d2 de ladite perforation (4) est égale à la valeur du diamètre d4 dudit orifice supérieur (15) dudit canal (14), et que l'axe (x) passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **distincts** l'un par rapport à l'autre d'une distance d'une valeur inférieure ou égale à [valeur de d4 / 2] **(Figures 19, 21, 22, 24, 29 et 30)**, ou

- lorsque la valeur du diamètre inférieure d2 de ladite perforation (4) est **inférieure** à la valeur du diamètre d4 dudit orifice supérieur (15) dudit canal (14), et que l'axe (x) passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **distincts** l'un par rapport à l'autre d'une distance d'une valeur inférieure ou égale à [valeur de d4 / 2] **(Figures 20, 23, 25, 26, 27 et 28)**.

Selon un mode de réalisation particulier, ledit module inférieur (107) comporte **au moins deux canaux (14)**.

Selon un mode de réalisation particulier, chacun des orifices supérieurs (15) des au moins deux canaux (14) débouche sur une perforation (4) et les au moins deux orifices inférieurs (16) des au moins deux canaux (14) débouchent à l'extérieur de la puce respectivement à au moins deux emplacements distincts **(Figure 32)**.

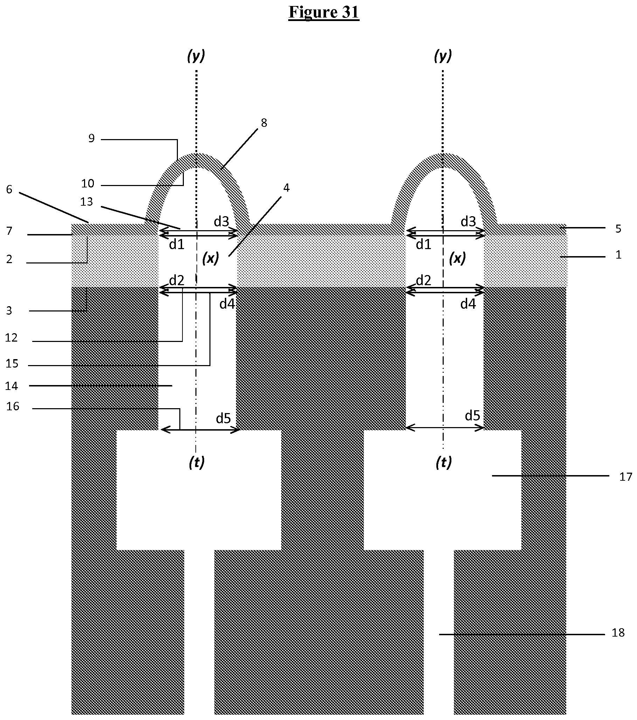

Selon un mode de réalisation particulier, chacun des orifices supérieurs (15) des au moins deux canaux (14) débouche sur une perforation (4) et les au moins deux orifices inférieurs (16) des au moins deux canaux (14) débouchent respectivement sur un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107) **(Figure 31)**.

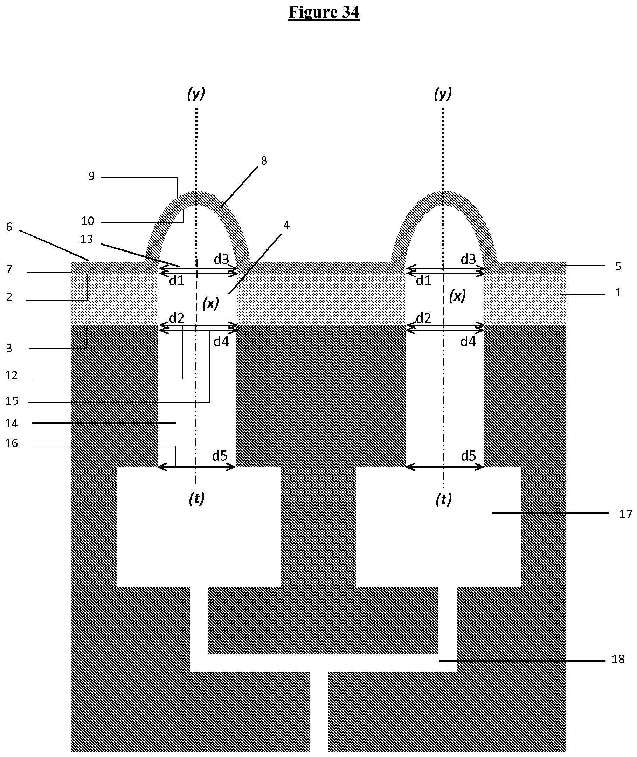

Selon un mode de réalisation particulier, chacun des orifices supérieurs (15) des au moins deux canaux (14) débouche sur une perforation (4) et les au moins deux orifices inférieurs (16) des au moins deux canaux (14) débouchent respectivement sur un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107), lesdits canaux de sortie de chacun desdits réservoirs étant connectés entre eux pour déboucher à l'extérieur de la puce au même emplacement **(Figure 34)**.

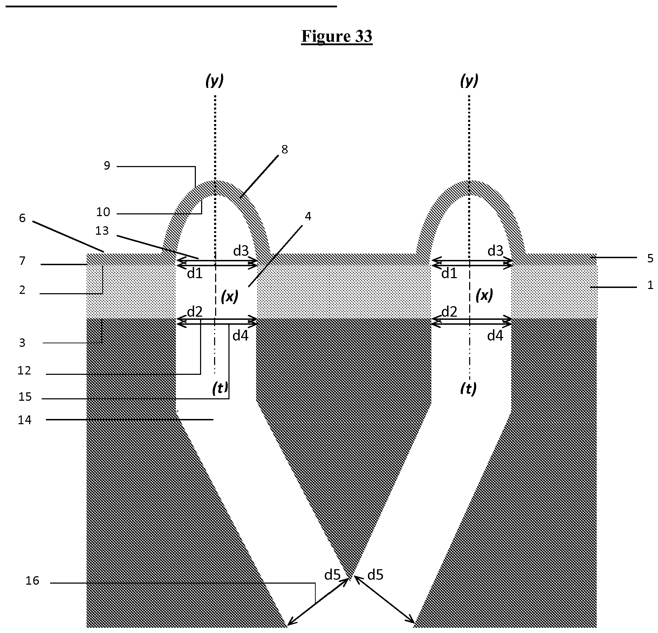

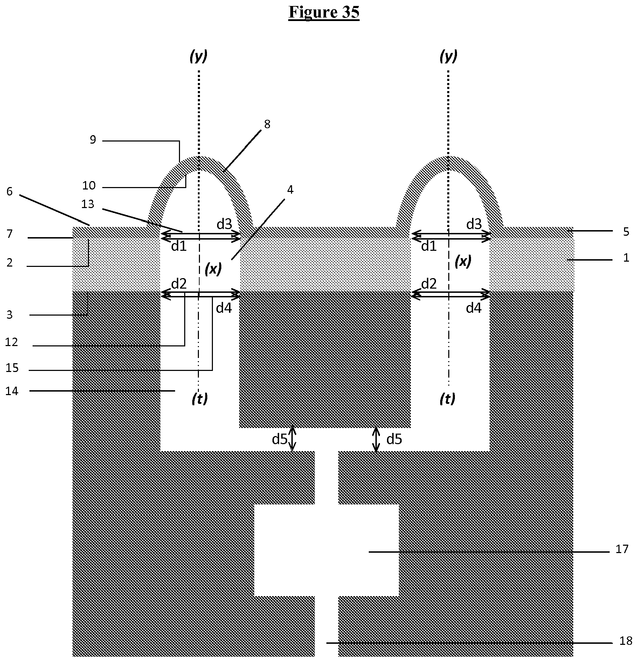

Selon un mode de réalisation particulier, les au moins deux canaux (14) sont connectés entre eux pour former un réseau, tel que chacun des orifices supérieurs (15) des au moins deux canaux (14) débouche sur une perforation (4) et l'orifice inférieur (16) de chacun des au moins deux canaux (14) débouchent eux-même à l'extérieur de la puce au même emplacement ou via un moyen intermédiaire constitué d'un réservoir (17) apte à récupérer des liquides et permettant un acheminement à l'extérieur de la puce via un canal de sortie (18) débouchant à l'extérieur du socle (109) dudit module inférieur (107) **(Figures 33 et 35)**.

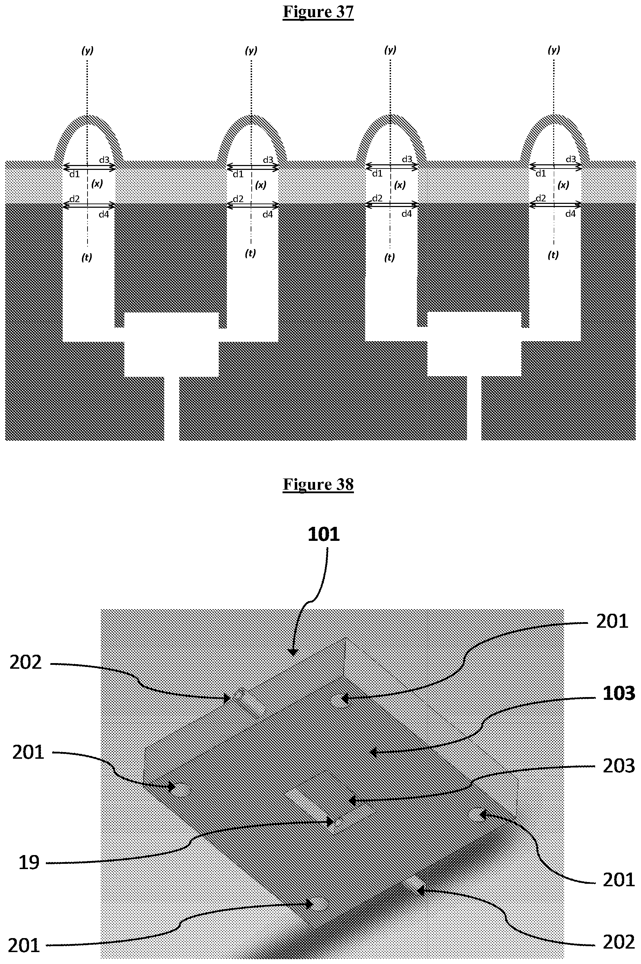

Selon un mode de réalisation particulier, chacun des orifices supérieurs (15) des au moins deux canaux (14) débouche sur une perforation (4)
et un premier ensemble d'au moins deux orifices inférieurs (16) des au moins deux canaux (14) débouchent sur un premier moyen intermédiaire constitué par un réservoir (17),
et un au moins deuxième ensemble d'au moins deux orifices inférieurs (16) des au moins deux canaux (14) débouchent sur au moins un deuxième moyen intermédiaire constitué par un réservoir (17),
lesdits premier et au moins deuxième réservoirs (17) étant aptes à récupérer des liquides et permettant chacun un acheminement à l'extérieur de la puce via des canaux de sortie (18) respectifs débouchant à l'extérieur du socle (109) dudit module inférieur (107), à des emplacements différents **(Figure 37)**.

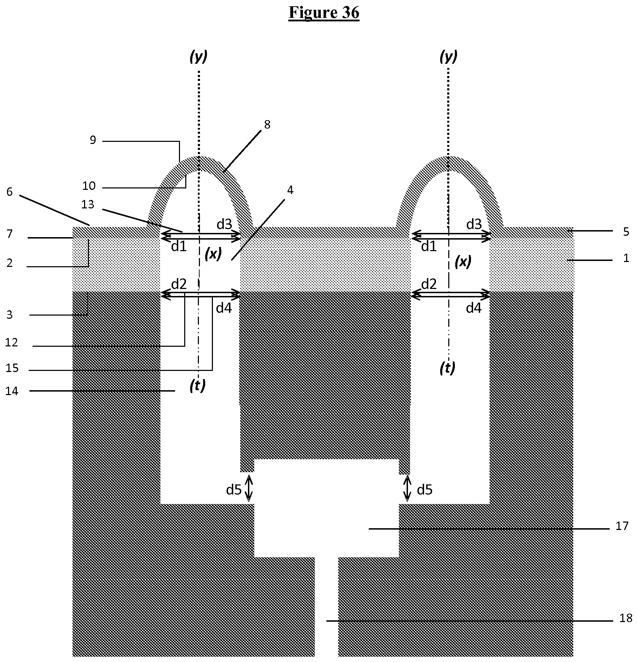

Selon un mode de réalisation particulier, chacun des orifices supérieurs (15) des au moins deux canaux (14) débouche sur une perforation (4) et les au moins deux orifices inférieurs (16) de l'ensemble des au moins deux canaux (14) débouchent sur un même moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107) **(Figure 36)**.

Selon un mode de réalisation particulier, ledit module inférieur (107) a une hauteur de 100 μm à 2 cm.

Selon un mode de réalisation particulier, ledit socle (109) du module inférieur (107) a une hauteur de 100 μm à 2 cm.

Selon un mode de réalisation particulier, ladite unité inférieure (108) a une hauteur de 100 μm à 2 cm.

Selon un mode de réalisation particulier, le module inférieur (107) est composé de plastique bioimprimé, de polycarbonate, de plastique de culture tissulaire ou de SU8.

Selon un mode de réalisation particulier, le module inférieur (107) est composé de plastique bioimprimé, de polycarbonate, de plastique de culture tissulaire, de verre ou de résine SU-8, et ledit le module inférieur (107) est transparent.

Selon un mode de réalisation particulier, le socle du module inférieur (109) est composé de plastique bioimprimé, de polycarbonate, de plastique de culture tissulaire ou de SU8.

Selon un mode de réalisation particulier, le socle du module inférieur (109) est composé de plastique bioimprimé, de polycarbonate, de plastique de culture tissulaire, de verre ou de résine SU-8, et ledit socle du module inférieur (109) est transparent.

Selon un mode de réalisation particulier, l'unité inférieure (108) est composée de plastique bioimprimé, de polycarbonate, de plastique de culture tissulaire ou de SU8.

Selon un mode de réalisation particulier, l'unité inférieure (108) est composée de plastique bioimprimé, de polycarbonate, de plastique de culture tissulaire, de verre ou de résine SU-8, et ladite unité inférieure (108) est transparente.

## MODULE SUPERIEUR

Selon un mode de réalisation particulier, la puce microfluidique de culture cellulaire contient un module supérieur (101) comprenant :

- une unité supérieure (102) solide et creuse, laquelle est constituée d'une surface pleine définissant un volume ouvert (chambre) (203), et les bords de la surface pleine étant aptes à être en contact avec ladite unité centrale (105) dudit module central (104) et délimitant la surface de l'ouverture dudit volume ouvert ;
- et un socle (103),

ladite unité supérieure (102) étant intégrée dans ledit socle (103), et formant un tout avec ledit socle (103).

La chambre (203) est intégrée dans le socle (103) du module supérieur (101), et se trouve ainsi entourée sur toutes ses surfaces par ledit socle (103), à l'exception de la surface du volume ouvert de ladite chambre (203).

Selon un mode de réalisation particulier, ladite unité supérieure (102) dudit module supérieur (101) et ladite unité centrale (105) dudit module central (104) sont assemblées de sorte que ladite surface de l'ouverture de ladite unité supérieure (102) est positionnée au dessus de ladite face supérieure (6) de la membrane poreuse nanostructurée en 3D (5) de ladite unité centrale (105), ladite surface de l'ouverture et ladite face supérieure (6) ayant une forme et une surface identiques entre elles.

Dans un mode de réalisation particulier, l'unité supérieure comporte une ouverture sur l'extérieur (fenêtre), au niveau de la surface pleine constituant ladite chambre permettant d'insérer une pièce en verre transparente compatible avec l'observation au microscope, de préférence une lame de microscope, afin d'observer la culture de cellules sur la face externe de la membrane poreuse nanostructurée en 3D et la face externe de la au moins une protubérance.

L'ouverture est située de préférence à un endroit de la surface pleine de ladite chambre, telle qu'elle permet l'observation à la verticale de la culture de cellules sur la face externe de la protubérance.

Ladite pièce en verre transparente est positionnée sur ladite ouverture de la surface pleine constituant ladite chambre, et maintenue de façon étanche à ladite unité supérieure pour maintenir un espace clos entre l'unité supérieure et l'unité centrale.

De préférence, ladite pièce en verre transparente est maintenue de façon étanche à ladite unité supérieure à l'aide d'une colle étanche.

Selon un mode de réalisation particulier, ladite unité supérieure (102) dudit module supérieur (101) et ladite unité centrale (105) dudit module central (104) sont assemblées par des éléments de fixation (201,204) situés sur chacun des socles (103,106) du module supérieur (101) et du module central (104), de façon à assembler de manière étanche ladite surface de l'ouverture de ladite unité supérieure (102) et ladite face supérieure (6) de la membrane poreuse nanostructurée en 3D (5) de ladite unité centrale (105).

Selon un mode de réalisation particulier, ladite unité supérieure (102) solide et creuse dans laquelle ladite surface pleine comporte une face rectangulaire pleine et quatre faces pleines adjacentes à ladite face rectangulaire pleine et adjacentes entre elles, formant ladite chambre (203), les bords libres desdites quatre faces pleines délimitant ladite surface de l'ouverture de ladite chambre (206).

Selon un mode de réalisation particulier, le module supérieur (101) est composé de plastique bioimprimé, de polycarbonate, de plastique de culture tissulaire ou de SU8.

Selon un mode de réalisation particulier, le module supérieur (101) est composé de plastique bioimprimé, de polycarbonate, de plastique de culture tissulaire, de verre ou de résine SU-8, et ledit module supérieur (101) est transparent.

Selon un mode de réalisation particulier, le socle du module supérieur (103) est composé de plastique bioimprimé, de polycarbonate, de plastique de culture tissulaire ou de SU8.

Selon un mode de réalisation particulier, le socle du module supérieur (103) est composé de plastique bioimprimé, de polycarbonate, de plastique de culture tissulaire, de verre ou de résine SU-8, et ledit socle du module supérieur (103) est transparent.

Selon un mode de réalisation particulier, l'unité supérieure (102) est composée de plastique bioimprimé, de polycarbonate, de plastique de culture tissulaire ou de SU8.

Selon un mode de réalisation particulier, l'unité supérieure (102) est composée de plastique bioimprimé, de polycarbonate, de plastique de culture tissulaire, de verre ou de résine SU-8, et ladite unité supérieure (102) est transparente.

Selon un mode de réalisation particulier, ladite surface de l'ouverture de ladite chambre (203) de ladite unité supérieure (102) est positionnée au dessus de ladite face supérieure (6) de la membrane poreuse nanostructurée en 3D (5) de ladite unité centrale (105) pour former un espace clos délimité par ladite surface pleine de ladite unité supérieure (102) et ladite face supérieure (6) de la membrane poreuse nanostructurée en 3D (5).

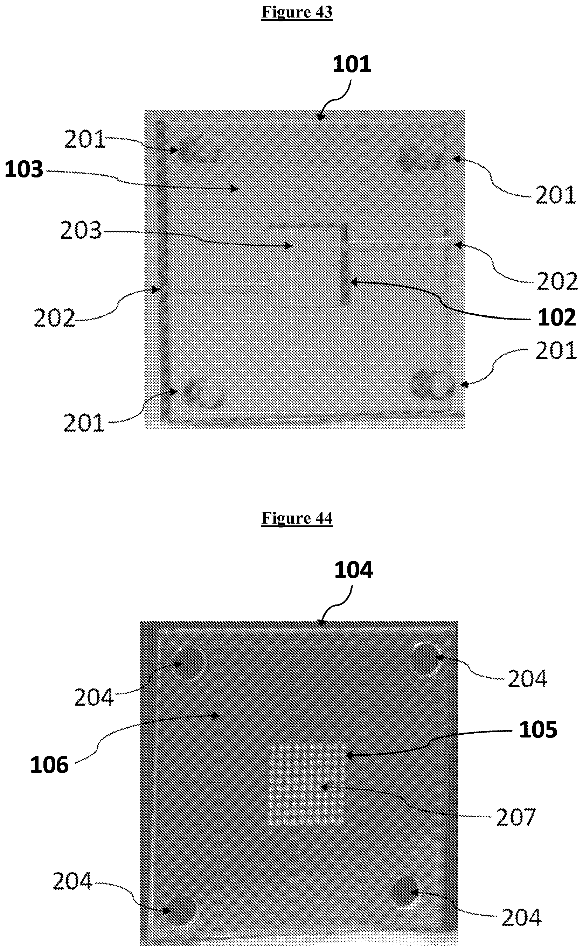

Selon un mode de réalisation particulier, ladite surface de l'ouverture de ladite chambre (203) de ladite unité supérieure (102) est positionnée au dessus de ladite face supérieure (6) de la membrane poreuse nanostructurée en 3D (5) de ladite unité centrale (105), et dans laquelle ladite au moins une face pleine de ladite unité supérieure (102) présente deux orifices débouchant respectivement sur un canal entrée/sortie (202),

pour former un espace clos délimité par ladite surface pleine de ladite unité supérieure (102), et ladite la face supérieure (6) de la membrane poreuse nanostructurée en 3D (5) de ladite unité centrale (105) pouvant communiquer avec l'extérieur de ladite puce microfluidique uniquement par lesdits deux orifices via lesdits canaux entrée/sortie (202) qui débouchent à l'extérieur du socle (109) **(Figures 38 et 43).**

L'unité supérieure comporte deux orifices débouchant respectivement sur un canal entrée/sortie. Les deux canaux entrée/sortie s'étendent à travers le socle du module supérieur, ladite unité et ledit socle formant une pièce d'un seul tenant, de telle sorte que lesdits deux canaux entrée/sortie débouchent à l'extérieur dudit socle supérieur.

Lorsque le module supérieur est assemblé au module central, l'assemblage de la chambre sur la membrane poreuse nanostructurée en 3D permet de définir un espace clos pouvant communiquer avec l'extérieur de ladite puce microfluidique uniquement par lesdits deux orifices via lesdits canaux entrée/sortie. Ces canaux permettent également l'introduction du milieu de culture dans la chambre, et éventuellement l'introduction des cellules qui sont cultivées sur la face supérieure de ladite membrane ou la face externe de la au moins une protubérance.

Ces canaux entrée/sortie peuvent être couplés à des capteurs permettant un suivi en continu des conditions et paramètres de culture (capteurs de CO2, O2, pression, température, glucose, pH). Dans un mode de réalisation alternatif, ces capteurs peuvent être directement intégrés dans la chambre (203).

Selon un mode de réalisation particulier, ledit module supérieur (101) a une hauteur de 50 μm à 2 cm, en particulier de 1 cm.

Selon un mode de réalisation particulier, ledit socle (103) dudit module supérieur (101) a une hauteur de 50 μm à 2 cm, en particulier de 1 cm.

Selon un mode de réalisation particulier, ladite unité supérieure (102) dudit module supérieur (101) a une hauteur de 50 μm à 2 cm, en particulier de 1 cm.

## ASSEMBLAGE DES TROIS MODULES

Selon un mode de réalisation particulier, la puce microfluidique de culture cellulaire, comporte un module supérieur (101), un module central (104) et un module inférieur (107), et dans laquelle les susdits modules sont assemblés tel que

ladite surface de l'ouverture de ladite unité supérieure (102) dudit module supérieur (101) est positionnée au dessus de ladite face supérieure (2) de la membrane poreuse nanostructurée en 3D (5) de ladite unité centrale (105) dudit module central (104), et l'orifice supérieur (15) du au moins un canal (14), de ladite face supérieure de ladite unité inférieure (108) dudit module inférieur (107), s'ouvre sur au moins une desdites perforations (4) dudit support (1) de ladite unité centrale (105),

ladite surface de l'ouverture de ladite unité supérieure (102) et ladite face supérieure (2) de la membrane poreuse nanostructurée en 3D (5) de ladite unité centrale (105) ayant une forme et une surface identiques entre elles,

ladite face supérieure de ladite unité inférieure (108) et ladite face inférieure dudit support (3) de ladite unité centrale (105) ayant une forme et une surface identiques entre elles,

et ledit module supérieur (101), ledit module central (104) et ledit module inférieur (107) étant assemblés par des éléments de fixation (201, 204) situés sur chacun des socles respectifs (103, 106, 109) **(Figures 42, 46 et 47).**

Les éléments de fixation situés sur les socles de chacun des modules permettent soit de verrouiller les modules deux à deux, pour assembler le module supérieur avec le module central et assembler le module central avec le module inférieur, soit de verrouiller deux modules sur le troisième module, pour assembler le module central et le module inférieur sur le module supérieur.

Ces éléments de fixation peuvent être des ergots ou éléments mâle-femelle, de préférence des éléments mâle-femelle.

Selon un mode de réalisation particulier, le socle du module supérieur comporte des plots (201) comme éléments de fixation mâle, et les socles des modules central et inférieur comportent des perforations (204) comme éléments de fixation femelle, aptes à se verrouiller sur lesdits plots du module supérieur.

Selon un mode de réalisation particulier, ladite face interne (10) de ladite protubérance (8) est en **totalité en regard** dudit orifice supérieur (15) dudit canal (14) : lorsque ladite face interne (10) de ladite au moins une protubérance (8) est **en totalité en regard de ladite au moins une perforation** (4) dudit support (1) :

- tel que lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus**; ou

- tel que la valeur du diamètre supérieur d1 de ladite au moins une perforation (4) est **supérieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

et lorsque ladite perforation (4) dudit support (1) est **en totalité en regard** dudit orifice supérieur (15) dudit canal (14) :

tel que l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont **confondus, ou distincts** l'un par rapport à l'autre d'une distance inférieure ou égale à [(valeur de d4 - valeur de d3)/2] lorsque la valeur de d4 est supérieure à la valeur de d2.

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou

- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus ou distincts l'un de l'autre d'une distance inférieure ou égale à [(valeur de d4 /2) + (4/10 de valeur de d3)].

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou

- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus ou distincts l'un de l'autre d'une distance inférieure ou égale à [[(valeur de d4 + valeur de d4) /2] - 10 $\mu$m].

Selon un mode de réalisation particulier, ladite face interne (10) de ladite protubérance (8) est en **partie en regard** dudit orifice supérieur (15) dudit canal (14) : lorsque ladite face interne (10) de ladite au moins une protubérance (8) est **en totalité en regard de ladite au moins une perforation** (4) dudit support (1) :

- tel que lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus**; ou

- tel que la valeur du diamètre supérieur d1 de ladite au moins une perforation (4) est **supérieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont **confondus ou distincts** l'un de l'autre d'une distance inférieure ou égale à [valeur de d4 /2].

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en partie en regard de ladite au moins une perforation (4) dudit support (1) :

- tel que la valeur du diamètre supérieur d1 de ladite au moins une perforation (4) est inférieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus, ou distincts l'un de l'autre d'une valeur inférieure ou égale à [(valeur de d1 /2) + (4/10 de valeur de d3)].

et ledit axe (x) et l'axe (t), passant par le centre dudit orifice supérieur (15) dudit canal (14) et perpendiculaire audit support (1), sont confondus lorsque la valeur du diamètre d2 est égal ou supérieur à la valeur du diamètre d4, ou sont distincts l'un par rapport à l'autre d'une distance d'une valeur inférieure ou égale à [(valeur de d4 - valeur de d1)/2], lorsque la valeur du diamètre d4 est supérieur à la valeur du diamètre d2.

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en partie en regard de ladite au moins une perforation (4) dudit support (1) :

- tel que la valeur du diamètre supérieur d1 de ladite au moins une perforation (4) est inférieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus, ou distincts l'un de l'autre d'une valeur inférieure ou égale à [[(valeur de d3 + valeur de d1) /2] - 10 $\mu$m].

et ledit axe (x) et l'axe (t), passant par le centre dudit orifice supérieur (15) dudit canal (14) et perpendiculaire audit support (1), sont confondus lorsque la valeur du diamètre d2 est égal ou supérieur à la valeur du diamètre d4, ou sont distincts l'un par rapport à l'autre d'une distance d'une valeur inférieure ou égale à [(valeur de d4 - valeur de d1)/2], lorsque la valeur du diamètre d4 est supérieur à la valeur du diamètre d2.

Selon un mode de réalisation particulier, ladite face interne (10) de ladite protubérance (8) est en **partie en regard** dudit orifice supérieur (15) dudit canal (14) : lorsque ladite face interne (10) de ladite au moins une protubérance (8) est **en partie en regard de ladite au moins une perforation (4)** dudit support (1) :

- tel que la valeur du diamètre supérieur d1 de ladite au moins une perforation (4) est **inférieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus, ou distincts l'un de l'autre d'une valeur inférieure ou égale à [(valeur de d3 - valeur de d1)/2],**

**et** ledit axe (x) et l'axe (t), passant par le centre dudit orifice supérieur (15) dudit canal (14) et perpendiculaire audit support (1), sont **confondus lorsque** la valeur du diamètre d2 est égal ou supérieur à la valeur du diamètre d4, **ou** sont distincts l'un par rapport à l'autre d'une distance d'une valeur **inférieure ou égale à [(valeur de d4 - valeur de d1)/2], lorsque** la valeur du diamètre d4 est supérieur à la valeur du diamètre d2.

Selon un mode de réalisation particulier, ladite face interne (10) de ladite protubérance (8) est en **partie en regard** dudit orifice supérieur (15) dudit canal (14) : lorsque ladite face interne (10) de ladite au moins une protubérance (8) est **en partie en regard de ladite au moins une perforation (4)** dudit support (1) :

- tel que la valeur du diamètre supérieur d1 de ladite perforation (4) est **égale à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont

**distincts l'un par rapport à l'autre d'une distance** d'une valeur inférieure [valeur de d1/2],

**et** ledit axe (x) et l'axe (t), passant par le centre dudit orifice supérieur (15) dudit canal (14) et perpendiculaire audit support (1), sont **confondus lorsque** la valeur du diamètre d2 est égal ou supérieur à la valeur du diamètre d4,

**ou** sont distincts l'un par rapport à l'autre d'une distance d'une valeur **inférieure ou égale à [(valeur de d4 - valeur de d1)/2], lorsque** la valeur du diamètre d4 est supérieur à la valeur du diamètre d2.

Selon un mode de réalisation particulier, ladite face interne (10) de ladite protubérance (8) est en **partie en regard** dudit orifice supérieur (15) dudit canal (14) : lorsque ladite face interne (10) de ladite au moins une protubérance (8) est **en partie en regard de ladite au moins une perforation (4)** dudit support (1) :

- tel que la valeur du diamètre supérieur d1 de ladite perforation (4) est **supérieure à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3) /2],

**et** ledit axe (x) et l'axe (t), passant par le centre dudit orifice supérieur (15) dudit canal (14) et perpendiculaire audit support (1), sont **confondus,**

**ou** ledit axe (x) et ledit axe (y) sont **confondus.**

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en partie en regard de ladite au moins une perforation (4) dudit support (1) :

- tel que la valeur du diamètre supérieur d1 de ladite perforation (4) est supérieure à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à sont confondus, ou distincts l'un de l'autre d'une valeur inférieure ou égale à [(valeur de d1 /2) + (4/10 de valeur de d3)]

et ledit axe (x) et l'axe (t), passant par le centre dudit orifice supérieur (15) dudit canal (14) et perpendiculaire audit support (1), sont distincts l'un par rapport à l'autre d'une distance tel que au moins 1/10 du diamètre d3 soit en totalité en regard du diamètre d4.

Selon un mode de réalisation particulier, ladite face interne (10) de ladite protubérance (8) est en partie en regard dudit orifice supérieur (15) dudit canal (14) : lorsque ladite face interne (10) de ladite au moins une protubérance (8) est en partie en regard de ladite au moins une perforation (4) dudit support (1) :

- tel que la valeur du diamètre supérieur d1 de ladite perforation (4) est supérieure à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à sont confondus, ou distincts l'un de l'autre d'une valeur inférieure ou égale à [[(valeur de d3 + valeur de d1) /2] - 10 $\mu$m]

et ledit axe (x) et l'axe (t), passant par le centre dudit orifice supérieur (15) dudit canal (14) et perpendiculaire audit support (1), sont distincts l'un par rapport à l'autre d'une distance tel que au moins 10 $\mu$m du diamètre d3 soit en totalité en regard du diamètre d4.

Dans un mode de réalisation, l'unité centrale contient :

- un support constitué d'une membrane non résorbable (1), comportant une face supérieure (2) et une face inférieure (3), perforé par au moins une perforation (4)

- une membrane poreuse nanostructurée en 3D (5) comportant une face supérieure (6) et une face inférieure (7), et comportant au moins une protubérance (8), ladite au moins une protubérance comportant une face externe (9) et une face interne (10) et formant une structure en relief du côté de la face supérieure (6) de la membrane nanostructurée en 3D (5), ladite protubérance étant notamment en forme de dôme creux présentant une base circulaire (13),

ladite face supérieure (6) de la membrane nanostructurée en 3D étant positionnée de façon solidaire sur ladite face inférieure (2) dudit support et ladite au moins une protubérance (8) étant du côté de la face supérieure (2) dudit support (1).

Ainsi, selon un mode particulier, ladite unité supérieure (102) dudit module supérieur (101) et ladite unité centrale (105) dudit module central (104) sont assemblées de sorte que ladite surface de l'ouverture de ladite unité supérieure (102) est positionnée au-dessus de ladite face supérieure (2) du support constitué d'une membrane non résorbable (1) de ladite unité centrale (105),

pour former un espace clos délimité par ladite surface pleine de ladite unité supérieure (102), ladite face supérieure (2) du support constitué d'une membrane non résorbable (1) et la face externe (9) des protubérances,

ladite surface de l'ouverture et ladite face supérieure (2) ayant une forme et une surface identiques entre elles,

ladite unité supérieure (102) dudit module supérieur (101) et ladite unité centrale (105) dudit module central (104) étant assemblées par des éléments de fixation (201,204) situés sur chacun des socles (103,106) du module supérieur (101) et du module central (104), de façon à assembler de manière étanche ladite surface de l'ouverture de ladite unité supérieure (102) et ladite face supérieure (2) du support constitué d'une membrane non résorbable (1) de ladite unité centrale (105),

et avantageusement ladite au moins une face pleine de ladite unité supérieure (102) présente deux orifices débouchant respectivement sur un canal entrée/sortie (202), permettant audit espace clos de communiquer avec l'extérieur de ladite puce microfluidique via lesdits canaux entrée/sortie (202) qui débouchent à l'extérieur du socle (109).

Selon un mode de réalisation particulier, ladite face interne (10) de ladite au moins une protubérance (8) est en totalité ou en partie en regard de ladite au moins une perforation (4) dudit support (1).

La membrane nanostructurée en 3D comportant au moins une protubérance et le support perforé par au moins une perforation sont positionnés l'un par rapport de façon à ce que la face interne de la dite protubérance soit en totalité ou en partie en regard de la section circulaire de ladite perforation au niveau de la face supérieure du support.

Selon un mode de réalisation particulier, ladite face interne (10) de ladite au moins une protubérance (8) est **en totalité en regard de ladite au moins une perforation** (4) dudit support (1) :

- lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus;** ou
- lorsque la valeur du diamètre supérieur d1 de ladite au moins une perforation (4) est **supérieure à** la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

Selon un mode particulier, la puce microfluidique de culture cellulaire, comporte un module supérieur (101), un module central (104) et un module inférieur (107), et dans laquelle les susdits modules sont assemblés tel que

ladite surface de l'ouverture de ladite unité supérieure (102) dudit module supérieur (101) est positionnée au-dessus de ladite face supérieure (2) du support constitué d'une membrane non résorbable (1) et de la face externe (9) de ladite au moins un protubérance (8) de ladite unité centrale (105) dudit module central (104),

et l'orifice supérieur (15) du au moins un canal (14), de ladite face supérieure de ladite unité inférieure (108) dudit module inférieur (107), s'ouvre sur au moins une desdites perforations (4) dudit support (1) de ladite unité centrale (105),

ladite surface de l'ouverture de ladite unité supérieure (102) et ladite face supérieure (2) du support constitué d'une membrane non résorbable (1) de ladite unité centrale (105) ayant une forme et une surface identiques entre elles,

ladite face supérieure de ladite unité inférieure (108) et ladite face inférieure dudit support (3) de ladite unité centrale (105) ayant une forme et une surface identiques entre elles,

et ledit module supérieur (101), ledit module central (104) et ledit module inférieur (107) étant assemblés par des éléments de fixation (201, 204) situés sur chacun des socles respectifs (103, 106, 109)

dans laquelle ladite face interne (10) de ladite protubérance (8) est en totalité en regard dudit orifice supérieur (15) dudit canal (14) :
lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou
- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2] ;

et lorsque ladite perforation (4) dudit support (1) est en totalité en regard dudit orifice supérieur (15) dudit canal (14) :
tel que l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus, ou distincts l'un par rapport à l'autre d'une distance inférieure ou égale à [(valeur de d4 - valeur de d3)/2] lorsque la valeur de d4 est supérieure à la valeur de d2.

La puce microfluidique de culture cellulaire peut être fabriquée par un procédé de fabrication, dans lequel la fabrication de ladite unité centrale (105) dudit module central (104) comprend :

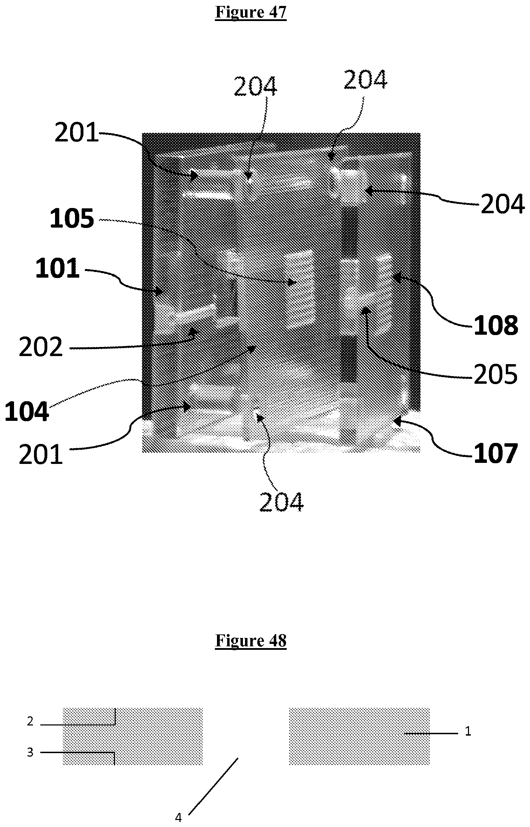

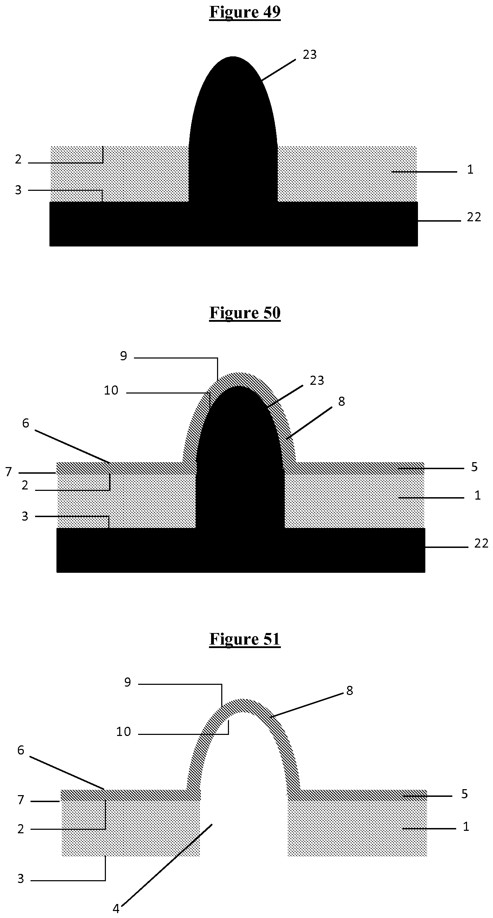

- une étape d'extrusion d'une solution de polymère résorbable (22) à travers la au moins une perforation (4) dudit support (1) pour former au moins une nanostructure 3D (23) du côté de ladite face supérieure (2) dudit support (1) **(Figures 48 et 49),** suivi de

- une étape de polymérisation de ladite solution de polymère résorbable pour rigidifier ladite au moins une nanostructure 3D (23), ladite au moins une nanostructure 3D (23) formant un moule en polymère résorbable (22) du côté de ladite face supérieure dudit support après polymérisation, suivi par

- une étape d'application d'au moins une couche continue d'au moins un polyélectrolyte recouvrant la face supérieure (2) dudit support (1) et la surface dudit moule en polymère résorbable (22), pour constituer la membrane poreuse nanostructurée en 3D (5) **(Figure 50),** par

- une étape de dissolution dudit moule en polymère résorbable pour obtenir ladite face inférieure (7) de ladite membrane poreuse nanostructurée en 3D (5) positionnée de façon solidaire sur ladite face supérieure (2) dudit support (1) et ladite face interne (10) de la au moins une protubérance (8) positionnée en totalité en regard de ladite au moins une perforation (4) **(Figure 51)**.

La première étape de fabrication du module central est une étape d'extrusion d'une solution polymérique résorbable à travers une ou des perforations dudit support constitué d'un matériau non résorbable, c'est-à-dire un matériau ne pouvant être éliminé ni par un procédé physique ni par un procédé chimique dans un solvant aqueux.

Le matériau polymérique résorbable utilisé pour la solution est initialement sous forme de liquide visqueux pour permettre l'extrusion à travers les perforations du support.

Le terme « visqueux » est utilisé ici pour qualifier un fluide qui a une résistance à la déformation sous une contrainte de cisaillement, qui est au moins la même que celle de l'eau pure et préférablement sensiblement plus grande que celle de l'eau pure. En d'autres termes, un liquide visqueux utilisé pour l'extrusion circule plus lentement que l'eau pure, propriété due à sa plus grande viscosité que l'eau pure. La propriété de viscosité du liquide utilisé pour l'extrusion est importante, car un tel liquide visqueux permet une meilleure conservation de la forme de la protubérance, pendant le processus d'extrusion, qu'une solution qui a la viscosité de l'eau pure. La plage préférée de la viscosité du liquide pour l'extrusion est comprise de 1 (eau pure) à 100 Pa.s, et de préférence, la viscosité du liquide est comprise de 0,1 Pa.s à 1 Pa.s.

Le terme « extrusion » est ici utilisé pour définir une étape dans laquelle un matériau sous forme de liquide visqueux est passé en force à travers une matrice pour donner une forme prédéterminée par ladite matrice, audit matériau visqueux. Dans le cadre de la présente invention, la matrice est constituée par le support comportant au moins une perforation et ledit matériau à l'état de liquide visqueux est constitué par le polymère résorbable. L'extrusion de ce polymère résorbable à l'état visqueux, de la face inférieur dudit support vers la face extérieure dudit support, au travers de la au moins une perforation, permet de donner une forme de nanostructure en 3D audit polymère résorbable du coté de la face supérieure dudit support.

Selon un mode de réalisation particulier, l'étape d'extrusion de ladite solution en polymère résorbable (22) à travers la au moins une perforation (4) dudit support (1), permet de former des nanostructures 3D (23) du coté de ladite face supérieure (2) dudit support (1) **en forme de dôme.**

La nanostructure 3D obtenue après l'extrusion à travers les perforations et la polymérisation de ladite solution polymérique résorbable, a pour effet technique de servir de moule pour la formation des protubérances de la membrane poreuse nanostructurée en 3D.

L'extrusion est arrêtée lorsque la solution polymérique résorbable a formé une nanostructure 3D de longueur désirée pour servir de moule à la protubérance souhaitée.

La solution polymérique résorbable est alors laissée sans contrainte pour permettre sa polymérisation ou gélification, et ainsi donner des nanostructures 3D solides qui dépassent du coté de la face supérieur dudit support à travers les perforations.

La polymérisation est réalisée directement sur le support constitué d'une membrane non résorbable et comportant au moins une perforation.

Après polymérisation, les nanostructures 3D obtenues permettent de constituer un moule pour l'obtention des protubérances de la membrane poreuse nanostructurée en 3D.

La surface formée par la face supérieure du support et les nanostructures en 3D formées par extrusion et dépassant du coté de la face supérieure dudit support à travers les perforations, est ensuite recouverte par une membrane poreuse constituée d'un film en polyélectrolytes.

Les polyélectrolytes ont la propriété de pouvoir prendre la forme de tout type de structure 3D constitué de n'importe quel type de matériau (même résorbable), et de conserver la forme 3D ainsi donnée, lors du retrait de la structure 3D ayant servi de moule.

Ce film en polyélectrolytes est un film multicouches, réalisé en utilisant la technique couche-par-couche.

Le but de cette technique est d'adsorber des couches successives de polyélectrolytes sur la surface formée par la face supérieure du support et les nanostructures en 3D dépassant à travers les perforations dudit support, pour créer une pluralité de fines couches de polyélectrolytes.

De manière préférentielle, les polyélectrolytes utilisés sont le PAH et le PSS. Les solutions de PAH et de PSS sont préparées à une concentration de 1 mg/ml avec 0.5 mol/l de NaCl.

Le film multicouches de polyélectrolytes est construit en commençant indifféremment par l'un des polyélectrolytes et en terminant indifféremment par l'un des polyélectrolytes, mais en tenant compte de l'alternance des couches entre des polyélectrolytes de charge opposée.

Entre les changements de solution de polyélectrolytes, la surface est rincée rigoureusement avec de l'eau MilliQ (18 MQ.cm).

Ce procédé est répété jusqu'à obtenir le nombre désiré de couches de polyélectrolytes.

Ce procédé permet d'obtenir un film multicouches en polyélectrolytes final qui constitue un film continu sur la totalité de la surface formée par la face supérieure du support et les nanostructures en 3D dépassant à travers les perforations dudit support.

Ainsi, ce film multicouches en polyélectrolytes final présente des protubérances obtenues par moulage lors de l'application des couches successives de polyélectrolytes sur les nanostructures 3D issues de l'extrusion au travers des perforations du support.

Une fois que la surface formée par la face supérieure du support et les nanostructures en 3D, est recouverte par le film continu multicouches en polyélectrolytes, le moule formé par les nanostructures 3D en matériau polymérique résorbable est dissout.

Il est à noter que l'utilisation d'un support constitué d'un matériau non résorbable, c'est-à-dire un matériau ne pouvant être éliminé ni par un procédé physique ni par un procédé chimique dans un solvant aqueux, permet de conserver ledit support intact à l'issu de cette étape de dissolution.

La dissolution du matériel polymérique résorbable donne un film continu multicouches en polyélectrolytes fermement lié au support, et qui présentent des protubérances conservant la forme des nanostructures 3D sur lesquelles elles ont été moulées. Ce film continu constitue ainsi la membrane poreuse nanostructurée en 3D.

La structure finale composée de la membrane nanostructurée poreuse en 3D fixée sur le support, est facilement manipulable et forme le module central de ladite puce microfluidique de culture cellulaire.

Ce module central peut être désinfecté en lavant trois fois consécutivement avec de l'éthanol à 70%, puis en lavant trois à quatre fois consécutivement avec du milieu de culture cellulaire pour enlever toute trace résiduelle d'éthanol.

Il est également possible de stériliser le module central en utilisant du gaz d'oxyde d'éthylène.

Selon un mode de réalisation particulier, l'étape d'extrusion de ladite solution en polymère résorbable (22) à travers la au moins une perforation (4) dudit support (1) est réalisée à **l'aide d'un système de pompage.**

Cette extrusion de la solution polymérique résorbable est réalisée à l'aide d'un système de pompage qui peut être contrôlé manuellement ou mécaniquement (par un moteur).

Le système de pompe peut être constitué par exemple par une seringue ou un piston ou pour induire un flux continu de ladite solution de polymère résorbable (22) à travers ladite au moins une perforation (4).

Dans un autre mode de réalisation, ladite solution de polymère résorbable (22) peut être extrudée à travers ladite au moins une perforation (4) à l'aide de deux plaques plates parallèles qui peuvent être déplacées l'une vers l'autre, soit manuellement, soit mécaniquement, à l'aide d'un moteur par exemple. L'une des plaques plates est positionnée sur la face externe du module supérieur (204) et l'autre plaque plate est positionnée sur la face externe du module inférieur (101), les deux plaques plates étant ainsi positionnées de manière parallèle l'une par rapport à l'autre. Le mouvement desdites plaques plates et parallèles, l'une vers l'autre permet ainsi d'appliquer une force à ladite solution de polymère résorbable (22). Dans un tel cas, la force appliquée à la solution de polymère résorbable (22) oblige ladite solution de polymère résorbable (22) à circuler à travers ladite au moins une perforation (4).

L'extrusion peut être réalisée dans un liquide ou dans l'air.

Selon un mode de réalisation particulier, l'étape d'extrusion de ladite solution en polymère résorbable (22) à travers la au moins une perforation (4) dudit support (1), est réalisé **dans un liquide.**

Dans le cas de l'extrusion dans un liquide, le liquide est choisi de manière à ne pas provoquer la résorption du polymère utilisé pour former la protubérance.

Dans le cas d'une nanostructure 3D extrudée dans un liquide, lorsque **le liquide est au dessus de la face supérieure du support,** cela entraine une pression statique de fluide qui constitue une force dirigée en sens opposé au développement en longueur de la nanostructure 3D, et agit ainsi à l'encontre du développement en longueur de la nanostructure 3D. Cette magnitude de la pression statique de fluide est calculée en multipliant la densité ($\rho$) du liquide par la profondeur ($t$) du liquide au dessus de la face supérieure du support et par la force de gravité ($g$), soit sous forme de formule $\rho tg$. Cette magnitude de la pression statique de fluide agit de manière à réduire la hauteur de la nanostructure 3D. Par exemple, pour une profondeur de 1 mm d'une solution de sel dilué (densité = 1), solution utilisée dans le cas de mimétisme des fluides extracellulaires du corps, la magnitude de la pression statique de fluide qui agit contre la formation en longueur de la nanostructure 3D est de 9.8 milliPascals.

Comme le module de Young de la solution de polyélectrolyte utilisée en tant que polymère résorbable pour l'extrusion, est en général de l'ordre de 100 à 400 MegaPascals, il y a alors une réduction négligeable de la longueur de la nanostructure 3D formée durant l'extrusion (par exemple, dans le pire des cas, pour un module de Young de 100 MegaPascals, la réduction de la longueur sera de l'ordre de $10^{-5}$%).

Dans le cas d'une nanostructure 3D extrudée dans un liquide, **si le liquide est en dessous de la face inférieure du support,** il n'y a alors pas de force de résistance statique de fluide qui agit à l'encontre de la formation en longueur de la nanostructure 3D. Dans ce cas, la protubérance atteint la même longueur qu'une nanostructure 3D extrudée dans l'air.

Selon un mode de réalisation particulier, l'étape d'extrusion de ladite solution en polymère résorbable (22) à travers la au moins une perforation (4) dudit support (1), est réalisé **dans l'air.**

Dans le cas d'une nanostructure 3D extrudée dans l'air, il n'y a pas de pression agissant à l'encontre de la formation en longueur de la nanostructure 3D.

Un procédé de fabrication alternatif de la puce microfluidique de culture cellulaire peut être tel que la fabrication de ladite unité centrale (105) dudit module central (104) comprend :

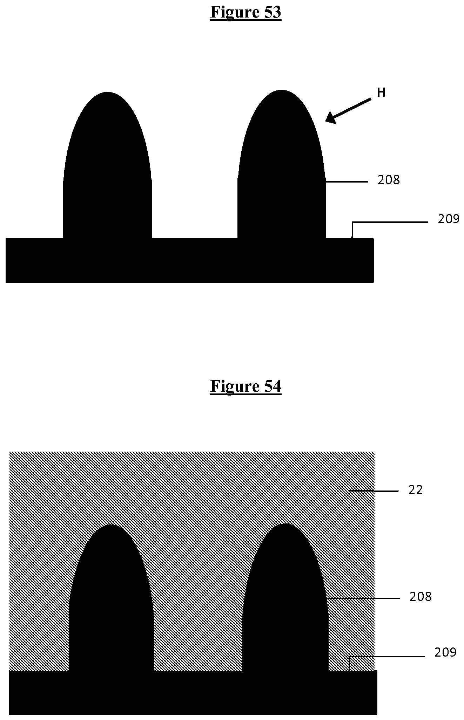

- une étape de coulage d'une solution de polymère résorbable (22) sur la face supérieure (209) d'un moule (H), ledit moule étant en particulier en plastique, comprenant au moins une nanostructure 3D moulée (208) sur ladite face supérieure (209), (figures 53 et 54) suivi de

- une étape de polymérisation de ladite solution de polymère résorbable (22) pour rigidifier ladite solution de polymère résorbable et former une matrice en polymère résorbable (210), suivi de

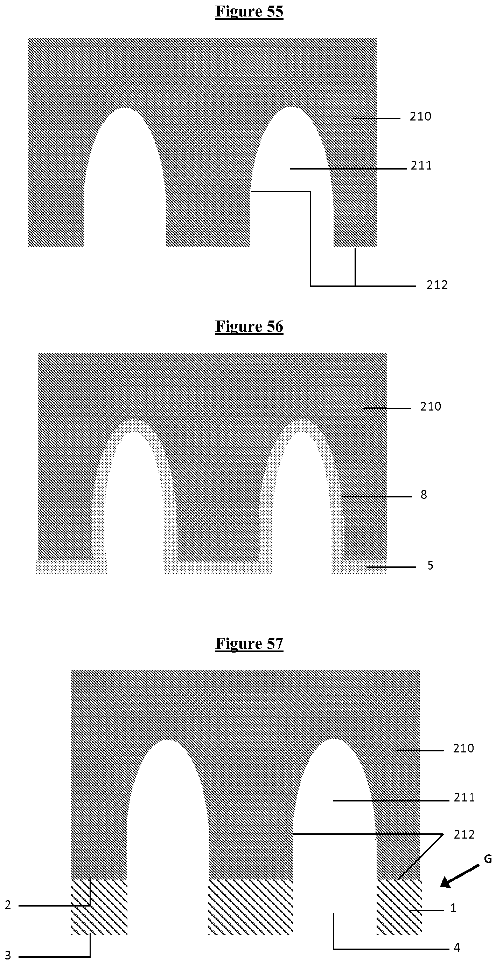

- une étape de retrait dudit moule pour former une matrice en polymère résorbable (210) comportant au moins un moule négatif de ladite au moins une nanostructure 3D (211), (figure 55), suivi de

- une étape d'application d'au moins une couche continue d'au moins un polyélectrolyte sur la face inférieure (212) de ladite matrice en polymère résorbable (210) comportant au moins un moule négatif de la dite au moins une nanostructure 3D (211), pour constituer une membrane poreuse nanostructurée en 3D (5) comprenant au moins une protubérance (8) (Figure 56), suivi de

- une étape de dissolution de ladite matrice en polymère résorbable.

Selon un mode particulier, la fabrication de ladite unité centrale (105) dudit module central (104) comprend :

- une étape de coulage d'une solution de polymère résorbable (22) sur la face supérieure (209) d'un moule (H), ledit moule étant en particulier en plastique, comprenant au moins une nanostructure 3D moulée (208) sur ladite face supérieure (209), (figures 53 et 54) suivi de

- une étape de polymérisation de ladite solution de polymère résorbable (22) pour rigidifier ladite solution de polymère résorbable et former une matrice en polymère résorbable (210), suivi de

- une étape de retrait dudit moule (H) pour former une matrice en polymère résorbable (210) comportant au moins un moule négatif de ladite au moins une nanostructure 3D (211), (figure 55), suivi de

- une étape d'assemblage de ladite matrice en polymère résorbable (210) comportant au moins un moule négatif de ladite au moins une nanostructure 3D (211) avec un support constitué d'une membrane non résorbable (1) comportant au moins une perforation (4), de manière à ce que ledit au moins un moule négatif de ladite au moins une nanostructure 3D (211) soit dans l'alignement de ladite au moins une perforation (4) dudit support (1), (figure 57) suivi de

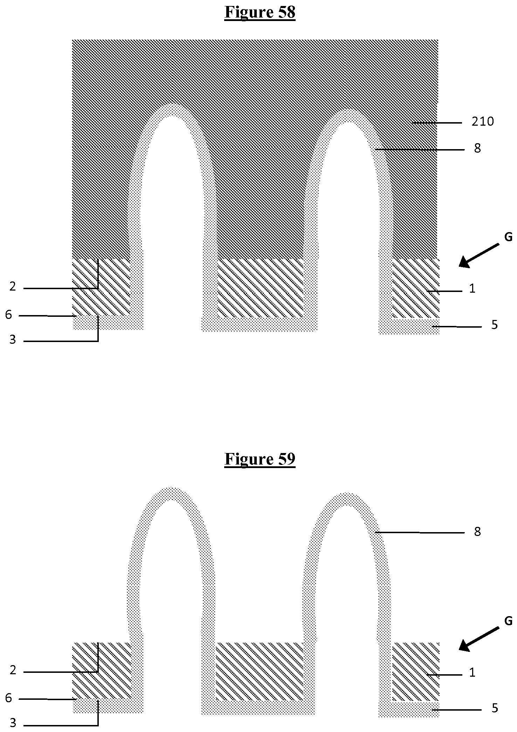

- une étape d'application d'au moins une couche continue d'au moins un polyélectrolyte sur la surface continue formée par la face inférieure (3) dudit support (1) et la face inférieure (212) de ladite matrice en polymère résorbable (210) comportant au moins un moule négatif de la dite au moins une nanostructure 3D (211) au niveau des perforations (4) dudit support (1), pour constituer une membrane poreuse nanostructurée en 3D (5) comprenant au moins une protubérance (8), (figure 58) suivi de

- une étape de dissolution de ladite matrice en polymère résorbable (210) (figure 59).

Selon un mode particulier, la fabrication du module central comprenant l'unité centrale, comprend :

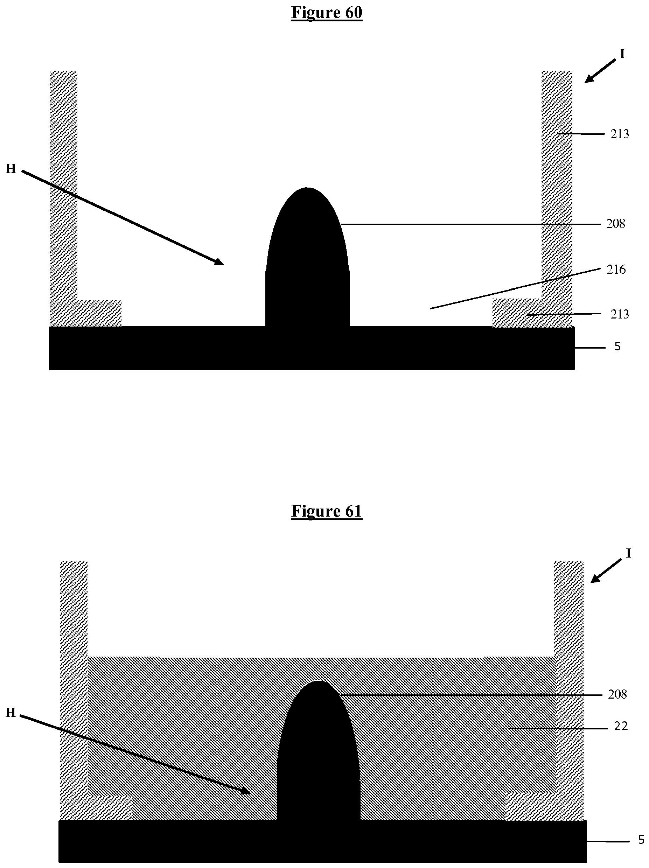

(i) une étape d'assemblage d'une pièce de soutien (I, i), constituée d'un cadre latéral (213), d'une face supérieure ouverte (214) et d'une face inférieure pleine (215) comportant une découpe (216) au format de l'unité centrale, et d'un moule (H, H1, H2, h1, h2), aux formes et dimensions de ladite pièce de soutien (I, i), comprenant au moins une nanostructure 3D moulée (208) sur la face supérieure (209), ledit moule étant en particulier en plastique (figure 60),

(ii) une étape de coulage d'une solution de polymère résorbable (22) sur ladite face supérieure (209) dudit moule (H, H1, H2, h1, h2), (figure 61) suivi de

(iii) une étape de polymérisation de ladite solution de polymère résorbable (22) pour rigidifier ladite solution de polymère résorbable et former une matrice en polymère résorbable (210) comportant au moins un moule négatif de ladite au moins une nanostructure 3D (211), suivi de

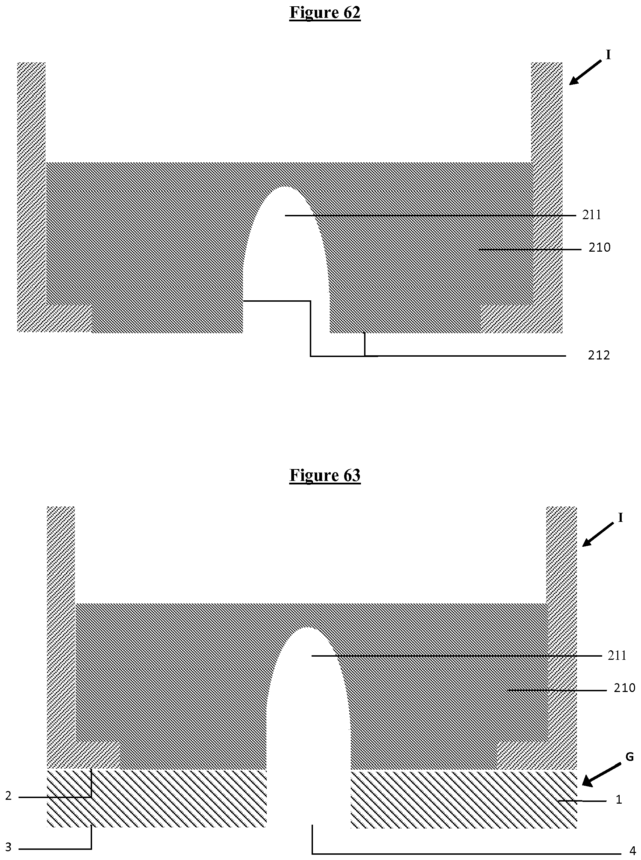

(iv) une étape de retrait dudit moule (H, H1, H2, h1, h2), pour obtenir ladite matrice en polymère résorbable (210) comportant au moins un moule négatif de ladite au moins une nanostructure 3D (211) formée dans ladite découpe (216) de la face inférieure pleine (215) de ladite pièce de soutien (I, i), (figure 62) suivi de

(v) une étape d'assemblage de ladite pièce de soutien (I, i), avec une pièce perforée (G, G1, G2, g1, g2), comprenant un support constitué d'une membrane non résorbable (1) perforé par au moins une perforation (4) intégré dans un socle (106), (figure 63) ladite pièce perforée (G, G1, G2, g1, g2) étant aux formes et dimensions de ladite pièce de soutien (I, i), et le nombre de perforations (4) de ladite pièce perforée (G, G1, G2, g1, g2) étant identique au nombre de nanostructures 3D moulées (211) dans ledit moule (H, H1, H2, h1, h2) utilisé à l'étape (i), de manière à ce que ladite au moins une perforation (4) dudit support (1) soit dans l'alignement avec ledit au moins un moule négatif de ladite au moins une nanostructure 3D (211),
suivi de

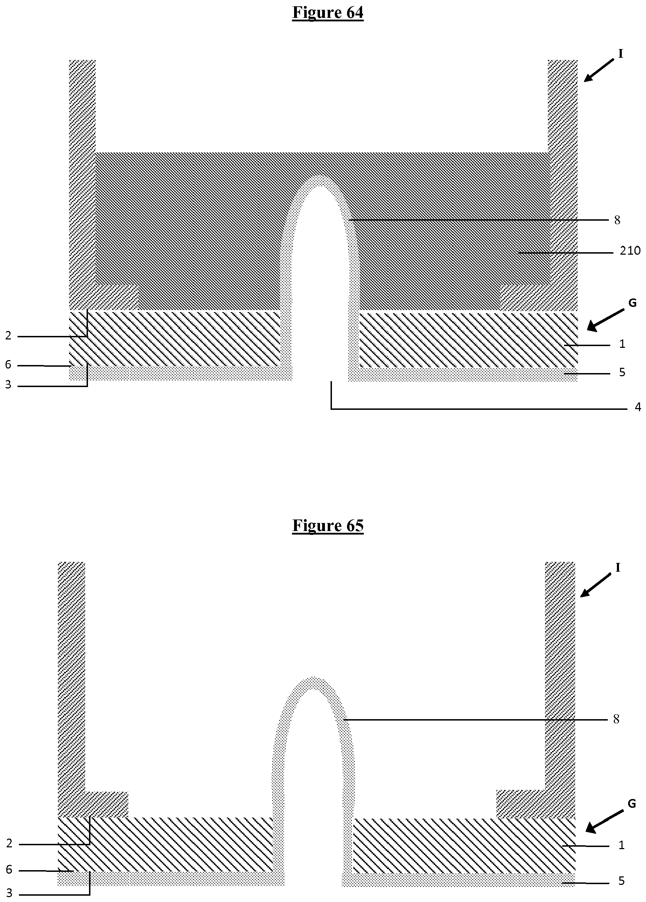

(vi) une étape d'application d'au moins une couche continue d'au moins un polyélectrolyte sur la surface continue constituée de la face inférieure (3) dudit support (1) et de la face inférieure (212) de ladite matrice en polymère résorbable (210) comportant au moins un moule négatif de la dite au moins une nanostructure 3D (211), au niveau de ladite au moins une perforation (4) dudit support (1), pour constituer une membrane poreuse nanostructurée en 3D (5) comportant au moins une protubérance (8) (figure 64), suivi de

(vii) une étape de dissolution de ladite matrice en polymère résorbable (210) comportant au moins un moule négatif de ladite au moins une nanostructure 3D (211), (figure 65),

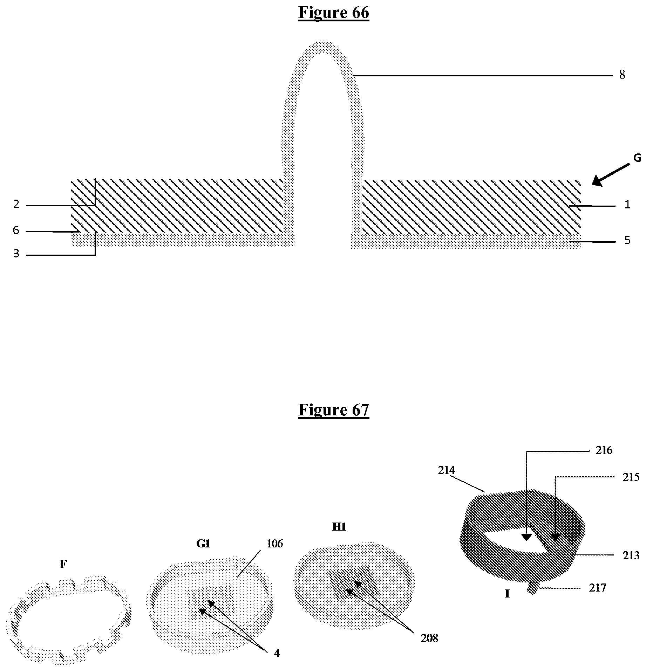

(viii) une étape de retrait de la pièce de soutien (I, i) pour obtenir ladite pièce perforée (G, G1, G2, g1, g2) comprenant sur sa face inférieure (3) une membrane poreuse nanostructurée en 3D (5), et du côté de sa face supérieure ladite au moins une protubérance (figure 66).

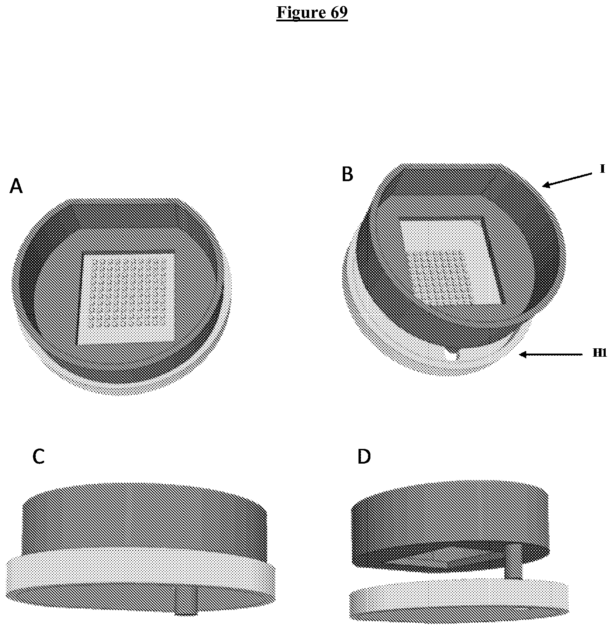

Comme montré à la figure 69, l'étape (i) du procédé décrit précédemment correspond à l'assemblage de la pièce de soutien I avec le moule H, en particulier un moule H1 comprenant 100 nanostructures 3D moulées (208), dans un alignement reproductible et spécifique qui est guidé par la section plate des pièces et la goupille d'alignement (217) de la pièce de soutien I qui s'insère dans le trou d'alignement (218) du moule H1.

Pour l'assemblage de la pièce de soutien I et du moule H, la face pleine (215) de ladite pièce de soutien I est placée sur la face supérieure (209) du moule H, de façon à ce que les nanostructures 3D moulées (208) dudit moule H viennent se positionner au niveau de la découpe (216) de ladite face pleine de la pièce de soutien I.

La chambre ainsi formée par la connexion du moule H avec la pièce de soutien I est ensuite remplie avec le polymère résorbable (22) selon l'étape (ii).

Après polymérisation du polymère résorbable (étape iii), une matrice en polymère résorbable (210) comportant au moins un moule négatif de ladite au moins une nanostructure 3D (211) est formée dans la découpe (216) sur la face pleine (215) de la pièce de soutien I.

Le moule H est ensuite retirée de la pièce de soutien I selon l'étape (iv). La base de la pièce de soutien I est alors formée au niveau de la découpe (216) par une matrice en polymère résorbable (210) comportant au moins un moule négatif d'au moins une nanostructure 3D (211).

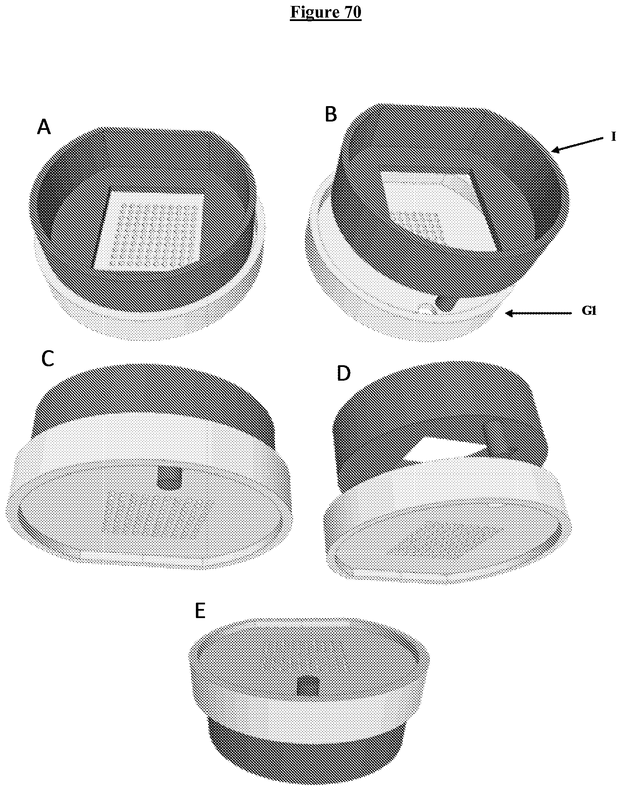

La pièce de soutien I est ensuite assemblée avec la pièce perforée G, en particulier une pièce perforée G1 comprenant 100 perforations (4), selon l'étape (v), dans un alignement reproductible et spécifique qui est guidé par la section plate des pièces et la goupille d'alignement (217) de la pièce de soutien I qui s'insère dans le trou d'alignement (218) de la pièce perforée G (figure 70).

La pièce perforée G est constituée d'un support (1) comprenant au moins une perforation (4), ledit support (1) étant inséré dans un socle (106).

Le choix de la pièce perforée (G1, G2, g1, g2) dépend du choix du moule (H1, H2, h1, h2) dans l'étape (i), la forme et le nombre de nanostructures 3D du moule (H1, H2, h1, h2) devant être identique à la forme et au nombre de perforations de la pièce perforée (G1, G2, g1, g2) (Figures 67, 68, 72, 73).

La face supérieure (2) du support (1) de la pièce perforée G se positionne au contact de la face inférieure (212) de ladite matrice en polymère résorbable (210).

Les perforations (4) de la pièce perforée G sont alors alignées avec les moules négatifs des nanostructures 3D (211) de ladite matrice (210) grâce à la goupille d'alignement (217) de la pièce de soutien I qui s'enclenche dans le trou d'alignement (218) de la pièce perforée G.

Les pièces I et G1 assemblées sont ensuite retournées comme montré à la figure 70-E, de façon à avoir la pièce G1 au-dessus de la pièce de soutien I.

Les parois des perforations de la pièce G1 permettent la formation d'une chambre, de sorte à faciliter la formation des protubérances (8) par l'application d'au moins une couche continue d'au moins un polyélectrolyte selon l'étape (vi), sur la surface continue formée par la face inférieure (3) du support (1) et la face inférieure (212) de ladite matrice en polymère résorbable (210) comprenant au moins un moule négatif de ladite au moins une nanostructure 3D (211), au niveau des perforations (4) dudit support (1).

Une fois la au moins une protubérance (8) formée, le polymère résorbable formant ladite matrice (210) de moules négatifs (211) est dissout selon l'étape (vii).

La pièce de soutien I est alors retirée de la pièce perforée G1.

La pièce perforée G1 comprend alors du côté de la face inférieure (3) du support (1) une membrane poreuse nanostructurée en 3D (5), et du côté de la face supérieure (2) du support (1), ladite au moins une protubérance dans le prolongement de ladite au moins une perforation du support de la pièce perforée G1, (figure 66).

Dans ce mode de réalisation, la face supérieure (6) de la membrane poreuse nanostructurée en 3D (5) est solidaire (au contact) de la face inférieure (3) du support constitué d'une membrane non résorbable (1).

Cette pièce perforée G1 alors obtenue comprenant un support (1) avec des perforations (4) et la membrane nanostructurée en 3D (5) comportant des protubérances (8), constitue le module central de la puce microfluidique et est utilisée pour la culture de cellules de chaque côté de ladite au moins une protubérance.

**Exemples de pièces I, H et G de forme circulaire**

Selon un mode de réalisation particulier, ladite pièce de soutien (I), ledit moule (H1, H2) et ladite pièce perforée (G1, G2) sont de forme circulaire, permettant en particulier de s'adapter à des boites de culture cellulaire d'un diamètre de 35 mm.

La figure 67 présente un mode de réalisation particulier du procédé dans lequel ledit moule (H1) et ladite pièce perforée (G1) comportent respectivement 100 nanostructures 3D moulées et 100 perforations.

Un tel procédé permet l'obtention d'une unité centrale avec 100 protubérances.

La figure 68 présente un mode de réalisation particulier du procédé dans lequel ledit moule (H2) et ladite pièce perforée (G2) comportent respectivement 9 nanostructures 3D moulées et 9 perforations.

Un tel procédé permet l'obtention d'une unité centrale avec 9 protubérances.

Ces exemples de nombres de perforations et de nanostructures 3D moulées, ne sont pas exhaustifs.

Le choix du moule et de la pièce perforée dépend du nombre de protubérances désiré pour la membrane nanostructurée en 3D de l'unité centrale.

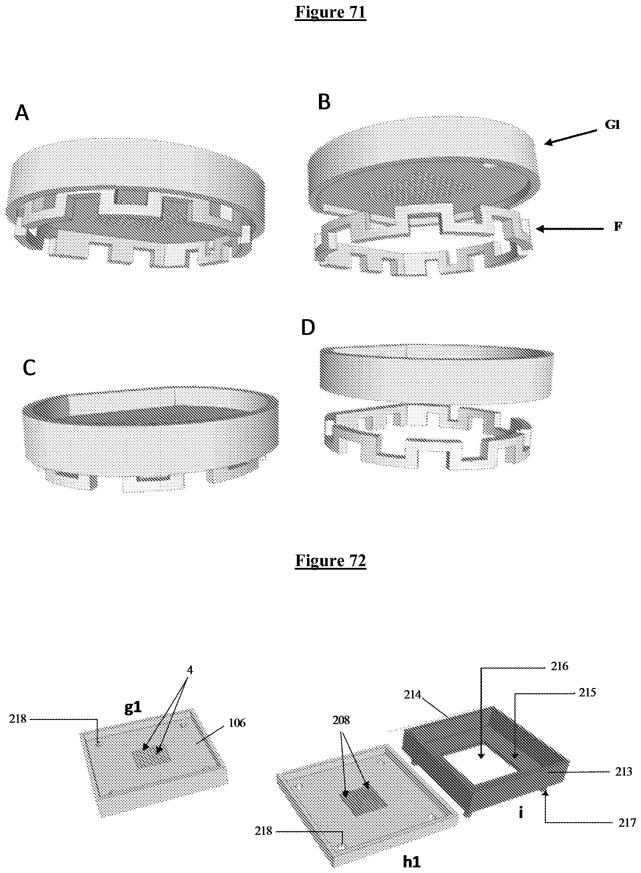

Dans ce mode particulier de réalisation, le module central constitué par la pièce perforée G avec les protubérances obtenue à l'issue du procédé détaillé ci-dessus, peut être placé sur une chambre de culture cellulaire, tel qu'une boite de culture cellulaire de diamètre 35 mm contenant du milieu de culture, par l'intermédiaire d'une pièce F, comme montré à la figure 71.

Selon un mode de réalisation, le module central obtenu par le procédé décrit ci-dessus, est placé sur un module inférieur tel que décrit, comprenant au moins un canal pour collecter les sécrétions de la au moins une protubérance.

Le module inférieur est de forme et de dimensions identiques audit module central.

Le module inférieur est assemblé au module central dans un alignement reproductible et spécifique qui est guidé par la section plate des pièces et la goupille d'alignement de la pièce du module inférieur qui s'insère dans le trou d'alignement du module central.

Le module inférieur comprend un nombre de canaux identique au nombres de perforations et donc de protubérances du module central, de manière à ce que l'assemblage dudit module central avec ledit module inférieur permette l'alignement des canaux avec les perforations et donc les protubérances, pour collecter les sécrétions des cellules via un sytème microfluidique.

Dans ce autre mode de réalisation particulier de la puce microfluidique, le module inférieur est remplacé par la pièce F. Cette pièce F, utilisé comme support du module central sur la boite de culture telle que représentée aux figures 67, 68 et 71 comporte des ouvertures carrées permettant la circulation du milieu de culture pour apporter les nutriments aux cellules en croissance sur la face interne de ladite au moins une protubérance.

## Exemples de pièces I, H et G de forme carrée

Selon un mode de réalisation particulier, ladite pièce de soutien (i), ledit moule (hl, h2) et ladite pièce perforée (g1, g2) sont de forme carrée.

La figure 72 présente un mode de réalisation particulier du procédé dans lequel ledit moule (hl) et ladite pièce perforée (gl) comportent respectivement 100 nanostructures 3D moulées et 100 perforations.

Un tel procédé permet l'obtention d'une unité centrale avec 100 protubérances.

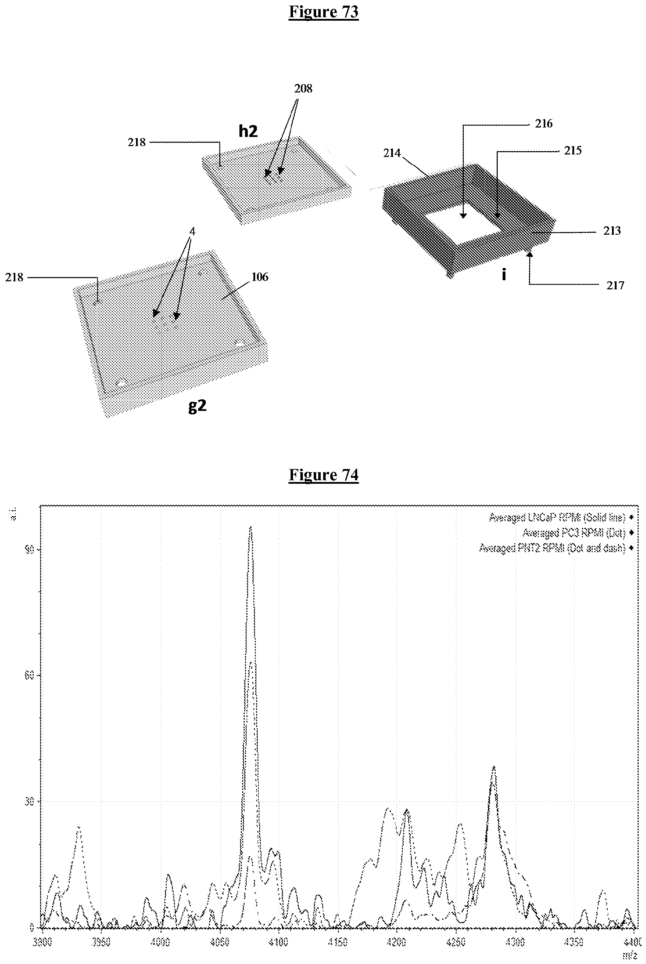

La figure 73 présente un mode de réalisation particulier du procédé dans lequel ledit moule (h2) et ladite pièce perforée (g2) comportent respectivement 9 nanostructures 3D moulées et 9 perforations.

Un tel procédé permet l'obtention d'une unité centrale avec 9 protubérances.

Ces exemples de nombres de perforations et de nanostructures 3D moulées, ne sont pas exhaustifs.

Le choix du moule et de la pièce perforée dépend du nombre de protubérances désiré pour la membrane nanostructurée en 3D de l'unité centrale.

La solution polymérique résorbable est préférablement réalisée avec du chitosan, de l'agarose ou de l'alginate.

Selon un mode de réalisation particulier, ladite solution en polymère résorbable (22) est du **chitosan.**

Lorsque le chitosan est utilisé, le moule résorbable peut être préparé en dissolvant 2% de chitosan dans 2% d'acide acétique durant une nuit, puis en diluant à 1.5% de chitosan avec de l'éthanol. La solution de chitosan est ensuite polymérisée dans un bain chaud à 5M de NaOH : éthanol à un ratio 1 :1.

Selon un mode de réalisation particulier, l'étape de polymérisation de ladite solution en polymère résorbable (22), ladite solution en polymère résorbable (22) étant du **chitosan,** est réalisée par une incubation avec une solution à 2% **d'acide acétique.**

Lorsque le chitosan est utilisé comme matériel polymérique résorbable, la dissolution est réalisée par une incubation sur la nuit avec une solution à 2% d'acide acétique, selon un protocole bien connu de l'Homme du métier.

Selon un mode de réalisation particulier, ladite solution en polymère résorbable (22) est **de l'agarose.**

Lorsque l'agarose est utilisé, le moule résorbable peut être préparé en chauffant et en dissolvant 40 $\mu$g/ml d'agarose dans du PBS (tampon phosphate salin). L'agarose est polymérisé en plaçant la solution obtenue à température au dessous de son point de gélification.

Selon un mode de réalisation particulier, l'étape de polymérisation de ladite solution en polymère résorbable (22), ladite solution en polymère résorbable (22) étant **de l'agarose,** est réalisée par une incubation à une température supérieure à la température de gélification de l'agarose.

Lorsque l'agarose est utilisé comme matériel polymérique résorbable, la dissolution est réalisée par un chauffage lent à partir de la température ambiante à une température de 70°C, durant 120 minutes, puis en laissant la température de l'agarose revenir à température ambiante sur une nuit.

Ce chauffage peut être réalisé dans un bain-marie. Il est important que la température augmente lentement pour minimiser les courants de convection thermiques qui pourraient abimer la membrane poreuse nanostructurée en 3D.

Des variantes de ce protocole de chauffage, bien connues de l'Homme du métier, incluent l'addition de DMSO dans l'eau du bain-marie pour modifier les propriétés de gélification de l'agarose.

Selon un mode de réalisation particulier, ladite solution en polymère résorbable (22) est **de l'alginate.**

Selon un mode de réalisation particulier, l'étape de polymérisation de ladite solution en polymère résorbable, ladite solution en polymère résorbable (22) étant **de l'alginate,** est réalisée par une incubation sur la nuit avec une solution dépourvue de $Ca^{2+}$ et additionnée d'un agent chélateur d'ions $Ca^{2+}$, tel que l'EDTA ou l'EGTA.

Le film multicouches de polyélectrolytes comporte comme paramètres variables :

- le nombre de couches,

- l'épaisseur de chacune des couches,

- la charge du ou des polyélectrolytes utilisés.

En variant le nombre de couches, la rugosité, l'épaisseur et la rigidité du film multicouches final peuvent être modifiées.

De préférence, le film est composé de 15 couches de 2 nm d'épaisseur, de polyélectrolytes.

En variant le nombre de couches ou le type de charge des polyélectrolytes utilisés, l'hydrophobicité du film multicouches final peut également être modifiée.

L'extrusion de la nanostructure 3D peut être sujette aux défauts suivants dus au système de pompage utilisé pour l'extrusion :

- défaut de translation, lorsqu'il y a translation de la protubérance par rapport à partir de l'emplacement prévu directement aligné avec la perforation du support,

- défaut d'extrusion, lorsqu'il y a des défauts dans la forme de la protubérance, comme par exemple un épaississement de la base ou d'autres défauts qui seront connus de l'homme du métier.

Une protubérance ainsi formée à partir de nanostructures 3D avec un défaut de translation ou un défaut d'extrusion peut continuer d'exercer sa fonction technique prévue initialement au sein du dispositif, cependant, comme la protubérance ainsi formée possède une forme moins optimale, la performance de celle-ci au sein du dispositif est également moins optimale. Toutefois, le dispositif peut continuer d'exercer sa fonction prévue, mais avec une performance réduite.

La protubérance peut présenter différents changements tels que :

- une inclinaison par rapport à un axe (y), passant par le centre de ladite ouverture et qui est perpendiculaire audit support,

- une variation de sa hauteur,

- une translation par rapport à la perforation, due à la translation de la membrane poreuse nanostructurée en 3D sur le support.

Ces changements sont dus au procédé de préparation du module central, et en particulier à la phase d'extrusion de la solution polymérique à travers les perforations dudit support.

Certains changements sont également entraînés directement lors de l'utilisation de la protubérance dans le dispositif.

**I- Exemple d'utilisation de la puce pour une co-culture**

**1. Conditions de maintien de cultures lignées de cellules épithéliales de prostate et de cellules stromales**

Le milieu de culture utilisé pour l'ensemble des expériences est un milieu Kératinocyte sans serum (KSFM, Keratinocyte Serum Free Médium) (Life Technologies, Carlsbad, CA, Ref. 17005-075) supplémenté de 5 ng/mL de facteur de croissance épidermal (EGF) et de 50 $\mu$g/mL d'extrait pituitaire bovin.
Les lignées de cellules épithéliales de prostate et de cellules stromales sont maintenues dans ce milieu et sont cultivées dans une atmosphère à 37°C et 5% $CO_2$.
Le repiquage des cellules dans un milieu frais est effectué tous les trois jours pour les cellules épithéliales et tous les deux jours pour les cellules stromales. Pour cela, les cellules sont lavées avec une solution de tampon phosphate salin de Dulbecco (D-PBS) sans calcium et sans magnésium (Life Technologies, Réf. 14190), puis incubées avec 1 mL de Trypsine-EDTA à 0,25 mg/mL, à 37°C, (Lonza, Basel, CH, Réf. CC-5012) pendant environ 7 minutes. Pour l'ensemble des expériences, le milieu de culture des cellules a été supplémenté chaque jour avec du milieu de culture frais.

**2. Préparation des cellules avant l'introduction dans l'unité centrale**

Une dissociation chimique des cellules est réalisée par une incubation de 5 minutes à 37°C avec 1 ml de la trypsine-EDTA à 0,25 mg/ml (Life Technologies, Réf. 25300-054) dans du milieu PBS sans calcium et sans magnésium.

Indépendamment, une puce microfluidique est stérilisée en faisant circuler une solution à 70% d'éthanol (volume/volume) à travers les canaux, puis en séchant l'ensemble du système microfluidique dans un four à une température entre 35°C et 45°C pendant au moins 30 minutes, puis en l'exposant à un rayonnement U.V. et à l'ozone pendant 40 min.

**3. Préparation de la membrane poreuse nanostructurée en 3D de l'unité centrale**

La membrane poreuse nanostructurée en 3D est constituée de couches successives de polyélectrolytes alternant une couche de polyélectrolyte chargé positivement et une couche de polyélectrolyte chargé négativement. Selon le procédé de fabrication, cette même membrane constitue les protubérances.

La face externe et la face interne des protubérances, constituées de la membrane poreuse en polyélectrolytes, sont recouvertes d'une préparation de matrice extracellulaire (ECM) composée de Matrigel® et/ou de collagène, de fibronectine ou d'acide hyaluronique.

La matrice Matrigel® utilisé ici est un produit commercial fabriqué par la société Corning®.

Il s'agit d'une préparation de membrane basale reconstituée qui est extraite de sarcome Engelbreth-Holm-Swarm (EHS) de souris, tumeur riche en protéines de matrice extracellulaire. Une fois isolée, cette matière est composée d'environ 60% de laminine, de 30% de collagène IV, et de 8% d'entactine. L'entactine est une molécule de pontage qui interagit avec la laminine et le collagène IV, et contribue à l'organisation structurelle de ces molécules de la matrice extracellulaire.

La matrice Matrigel® de Corning® contient également des protéoglycanes héparane sulfate (perlecan), du facteur de croissance transformant β (TGF-β), du facteur de croissance épidermique, du facteur de croissance de type insuline, du facteur de croissance de fibroblastes, un activateur tissulaire du plasminogène et d'autres facteurs de croissance qui sont naturellement présent dans la tumeur EHS. Il contient également des métalloprotéinases de matrice résiduelles dérivées de cellules tumorales.

Le Matrigel® peut être utilisé seul pour fonctionnaliser la membrane poreuse, à une concentration de 6 mg/ml, ou en mélange avec du collagène de type I à une concentration comprise entre 0,75 et 2,5 mg/ml.

**4. Introduction des deux types cellulaires et co-culture cellulaire dans l'unité centrale**

4.1.Introduction des cellules épithéliales

Dans un premier temps, les cellules épithéliales sont introduites pour former une couche jointive de cellules, c'est-à-dire une culture de cellules au stade de la confluence.

Selon un mode de réalisation particulier, les cellules épithéliales sont introduites sur les faces internes des protubérances de l'unité centrale.

Trois heures sont requises pour obtenir l'adhésion des cellules et 24h pour la formation d'une couche de cellules jointives, c'est-à-dire à un stade de confluence des cellules. Ces cellules adhérentes et prolifératives sécrètent leur propre matrice extracellulaire et établissent ainsi une couche basale jouant le rôle de barrière.

La face interne des protubérances de l'unité centrale est ainsi recouverte d'une monocouche dense de cellules épithéliales, qui sert de support physiologiquement pour la croissance et la différenciation des cellules humaines, isolées à partir de l'urine du patient.

L'introduction des cellules sur la face interne des protubérances peut se faire selon 3 modes :

- retourner le module central afin d'avoir les sections inférieures des perforations vers le haut et pipeter manuellement une suspension cellulaire.

- retourner le module central afin d'avoir les sections inférieures des perforations vers le haut et utiliser un robot manipulateur de fluides pour introduire une suspension cellulaire.

- assembler le module supérieur, le module central et le module inférieur et remplir l'unité centrale du côté de la face interne des protubérances à l'aide des canaux microfluidiques de l'unité inférieure. Dans le cas de ce pré-assemblage des trois modules, les cellules sont donc introduites via les canaux du module inférieur. Ce mode d'introduction des cellules après un pré-assemblage des trois modules est préféré aux deux autres modes, car il prévient toute contamination bactérienne du fait que le système préalablement stérilisé est maintenu clos.

Selon un mode de réalisation particulier, les cellules épithéliales sont introduites à une concentration de $3.10^6$ cellules/mL dans l'unité centrale soit directement via les perforations (1er et 2ème mode) avec une seringue, soit via les canaux de l'unité inférieure (3ème mode) en utilisant un système fluidique automatisé et contrôlé en pression et débit (Fluigent) ou un pousse-seringue. L'utilisation d'une pompe à seringue avec un débit réglable est préférée afin de fournir une introduction douce et contrôlée des cellules.
Un flux stable et continu est délivré par l'utilisation de pompes à pression (Fluigent, France). Des récipients pressurisés contenant du milieu de culture sont maintenus dans une chambre à température et $CO_2$ contrôlés. Le débit est ajusté à environ 5-10 mL/h (10 mbar) et l'adhésion et la prolifération des cellules est observée dans le temps. Tous les échantillons sont gardés dans un incubateur humidifié à 37°C et 5% $CO_2$.

Dans un mode de réalisation particulier, l'unité centrale comporte des protubérances d'une hauteur de 350 µm avec une base circulaire de 150 µm de diamètre. L'aire de la surface interne de la protubérance est alors de 329 700 µm², sur laquelle environ 50 cellules épithéliales sont dénombrées au stade de confluence (couche de cellules jointives), soit environ une cellule pour 66 µm².

4.2.Introduction des cellules sur la face externe des protubérances

Dans un second temps, une fois la couche de cellules épithéliales jointives (ou confluentes) formée sur la face interne des protubérances, les cellules stromales sont dispensées sur la membrane poreuse au niveau des faces externes et internes des protubérances.

L'introduction des cellules sur la face interne des protubérances peut se faire selon deux modes :

- retourner le module central afin d'avoir les sommets des protubérances vers le haut et pipeter manuellement une suspension cellulaire.
- assembler le module supérieur, le module central et le module inférieur et remplir l'unité centrale du côté de la face interne des protubérances à l'aide des canaux microfluidiques de l'unité inférieure. Dans le cas de ce pré-assemblage des trois modules, les cellules sont donc introduites via les canaux entrée/sortie de l'unité supérieure. Ce mode d'introduction des cellules après un pré-assemblage des trois modules, est préféré aux autres modes, car il prévient toute contamination bactérienne du fait que le système préalablement stérilisé est maintenu clos.

Selon un mode de réalisation particulier, les cellules stromales sont introduites via les canaux entrée/sortie du module supérieur à une concentration de $3.10^6$ cellules/mL dans l'unité centrale soit directement à l'aide d'une seringue (1er mode), soit via les canaux du module supérieur (2ème mode) en utilisant un système fluidique automatisé et contrôlé en pression et débit (Fluigent) ou un pousse-seringue.

Les cellules stromales adhèrent très rapidement (moins d'une heure).

Il n'est pas nécessaire que les cellules stromales forment une couche de cellules confluentes (ou jointives), leur simple adhésion sur la face externe dans cet exemple suffit.

De manière générale, le ratio entre les cellules épithéliales et les cellules stromales est de 1 :2.

Ainsi, selon un mode de réalisation particulier, pour une co-culture sur une surface de 0.7 cm², la membrane poreuse au niveau des faces externes et internes des protubérances est fonctionnalisée avec 90 µl d'une solution de Matrigel® diluée à 6 mg/ml, puis ensemencée pour obtenir au final 7000 cellules épithéliales/cm² et 14 000 cellules stromales (fibroblastes)/cm².

Le milieu de culture, introduit via les canaux entrée/sortie du module supérieur et via les canaux du module inférieur pour alimenter les cultures cellulaires, est identique de part et d'autre des protubérances et est constitué par du milieu de culture KSFM supplémenté de 5 ng/mL de facteur de croissance épidermal (EGF) et de 50 μg/mL d'extrait pituitaire bovin.

4.3. Exemples de cellules épithéliales et de cellules stromales

Ces cellules épithéliales peuvent être des lignées cellulaires commerciales non tumorigènes (prostate ou vessie ou rein) ou des cultures primaires commerciales.

Ces cellules stromales peuvent être :

- soit des fibroblastes (cultures primaires commerciales ou lignées),
- soit des cellules mésenchymateuses (cultures commerciales ou lignées),
- soit autres cellules stromales (endothéliales, ...).

Les deux types de cellules utilisés pour former ces monocouches cellulaires, sont dites « neutres » ou « saines », elles sont non tumorigènes et jouent uniquement un rôle de couche basale. Ces cellules « neutres » forment au stade de la confluence une couche très jointive de cellules sur la face interne et externe des protubérances, établissant des jonctions serrées qu'il est possible de caractériser par immunofluorescence et imagerie (cf. marquage de E-cadhérine partie 5).

4.4. Interchangeabilité des cultures sur les faces internes et externes des protubérances

Selon un mode de réalisation particulier, les cellules épithéliales sont introduites sur la face interne des protubérances et les cellules stromales sont introduites sur la face externe des protubérances.

Cependant, la co-culture peut être établie de façon interchangeable, c'est-à-dire que les cellules stromales peuvent également être introduites sur la face interne des protubérances, et les cellules épithéliales sur la face externe des protubérances. Dans les deux cas, la couche de polyélectrolyte se trouvant entre les deux types cellulaires, permet de constituer une barrière poreuse grâce à son maillage de polyélectrolytes chargés positivement et négativement.

**5. Visualisation des cellules dans l'unité centrale (Preuve de concept de la co-culture sur les protubérances)**

Afin de valider la méthode de co-culture sur les protubérances de l'unité centrale, un immunomarquage est réalisé.
Cet immunomarquage est donc effectué sur des cellules mortes (fixées par du PFA) et cette visualisation a pour seul but de contrôler que la co-culture est bien en place et que la méthodologie d'introduction des cellules est correcte.

Les cellules sont visualisées dans le module central par immunomarquage.

- La phalloïdine est utilisée pour identifier les filaments d'actine corticale, qui suivent les contours de la membrane plasmique et, par conséquent fournissent un moyen pour délimiter l'étendue de la cellule et de sa membrane.
- L'E-cadhérine est utilisée pour détecter les jonctions cellule-cellule. L'immunocoloration est réalisée en introduisant l'E-cadhérine avec une pompe à seringue via les canaux à température ambiante.
  Après la formation d'une couche confluente de cellules épithéliales, environ 24h après leur introduction, elles sont fixées pendant 20 minutes avec 4% de Perfluoroalkoxy (PFA) (volume à volume) dans une solution composée de 10% de sucrose dans un tampon cytosquelette (solution A).
  Les cellules sont ensuite lavées avec de la solution A et perméabilisées pendant 3 minutes avec une solution A additionnée de 0,1% de Triton TX-100. Un lavage avec une solution de TBS est réalisé pendant 10 minutes, suivi d'un second lavage avec une solution de PBS pendant 30 minutes. L'autofluorescence du PFA est inactivée par le $NH_4Cl$ contenu dans la solution de TBS. Les sites non spécifiques sont bloqués par une incubation avec une solution de PBS à 10% de sérum de chèvre et à 3% de BSA. Les cellules sont ensuite incubées avec un anticorps primaire pendant une heure. L'anticorps primaire utilisé est un anticorps anti-E-cadhérine (Abcam, Ref. ab1416) dilué au 1/50 dans une solution de PBS à 0.1% de Tween-20 et 1% de BSA. Les cultures sont ensuite lavées pendant 30 minutes avec une solution de PBS, puis incubées avec un anticorps secondaire anti-souris couplé au cytochrome Cy3 (Jackson, Ref. 115-162-062), dilué au 1/1000 et de la Phalloidin FITC (Sigma, Ref. P5282) diluée au 1:1000 dans une solution de PBS à 0.1% de Tween-20 et 1% de BSA, pendant 20 minutes.
- Après un lavage de 30 minutes avec une solution de PBS, les noyaux sont contre-colorés avec du colorant Hoechst (Life Technologies, Réf. H-1399), dilué au 1:7000, pendant 5 minutes. Les cellules sont ensuite lavées pendant 10 minutes et le milieu fluorescent Dako est introduit manuellement.
- Les points focaux d'adhésion ont été détectés par marquage en utilisant de la Vinculine. Pour le contre-marquage à la Vinculine, les cellules sont pré-perméabilisées pendant 40 secondes avec du Triton X-100 et fixées avec une solution de PBS à 4% de PFA (v/v), pendant 20 minutes, puis lavées une fois avec une solution de PBS.
  Pour éviter toute adsorption non spécifique d'anticorps, les cellules sont incubées avec une solution à 0.1% de BSA et à 10% de sérum de chèvre pendant une heure.

Les cellules sont ensuite incubées pendant une heure avec un anticorps primaire dirigé contre la Vinculine (Sigma, Ref. V9131) dilué à 1:700 dans une solution de PBS à 0.05% de Tween 20 et 5% de sérum de chèvre, puis lavées 4 fois consécutives pendant 45 minutes avec une solution de PBS.

Les cellules sont ensuite incubées avec un anticorps anti-souris couplé au cytochrome Cy5, dilué 1/500 dans une solution de PBS à 0,05% de Tween 20 et à 5% de sérum de chèvre (Jackson).

Le module central est ensuite lavé 4 fois pendant 15 minutes avec une solution de PBS. Les noyaux et l'actine sont colorés comme décrit ci-dessus.

La co-culture est observée par microscopie à fluorescence ou peut être observée par d'autres modes de microscopie tel que la microscopie à contraste de phase, l'imagerie sans lentilles, la microscopie confocale, la microscopie à feuille de lumière.

Les images sont capturées durant la culture cellulaire.

Pour fournir une vue d'ensemble de la largeur totale du dispositif, des images de cellules sont enregistrées à l'aide d'un capteur sans lentille. Des analyses SEM sont également réalisées.

Dans un mode de réalisation particulier, les images en fluorescence du module central contenant la co-culture de cellules, sont obtenues à l'aide d'un microscope Zeiss AxioImager Z1 avec un objectif 20x équipé du module droit Apotome pour les acquisitions en profondeur de champ z-stack, avec la prise d'image tous les 3 mm dans l'axe z, pour un tube de 150 mm de diamètre. Les images sont enregistrées à l'aide d'une caméra monochrome digital AxioCam MRm montée sur le microscope.

## 6. Visualisation des cellules dans l'unité centrale en temps réel

Les cultures cellulaires dans l'unité centrale peuvent être suivies en temps réel par une observation au microscope en contraste de phase qui permet de visualiser les cellules non marquées et vivantes, du fait de la transparence des matériaux constituant les modules.

## II- Exemple d'utilisation de la puce pour le diagnostic

## 1. Introduction des cellules provenant du patient

Selon un mode de réalisation particulier, les cellules épithéliales sont introduites sur la face interne des protubérances et les cellules stromales sont introduites sur la face externe des protubérances.

Une fois une monocouche de cellules obtenue sur chacune des faces, soit après 24h, la puce microfluidique, ainsi pourvue de cellules, peut être utilisée pour le diagnostic d'un patient.

Pour cela, des cellules sont isolées à partir d'un échantillon d'urine de patient d'au moins 50 mL, en particulier de 50 à 100 ml. L'isolement est réalisé par centrifugation de l'échantillon d'urine à une faible vitesse, en particulier 800 g pendant 5 min, permettant la sédimentation des cellules contenues dans l'échantillon d'urine. Cette étape de centrifugation est bien connue de l'Homme du métier.

Le culot de cellules sédimentées est ensuite resuspendu dans du milieu de culture et la suspension de cellules est directement introduite dans la puce microfluidique, ce qui signifie que les cellules n'exigent pas de pré-culture avant leur introduction dans la puce.

La concentration des cellules obtenue à partir de l'échantillon d'urine est ou varie de quelques centaines de cellules à plusieurs milliers.

Les cellules isolées de l'urine du patient peuvent être introduites du côté de la face de la protubérance qui supporte la culture des cellules épithéliales, ou du côté de la face de la protubérance qui supporte la culture des cellules stromales. Autrement dit, ces cultures étant interchangeables de part et d'autre de la protubérance, les cellules isolées de l'urine du patient peuvent être introduites aussi bien sur la face interne que sur la face externe des protubérances.

Selon un mode de réalisation particulier, les cellules isolées de l'urine du patient sont introduites du côté de la face de la protubérance qui supporte la culture des cellules épithéliales. Ainsi, elles sont introduites soit via les canaux de l'unité inférieure, lorsque la monocouche de cellules épithéliales est formée du côté de la face interne des protubérances, soit via les canaux entrée/sortie de du module supérieur lorsque la monocouche de cellules épithéliales est formée du côté de la face externe des protubérances.

Les cellules isolées à partir de l'urine du patient sont des cellules uroépithéliales (ou urothéliales) exfoliées, incluant à la fois les cellules épithéliales de vessie, de prostate et de rein.

Dans un mode de réalisation particulier, la face interne des protubérances est recouverte d'une couche pré-formée de cellules épithéliales préalablement cultivées, la face externe des protubérances est recouverte d'une couche pré-formée de fibroblastes (cellules stromales), et les cellules isolées sont dispensées via les canaux du module inférieur.

Ces cellules isolées s'insèrent dans cette couche pré-formée de cellules épithéliales saines du coté de la face interne des protubérances, et qui est supportée par une couche de fibroblastes sains.

## 2. Observation de la prolifération des cellules provenant du patient

La prolifération des cellules isolées est alors suivie, afin d'observer la progression de la prolifération des cellules isolées dans le dispositif et examiner si cette prolifération aboutit au remplacement des cellules basales saines et affecte le profil sécrétoire globale du tissu.

## 3. Récupération des sécrétions

Une fois l'introduction des cellules isolées à partir de l'urine du patient réalisée, les cellules épithéliales de patients sont stimulées par ajout de 0,1 ng/mL de DHT (Dihydrotestostérone) sur la face externe ou interne de la protubérance Cette stimulation des cellules par DHT dure entre 24h et 48h.

La membrane constituant les faces externes et internes des protubérances étant poreuse, cette stimulation peut être effectuée indifféremment d'un côté ou de l'autre des protubérances.

Les cellules épithéliales peuvent également être stimulées par l'ajout de mibolerone (hormone non métabolisée).

La stimulation des cellules épithéliales est ainsi, réalisée après l'adhésion des deux types cellulaires de part et d'autre des protubérances, et après leur croissance jusqu'au stade de confluence.

Les sécrétions peuvent être récupérées lorsque les cellules isolées du patient adhèrent et s'insèrent dans cette couche pré-formée de cellules épithéliales saines du coté de la face interne des protubérances, et qui est supportée par une couche de fibroblastes sains du coté de la face externe des protubérances. L'adhésion des cellules isolées du patient dure environ 3 h et leur intégration dure environ 6h.

L'accumulation d'un volume suffisant de sécrétions intervient progressivement.
La récupération finale des sécrétions pour l'analyse du sécrétome se fait après avoir laissé passer au moins 12h.

Plus particulièrement, les sécrétions sont récupérées au terme des 24 à 48h de stimulation au DHT.

Elles sont ensuite analysées par un dispositif permettant l'analyse de composés en solution. Selon un mode de réalisation particulier, le sécrétome est analysé par spectrométrie de masse.

Les sécrétions peuvent être analysées en ligne par des capteurs incorporés dans ladite puce.

Il est à noter que les différents modules composant ladite puce ne sont pas affectés lorsque les sécrétions sont récupérées ou lorsque les sécrétions sont analysées en continu par des capteurs en ligne.

La recherche de marqueurs spécifiques par des méthodes immunologiques peut également être réalisée dans les sécrétions récupérées.

Par exemple, la détection du PSA (antigène spécifique de la prostate), biomarqueur de référence du cancer de la prostate, peut être effectuée.

Pour une protubérance d'une hauteur de 350 $\mu$m et d'une base circulaire d'un diamètre de 150 $\mu$m, le volume de sécrétions récupéré au bout de 24h est d'environ 2 nL.

La détection et la quantification du PSA est réalisé par test Elisa.

Pour cela, environ 50 $\mu$l de milieu à l'intérieur de plusieurs protubérances sont collectés puis déposés dans une plaque 96 puits, placée à 37°C durant 45 min. Cinq lavages successifs à l'eau distillée sont nécessaires afin de bien éliminer les protéines non fixées à l'anticorps primaire anti-PSA.

100 $\mu$L d'anticorps secondaire anti-PSA libre couplés à la HRP (Horseradish peroxidase) sont ensuite ajoutés dans chaque puits avant 45 min d'incubation à 37°C de la plaque ELISA. Enfin, 100 $\mu$L de substrat (TMB) sont ajoutés donnant lieu à une réaction colorimétrique enzyme substrat.

Après 15 min à 37°C, la réaction est arrêtée par l'ajout de 100 $\mu$L d'acide sulfurique et l'absorbance est détectée à l'aide d'un lecteur de plaque ELISA à 450 nm.

## III- Exemple d'utilisation de la puce pour le criblage de molécules

Dans un mode de réalisation particulier, la puce microfluidique de culture cellulaire, est utilisée pour le criblage de molécules.

## IV- Exemple d'utilisation de la puce pour déterminer l'effet d'un traitement de cancers urologiques sur un patient

Dans un mode de réalisation particulier, la puce microfluidique de culture cellulaire, est utilisée pour déterminer l'effet d'un traitement pour un cancer urologique sur un patient atteint d'un cancer urologique.

Dans ce mode de réalisation, l'analyse du sécrétome des cellules isolées de l'urine du patient, insérées dans la culture de cellules épithéliales sur la protubérance, est effectuée avant et après le traitement du patient, et/ou pendant le traitement.

La comparaison du sécrétome obtenu avant le traitement avec celui obtenu après le traitement, et/ou celui obtenu pendant le traitement, permet de déterminer l'effet du traitement sur le cancer urologique dont est atteint le patient.

## V- Discrimination de cellules non cancéreuses de cellules cancéreuses par l'analyse du sécrétome en MALDI-TOF

Trois types cellulaires utilisés (lignées) :

PNT2 : épithélium prostatique sain hormonosensible (lignée saine)
LNCaP : Epithélium prostatique tumoral : hormono-sensible (tumeur primaire) (lignée cancéreuse)
PC3 : Métastase, tumeur secondaire, hormono-résistante (lignée cancéreuse)

Réactifs :

Milieu RPMI 1640 GlutaMAX™ (Thermofisher Scientific), comprenant de la L-Alanyl-Glutamine, L-Arginine, D-Glucose (Dextrose)
SVF (serum de veau fetal) (PAN Biotech, Cat No: P30-3302, Lot No: P150205)

Culture cellulaire

Les cellules de chaque lignée cellulaire sont cultivées en milieu RPMI 1640 GlutaMAX™ dans des plaques 48 puits (culture cellulaire en 2D) à raison de 50.000 cellules/puits dans un volume de 100 $\mu$l, pendant 48h avec ou sans SVF.

Les 100$\mu$l de surnageant de culture sont ensuite prélevés directement dans chacun des puits (les cellules étant des cellules adhérentes). Pour chacune des conditions de culture (trois lignées cellules avec et sans SVF), 0.8 $\mu$l de surnageant sont analysés au MALDI-TOF sur matrice CHCA (acide cyano-4-hydroxycinnamique) en triplicas (soit 3 x 0.8$\mu$l).

MALDI sur matrice CHCA:

L'acide cyano-4-hydroxycinnamique ou CHCA est un dérivé d'acide cinnamique et est un membre de la famille des phénylpropanoïdes. Il est utilisé comme matrice pour les peptides et les nucléotides dans les analyses par spectrométrie de masse MALDI. La solution matricielle se compose d'une molécule cristallisée (CHCA par exemple) et d'une source de contre-ion telle que l'acide trifluoroacétique (TFA) pour générer des ions [M + H].

Méthodologie MALDI:

La solution matricielle est mélangée avec l'échantillon dans un ratio 1: 1, puis déposée sur une plaque MALDI. Les solvants se vaporisent, laissant seulement la matrice recristallisée, mais avec des molécules d'analyte incorporées dans des cristaux MALDI.

Puis, lorsque le laser est activé sur le cristal matriciel à l'endroit où de la gouttelette a séché, la matrice absorbe l'énergie laser. La matrice est alors désorbée et ionisée. La matrice transfère les protons aux molécules d'analyte, chargeant ainsi l'analyte qui peut ensuite être analysé dans n'importe quel spectromètre de masse à accélération d'ions. Dans le présent exemple, la spectrométrie de masse à temps de vol (TOF) est utilisée.

Obtention des Spectres MALDI-TOF :

Les mesures sur chacun des échantillons biologiques par lignée sont effectuées en triplicas. Chaque spectre est normalisé par rapport à l'AUC (Aire totale sous le spectre) puis traitement des spectres avec deux procédés « baseline substraction » (effet matrice) et « lissage ».

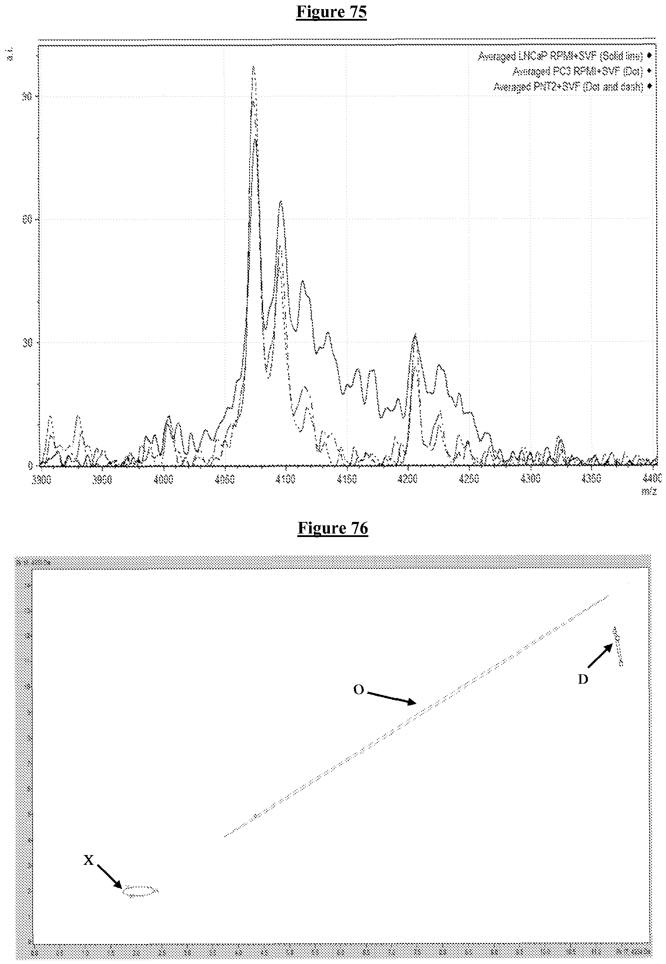

Le spectre moyen sur ces 3 mesures est représenté pour chacune des lignées cultivées avec SVF (Figure75) ou sans SVF (Figure 74).

Discrimination des pics

Méthodologie:

Type d'analyse: «2D Peak Distribution» (logiciel ClinPro Tools)

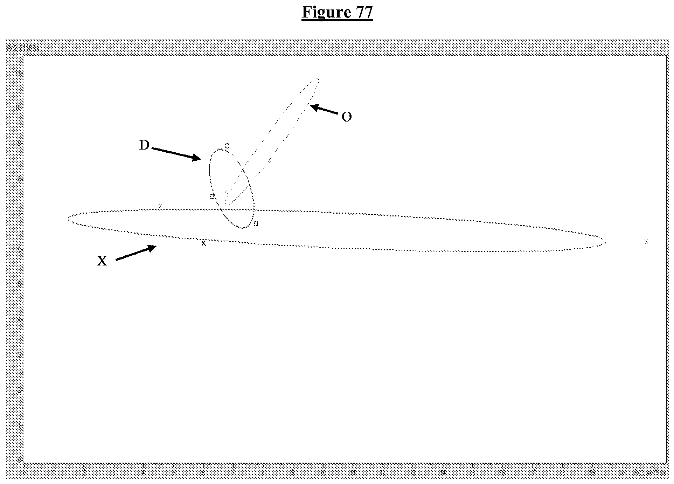

La vue en 2D de distribution de pic (« 2D Peak Distribution View ») affiche la distribution de deux pics (x, y) dans les spectres des trois modèles de classes, i. e. les trois lignées cellulaires cultivées sans SVF (Figure 76) et avec SVF (Figure 77). Les ellipses représentent l'écart-type de la moyenne de la classe de l'aire des pics (N = 3 mesures MALDI par lignée cellulaire). Les données sont affichées sur un plan bi-dimensionnel.

Par défaut, les deux premiers pics (= meilleurs séparateurs) sont représentés. L'axe des x représente les valeurs de l'aire du pic/intensité par rapport au pic le plus important en fonction de sa p-value, et l'axe y représente les valeurs de l'aire du pic/intensité pour le deuxième pic le plus important. Les mesures de l'axe sont données dans des unités arbitraires qui sont choisies automatiquement pour s'adapter au tracé optimal dans le plan.

L'étude des figures 76 et 77 montre que la privation des cellules en SVF facilite la lecture des spectres MALDI. Cela peut s'expliquer par le fait que la composition du SVF n'est pas bien connue et peut varier d'un lot à l'autre. En revanche, l'absence de SVF peut occasionner un stress des cellules qui sécrètent alors beaucoup plus de protéines. Cela explique pourquoi la discrimination des 3 lignées est plus difficile à faire sur le surnageant de cellules cultivées avec SVF.

La culture dans un milieu minimum par exemple avec BSA (albumine de sérum bovin) à la place du SVF, préservant la viabilité cellulaire et permettant d'obtenir des pics mieux contrôlés, peut être envisagée.

## FIGURES

1 support constitué d'une membrane non résorbable (unité centrale)
2 face supérieure du support constitué d'une membrane non résorbable (unité centrale)
3 face inférieure du support constitué d'une membrane non résorbable (unité centrale)
4 perforation du support constitué d'une membrane non résorbable (unité centrale)
5 membrane poreuse nanostructurée en 3D (unité centrale)
6 face supérieure de membrane poreuse nanostructurée en 3D (unité centrale)
7 face inférieure de membrane poreuse nanostructurée en 3D (unité centrale)
8 protubérance (unité centrale)
9 face externe de protubérance (unité centrale)

10 face interne de protubérance (unité centrale)

11 section de la perforation au niveau de la face supérieure du support (unité centrale)

12 section de la perforation au niveau de la face inférieure du support (unité centrale)

13 base circulaire de la protubérance (unité centrale)

14 canal (unité inférieure)

15 orifice supérieur du canal (unité inférieure)

16 orifice inférieur du canal (unité inférieure)

17 réservoir (unité inférieure)

18 canal du réservoir (unité inférieure)

19 orifices de l'unité supérieure débouchant sur les canaux entrée/sortie (unité supérieure)

20 orifice supérieur du canal (module inférieur)

21 orifice inférieur du canal (module inférieur)

22 polymère résorbable

23 nanostructure en 3D

24 cellule épithéliale

101 module supérieur

102 unité supérieure

103 socle du module supérieur

104 module central

105 unité centrale

106 socle du module central

107 module inférieur

108 unité inférieure

109 socle du module inférieur

201 éléments de fixation

202 canaux entrée/sortie (module supérieur)

203 chambre (unité supérieure)

204 éléments de fixation

205 ensemble d'orifices inférieurs des canaux (module inférieur)

206 ensemble d'orifices supérieurs des canaux (module inférieur)

207 ensemble de protubérances (module central)

208 nanostructure 3D moulée

209 face supérieure du moule

210 matrice polymère résorbable

211 moule négatif d'une nanostructure 3D

212 face inférieure de la matrice

F pièce de support du module central

213 cadre latéral de la pièce de soutien

214 face supérieure ouverte de la pièce de soutien

215 face inférieure pleine de la pièce de soutien

216 découpe de la face pleine de la pièce de soutien

217 goupille d'alignement

218 trou d'alignement

H, H1, H2, h1, h2 moule

I, i pièce de soutien

G, G1, G2, g1, g2 pièce perforée

**Figure 1 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, et dans laquelle la protubérance est en totalité en regard de la perforation.

**Figure 2 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est égale à la valeur du diamètre d3 de la base circulaire de ladite protubérance, et dans laquelle la protubérance est en totalité en regard de la perforation.

**Figure 3 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et à la valeur du diamètre d3 de la base circulaire de ladite protubérance, et dans laquelle la protubérance est en partie en regard de la perforation.

**Figure 4 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 5 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 6 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 7 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 8 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 9 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 10 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en partie en regard de la perforation.

**Figure 11** : Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 12 :** Vue schématique en coupe transversale d'une unité centrale comportant une protubérance dans la membrane nanostructurée en 3D, et une perforation dans le support, et dans laquelle la protubérance est inclinée selon un axe (z) par rapport à l'axe vertical (y).

**Figure 13 :** Vue schématique en coupe transversale d'une unité centrale comportant une protubérance dans la membrane nanostructurée en 3D, et une perforation dans le support, et dans laquelle la face interne de la protubérance est recouverte d'un ensemble d'un premier type cellulaire au stade de la confluence, et la face externe de la protubérance est recouverte d'un ensemble d'un deuxième type cellulaire au stade de la confluence.

**Figure 14 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en totalité en regard du canal.

**Figure 15 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est supérieure à la valeur du diamètre d5 de l'orifice inférieur, et dans laquelle la protubérance est en totalité en regard du canal.

**Figure 16 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

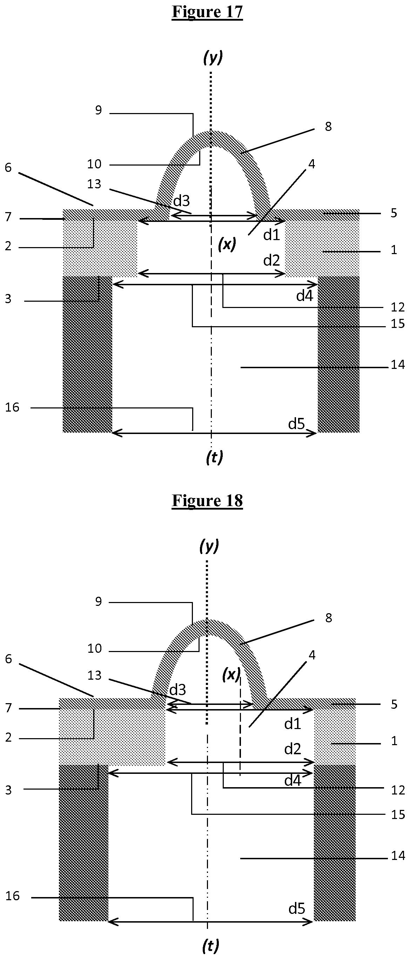

**Figure** 17 : Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

**Figure 18 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

**Figure 19 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 20 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

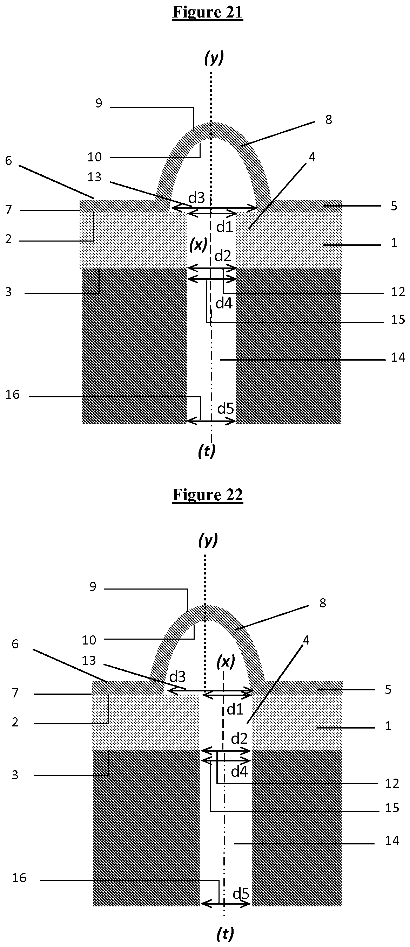

**Figure 21** : Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 22 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

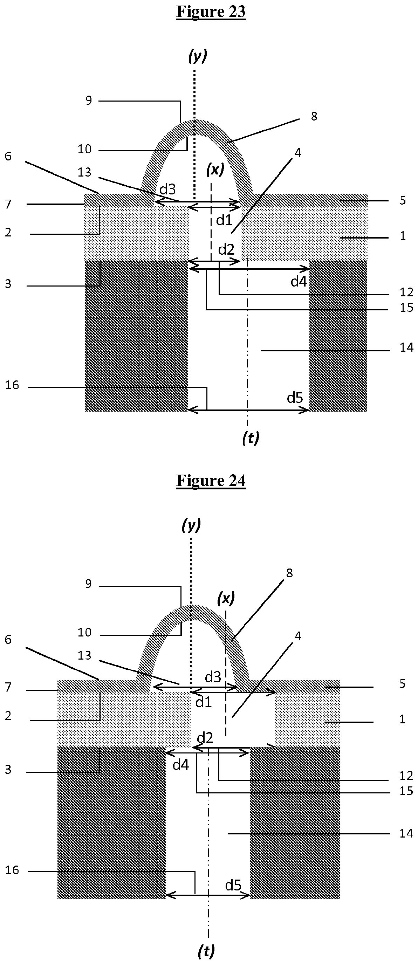

**Figure 23 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 24 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

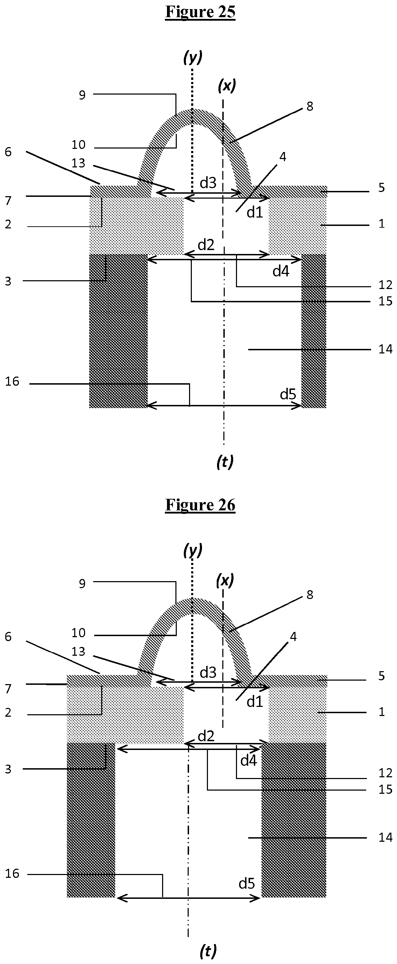

**Figure 25 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 26 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

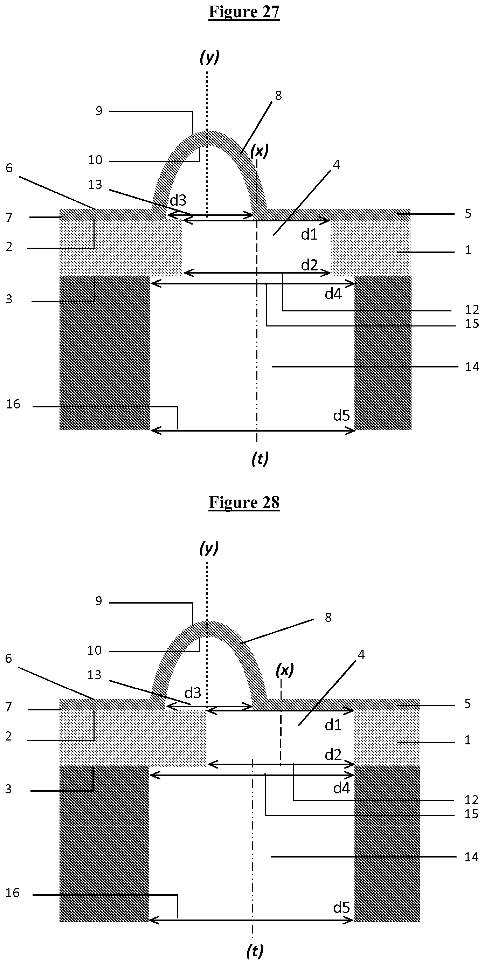

**Figure 27 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur d3 du diamètre de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 28 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

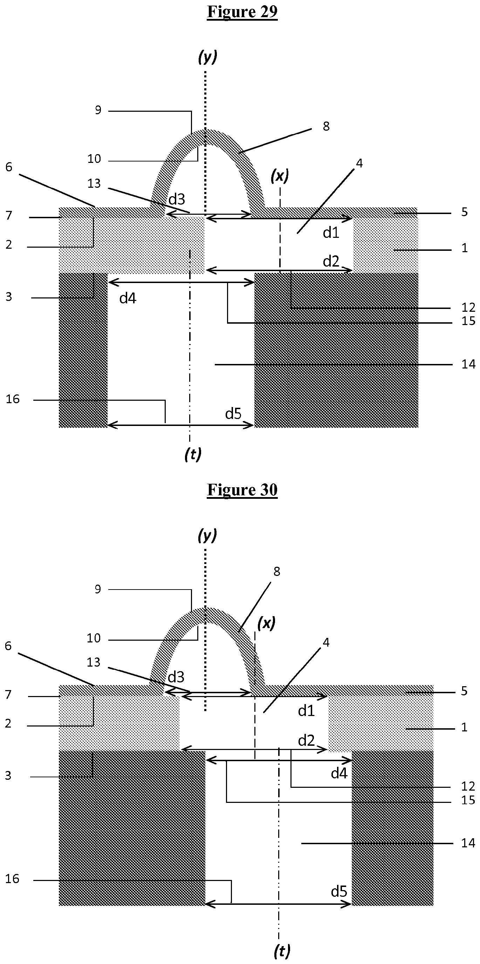

**Figure 29 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 30 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 31** : Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est égale à la valeur du diamètre d5 de l'orifice inférieur, et les deux orifices inférieurs des deux canaux débouchant respectivement sur un réservoir, débouchant à l'extérieur du module inférieur via un canal de sortie (protubérance en totalité en regard du canal).

**Figure 32 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est égale à la valeur du diamètre d5 de l'orifice inférieur, et les deux orifices inférieurs des deux canaux débouchant à l'extérieur du module inférieur à deux emplacements distincts (protubérance en totalité en regard du canal).

**Figure 33 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est égale à la valeur du diamètre d5 de l'orifice inférieur, et les deux canaux sont connectés entre eux de sorte que les deux orifices inférieurs des deux canaux débouchent à l'extérieur du module inférieur au même emplacement (protubérance en totalité en regard du canal).

**Figure 34 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est égale à la valeur du diamètre d5 de l'orifice inférieur, et les deux orifices inférieurs des deux canaux débouchant respectivement sur un réservoir, chacun des réservoirs débouchant à l'extérieur du module inférieur au même emplacement, via des canaux de sortie connectés entre eux (protubérance en totalité en regard du canal).

**Figure 35 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est supérieure à la valeur du diamètre d5 de l'orifice inférieur, et les deux canaux sont connectés entre eux de sorte que les deux orifices inférieurs des deux canaux débouchent au même emplacement sur un réservoir, lequel débouche à l'extérieur du module inférieur via un canal de sortie (protubérance en totalité en regard du canal).

**Figure 36 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est supérieure à la valeur du diamètre d5 de l'orifice inférieur, et les deux orifices inférieurs des deux canaux débouchent respectivement à deux emplacements distincts sur un même réservoir, lequel débouche à l'extérieur du module inférieur via un canal de sortie (protubérance en totalité en regard du canal).

**Figure 37 :** Vue schématique en coupe transversale d'une unité centrale comportant quatre protubérances dans la membrane nanostructurée en 3D, et quatre perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant quatre canaux, dont la valeur du diamètre d4 de l'orifice supérieur de chacun des canaux est égale à la valeur du diamètre d2 de la section inférieure de chacune des perforations, et les deux orifices inférieurs d'un premier ensemble de deux canaux débouchent respectivement à deux emplacements distincts sur un premier réservoir, et les deux orifices inférieurs d'un deuxième ensemble de deux canaux débouchent respectivement à deux emplacements distincts sur un deuxième réservoir, le premier et le second réservoir débouchant respectivement à l'extérieur du module inférieur à des emplacements distincts (protubérance en totalité en regard du canal).

**Figure 38 :** Vue schématique en perspective du module supérieur.

**Figure 39 :** Vue schématique en perspective du module central.

**Figure 40 :** Vue schématique en perspective prise au dessus de la face supérieure de la membrane poreuse nanostructurée en 3D, d'une unité centrale comportant un ensemble de protubérances.

**Figure 41 :** Vue schématique en perspective du module inférieur.

**Figure 42 :** Vue schématique en perspective de la puce microfluidique comportant l'assemblage du module supérieur, du module central et du module inférieur.

**Figure 43 :** Photo du module supérieur (vue du côté de l'ouverture de la chambre)

**Figure 44 :** Photo du module central. Vue du dessus de la face supérieure de la membrane poreuse nanostructurée en 3D comportant un ensemble de protubérances.

**Figure 45 :** Photo du module inférieur. Vue du dessus de la face supérieure de l'unité inférieure comprenant l'ensemble des orifices supérieurs des canaux.

**Figure 46** : Photo du module supérieur et du module inférieur.

**Figure 47 :** Photo du module supérieure, du module central et du module inférieure désassemblés.

**Figure 48** : Vue schématique en coupe transversale du support constitué d'une membrane non résorbable, de l'unité centrale, comportant une perforation.

**Figure 49 :** Vue schématique en coupe transversale du support constitué d'une membrane non résorbable, de l'unité centrale, comportant une perforation, au travers duquel un polymère résorbable a été extrudé pour former une nanostructure en 3D du côté de la face supérieure dudit support.

**Figure 50 :** Vue schématique en coupe transversale du support constitué d'une membrane non résorbable, de l'unité centrale, comportant une perforation, au travers duquel un polymère résorbable a été extrudé pour former une nanostructure en 3D du côté de la face supérieure dudit support sur laquelle une couche de polyélectrolyte a été appliquée pour obtenir la membrane poreuse nanostructurée en 3D comportant une protubérance moulée sur ladite nanostructure en 3D.

**Figure 51 :** Vue schématique en coupe transversale du support constitué d'une membrane non résorbable et comportant une perforation, de l'unité centrale, sur lequel est positionnée de façon solidaire la membrane nanostructurée en 3D comportant une protubérance creuse.

**Figure 52 :** Photo au microscope confocal de la face interne d'une protubérance soutenant une culture de cellules épithéliales au stade de la confluence.

**Figure 53 :** Vue schématique en coupe transversale d'un moule comprenant au moins une nanostructure 3D moulée du côté de sa face supérieure

**Figure 54 :** Vue schématique en coupe transversale d'un moule recouvert de polymère résorbable du côté la face supérieure dudit moule

**Figure 55 :** Vue schématique en coupe transversale d'une matrice en polymère résorbable comprenant au moins un moule négatif d'au moins une nanostructure 3D moulée

**Figure 56 :** Vue schématique en coupe transversale d'une matrice en polymère résorbable comprenant au moins un moule négatif d'au moins une nanostructure 3D moulée, la face inférieure de ladite matrice étant recouverte d'une couche polyélectrolyte.

**Figure 57 :** Vue schématique en coupe transversale d'une matrice en polymère résorbable comprenant au moins un moule négatif d'au moins une nanostructure 3D moulée assemblée avec une pièce perforée comprenant un support constitué d'une membrane non résorbable perforé par au moins une perforation, ladite matrice étant assemblée du côté de sa face inférieure avec la face supérieure dudit support, de façon à ce que le moule négatif de la nanostructure 3D soit dans l'alignement de ladite perforation du support.

**Figure 58 :** Vue schématique en coupe transversale d'une matrice en polymère résorbable comprenant au moins un moule négatif d'au moins une nanostructure 3D moulée assemblée avec une pièce perforée comprenant un support constitué d'une membrane non résorbable perforé par au moins une perforation, ladite matrice étant assemblée du côté de sa face inférieure avec la face supérieure dudit support, de façon à ce que le moule négatif de la nanostructure 3D soit dans l'alignement de ladite perforation du support, dans laquelle la surface continue constituée par la face inférieure dudit support et la face inférieure de ladite matrice en polymère résorbable comportant au moins un moule négatif au niveau de ladite au moins une perforation dudit support, est recouvert d'une couche de polyélectrolyte pour former une membrane nanostructurée en 3D comportant au moins une protubérance.

**Figure 59** : Vue schématique en coupe transversale du module central correspondant à la pièce perforée comprenant du côté de la face inférieure du support, une membrane poreuse nanostructurée en 3D et du côté de la face supérieure du support, au moins une protubérance dans le prolongement de la au moins une perforation du support de la pièce perforée.

**Figure 60 :** Vue schématique en coupe transversale d'un moule comprenant au moins une nanostructure 3D moulée du côté de sa face supérieure, assemblé du côté de sa face supérieure avec une pièce de soutien comportant une découpe dans sa face inférieure.

**Figure 61** : Vue schématique en coupe transversale d'un moule comprenant au moins une nanostructure 3D moulée du côté de sa face supérieure, assemblé du côté de sa face supérieure avec une pièce de soutien comportant une découpe dans sa face inférieure, où ledit moule est recouvert de polymère résorbable du côté sa face supérieure.

**Figure 62 :** Vue schématique en coupe transversale d'une matrice en polymère résorbable comprenant au moins un moule négatif d'au moins une nanostructure 3D moulée, ladite matrice étant formée au niveau de la découpe de la pièce de soutien

**Figure 63 :** Vue schématique en coupe transversale d'une pièce de soutien contenant au niveau de la découpe de sa face inférieure pleine, un matrice en polymère résorbable comprenant au moins un moule négatif d'au moins une nanostructure 3D moulée, ladite pièce de soutien étant assemblée à une pièce perforée comprenant un support avec au moins une perforation, de façon à ce que ledit moule négatif d'au moins une nanostructure 3D moulée soit dans l'alignement de ladite perforation.

**Figure 64 :** Vue schématique en coupe transversale d'une pièce de soutien contenant au niveau de la découpe de sa face inférieure pleine, un matrice en polymère résorbable comprenant au moins un moule négatif d'au moins une nanostructure 3D moulée, ladite pièce de soutien étant assemblée à une pièce perforée comprenant un support avec au moins une perforation, de façon à ce que ledit moule négatif d'au moins une nanostructure 3D moulée soit dans l'alignement de ladite perforation, la surface continue constituée par la face inférieure dudit support et la face inférieure de ladite matrice en polymère résorbable comportant au moins un moule négatif au niveau de ladite au moins une perforation dudit support, étant recouvert d'une couche de polyélectrolyte pour former une membrane nanostructurée en 3D comportant au moins une protubérance.

**Figure 65 :** Vue schématique en coupe transversale du support de la pièce perforée comprenant du côté de sa face inférieure, une membrane poreuse nanostructurée en 3D et du côté de sa face supérieure, au moins une protubérance en polyélectrolyte dans le prolongement de la au moins une perforation du support de la pièce perforée, et la pièce de soutien I.

**Figure 66 :** Vue schématique en coupe transversale du module central correspondant à la pièce perforée comprenant du côté de la face inférieure du support, une membrane poreuse nanostructurée en 3D et du côté de la face supérieure du support, au moins une protubérance dans le prolongement de la au moins une perforation du support de la pièce perforée.

**Figure 67 :** Vue schématique en perspective d'une pièce de soutien I de forme circulaire, d'un moule H de forme circulaire et comprenant 100 nanostructures 3D moulées (H1), d'une pièce perforée G de forme circulaire et comprenant 100 perforations (G1) et d'une pièce F, support du module central.

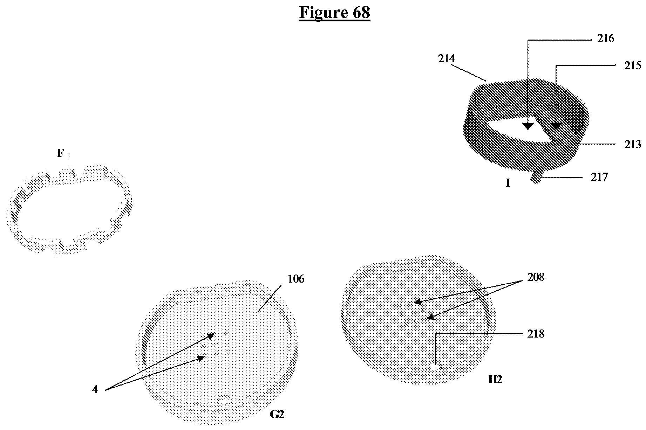

**Figure 68 :** Vue schématique en perspective d'une pièce de soutien I de forme circulaire, d'un moule H de forme circulaire et comprenant 9 nanostructures 3D moulées (H2), d'une pièce perforée G de forme circulaire et comprenant 9 perforations (G2) et d'une pièce F, support du module central.

**Figure 69 :** Vues schématiques en perspective de l'assemblage d'une pièce de soutien I de forme circulaire sur un moule H de forme circulaire et comprenant 100 nanostructures 3D moulées (H1), via la goupille d'alignement de I et le trou d'alignement de H1. **A :** Vue du dessus lorsque les deux éléments sont assemblés. **B :** Vue du dessus lorsque les éléments sont désassemblés. **C :** Vue de profil lorsque les deux éléments sont assemblés. **D :** Vue de profil lorsque les deux éléments sont désassemblés.

**Figure 70 :** Vues schématiques en perspective de l'assemblage d'une pièce de soutien I de forme circulaire sur d'une pièce perforée G de forme circulaire et comprenant 100 perforations (G1) via la goupille d'alignement de I et le trou d'alignement de G1. **A :** Vue du dessus lorsque les deux éléments sont assemblés. **B :** Vue du dessus lorsque les éléments sont désassemblés. **C :** Vue du dessous lorsque les deux éléments sont assemblés. **D :** Vue du dessous lorsque les deux éléments sont désassemblés. **E :** Vue de profil lorsque les deux éléments assemblés sont retournés de manière à ce que G1 soit orienté vers le haut et I soit orienté vers le bas.

**Figure 71 :** Vues schématiques en perspective de l'assemblage d'une pièce perforée G de forme circulaire et comprenant 100 perforations (G1) sur une pièce F, support du module central. **A :** Vue du dessous des deux éléments assemblés. **B :** Vue du dessous lorsque que les deux éléments sont désassemblés. **C :** Vue de profil lorsque les deux éléments assemblés. **D :** Vue de profil lorsque les deux éléments sont assemblés.

**Figure 72 :** Vue schématique en perspective d'une pièce de soutien i de forme carrée, d'un moule H de forme carrée et comprenant 100 nanostructures 3D moulées (h1), d'une pièce perforée G de forme carrée comprenant 100 perforations (g1).

**Figure 73 :** Vue schématique en perspective d'une pièce de soutien i de forme carrée, d'un moule H de forme carrée et comprenant 9 nanostructures 3D moulées (h2), d'une pièce perforée G de forme carrée et comprenant 9 perforations (g2).

**Figure 74 :** Analyse Maldi de surnageants de cultures cellulaires 2D (sans serum SVF) contenant les sécrétions cellulaires de LNCaP (solid line), PC3 (Dot) et PNT2 (Dot and dash). Chaque spectre est normalisé par rapport à l'AUC (Aire totale sous le spectre) puis traité par « baseline substraction » et « lissage ». Un spectre moyen sur 3 mesures et pour chaque lignée est représenté. Les spectres sont obtenus sur matrice Maldi CHCA (acide Cyano-4-hydroxycinnamic).

**Figure 75 :** Analyse Maldi de surnageants de cultures cellulaires 2D (avec serum SVF) contenant les sécrétions cellulaires de LNCaP (solid line), PC3 (Dot) et PNT2 (Dot and dash). Chaque spectre est normalisé par rapport à l'AUC (Aire totale sous le spectre) puis traité par « baseline substraction » et « lissage ». Un spectre moyen sur 3 mesures et pour chaque lignée est représenté. Les spectres sont obtenus sur matrice Maldi CHCA (acide Cyano-4-hydroxycinnamic).

**Figure 76 :** Ellipses représentant la déviation standard de l'aire sous les pics 2D des spectres de MALDI obtenus pour les sécrétions de lignées cellulaires cultivées **sans SVF. O :** PC3 (lignée cancéreuse) **X :** Lignée LNCaP (lignée cancéreuse). **D :** Lignée PNT2 (lignée saine).

**Figure 77 :** Ellipses représentant la déviation standard de l'aire sous les pics 2D des spectres de MALDI obtenus pour les sécrétions de lignées cellulaires cultivées **avec SVF. O :** PC3 (lignée cancéreuse) **X :** Lignée LNCaP (lignée cancéreuse). **D :** Lignée PNT2 (lignée saine).

**Revendications**

1. Méthode de diagnostic *in vitro* d'un cancer urologique comprenant les étapes suivantes :

   - récupération des sécrétions produites par les cellules isolées à partir d'un échantillon d'urine d'un patient à diagnostiquer,
   - analyse des composants desdites sécrétions, dit sécrétome, produites par lesdites cellules isolées à partir d'un échantillon d'urine dudit patient à diagnostiquer ;
   - comparaison d'un sécrétome de cellules isolées à partir d'un échantillon d'urine d'un patient à diagnostiquer par rapport, soit à un sécrétome de référence obtenu à partir de sécrétions de cellules saines isolées d'un échantillon d'urine d'une personne saine, soit à un sécrétome de référence obtenu à partir de sécrétions de cellules saines dérivées de cultures de lignées cellulaires standard caractéristiques d'un organe urologique déterminé,
   - détermination de la présence d'un cancer urologique lorsqu'au moins une différence d'un composant est déterminée entre ledit sécrétome de cellules isolées à partir d'un échantillon d'urine dudit patient à diagnostiquer et ledit sécrétome de référence,

   ledit sécrétome et ledit sécrétome de référence étant constitués de l'ensemble des composants formant leurs sécrétions respectives.

2. Méthode de diagnostic *in vitro* d'un cancer urologique selon la revendication 1, dans laquelle la détermination d'au moins une différence d'un composant entre le sécrétome de cellules isolées à partir d'un échantillon d'urine d'un patient à diagnostiquer et le sécrétome de référence, correspondant notamment à

   - la présence d'un composant en plus forte ou en plus faible concentration,
   - l'absence d'un composant,
   dans le sécrétome de cellules isolées à partir d'un échantillon d'urine dudit patient à diagnostiquer, par rapport audit composant dans le sécrétome de référence ;
   - la présence d'un composant dans le sécrétome de cellules isolées à partir d'un échantillon d'urine dudit patient à diagnostiquer, lequel est absent dans le sécrétome de référence.

3. Méthode de diagnostic *in vitro* d'un cancer urologique selon l'une quelconque des revendications 1 à 2, dans laquelle le sécrétome de référence analysé est obtenu à partir de cellules saines isolées d'un échantillon d'urine d'une personne saine, ou obtenu à partir de lignées cellulaires standard dérivées de reins, de vessie, de prostate, la méthode de diagnostic comprenant en outre une étape de détermination de la localisation dudit cancer urologique dans la vessie, les reins ou la prostate, selon que la au moins une différence d'un composant est observée par rapport à un sécrétome de référence obtenu pour la vessie, les reins ou la prostate.

4. Méthode de diagnostic *in vitro* d'un cancer urologique selon l'une quelconque des revendications 1 à 3, dans laquelle les cellules isolées sont cultivées sur un milieu de culture en 2D comportant un tapis cellulaire de cellules épithéliales confluentes surmonté d'un tapis cellulaire de fibroblastes confluents.

5. Méthode de diagnostic *in vitro* d'un cancer urologique selon l'une quelconque des revendications 1 à 4, dans laquelle ledit milieu de culture en 2D présente au moins un repli de manière à former un milieu de culture en 3D, mimant la structure d'au moins une glande exocrine composée de structure acinaire/canaliculaire et comportant ledit tapis cellulaire de cellules épithéliales surmonté dudit tapis cellulaire de fibroblastes.

6. Méthode de diagnostic *in vitro* d'un cancer urologique selon l'une quelconque des revendications 1 à 5, dans laquelle ladite analyse du sécrétome est réalisée par spectrométrie de masse sans séparation préalable des composants dudit sécrétome, pour obtenir un spectre de masse spécifique du sécrétome des cellules isolées à partir de l'échantillon, et notamment comprenant la comparaison du spectre de masse d'un sécrétome de cellules isolées à partir d'un échantillon d'urine du patient à diagnostiquer par rapport à un spectre de masse d'un sécrétome de référence obtenu à partir de cellules saines isolées d'un échantillon d'urine d'une personne saine.

7. Méthode de diagnostic *in vitro* d'un cancer urologique selon l'une quelconque des revendications 1 à 6, dans laquelle la_localisation dudit cancer urologique est effectuée dans les reins, la vessie ou la prostate par la comparaison du spectre de masse du sécrétome de cellules isolées à partir d'un échantillon d'urine du patient à diagnostiquer par rapport aux spectres de masse des sécrétomes de référence obtenu séparément à partir de cellules saines de reins, de vessie et de prostate, isolées d'un échantillon d'urine d'une personne saine, et la détermination d'au moins une différence entre le spectre de masse du sécrétome de cellules isolées à partir d'un échantillon d'urine du patient à diagnostiquer et au moins un desdits sécrétomes de référence de reins, de vessie ou de prostate.

8. Méthode de diagnostic *in vitro* d'un cancer urologique selon l'une quelconque des revendications 1 à 7, dans laquelle les composants présents dans les sécrétions constituant le sécrétome sont des protéines, des peptides, des acides aminés, et de biomarqueurs nucléiques (ADN, ARN, miARN et ARNi) et notamment les composants constituant le sécrétome comprennent les protéines suivantes : PSA, PCA3, KLK15, SPINK1, PRSS3, cathepsine D, Apolipoprotéine A-I, PLK2,
et comprennent les biomarqueurs nucléiques suivants : miR-141, miR-375, transcrits de fusion TMPRSS2-ERG, les gènes codant pour SFPR1 et BNC1, les gènes codant pour l'hydrolase Gammaglutamyl (*GGH*), l'inhibiteur de liaison au diazépam (*DBI*), et le facteur de transcription E2F3.

9. Méthode de diagnostic *in vitro* d'un cancer urologique selon l'une quelconque des revendications 1 à 8, dans laquelle les composants constituant le sécrétome de cellules saines de prostate isolées à partir d'un échantillon d'urine d'une personne saine, comprennent les protéines suivantes : PSA, PCA3, KLK15, SPINK1 et PRSS3,
et comprennent l'ARN de fusion TMPRSS2-ERG et les miARN miR-141 et miR-375.

10. Méthode de diagnostic *in vitro* d'un cancer urologique selon l'une quelconque des revendications 1 à 8, dans laquelle les composants constituant le sécrétome de cellules saines de reins isolées à partir d'un échantillon d'urine d'une personne saine, comprennent la protéine cathepsine D, et comprennent les biomarqueurs nucléiques suivants : les gènes codant pour SFPR1 et BNC1.

11. Méthode de diagnostic *in vitro* d'un cancer urologique selon l'une quelconque des revendications 1 à 8, dans laquelle les composants constituant le sécrétome de cellules saines de vessie isolées à partir d'un échantillon d'urine d'une personne saine, comprennent les protéines suivantes : Apolipoprotéine A-I et PLK2,
et les biomarqueurs nucléiques suivants : les gènes codant pour l'hydrolase Gamma-glutamyl (*GGH*), l'inhibiteur de liaison au diazépam (*DBI*), et le facteur de transcription E2F3.

**Patentansprüche**

1. In-vitro-Verfahren zur Diagnose von Urogenitalkrebs, umfassend die folgenden Schritte:

    - Rückgewinnen der von den aus einer Urinprobe eines zu diagnostizierenden Patienten isolierten Zellen produzierten Sekretionen;
    - Analysieren der Bestandteile der als Sekretom bezeichneten Sekretionen, die von den aus einer Urinprobe des zu diagnostizierenden Patienten isolierten Zellen produziert wurden;
    - Vergleichen eines Sekretoms isolierter Zellen aus einer Urinprobe eines zu diagnostizierenden Patienten in Bezug auf entweder ein Referenzsekretom, gewonnen aus Sekretionen gesunder Zellen, die aus einer Urinprobe einer gesunden Person isoliert wurden, oder ein Referenzsekretom, gewonnen aus Sekretionen gesunder Zellen, die aus Kulturen von Standardzelllinien abgeleitet wurden, die für ein bestimmtes urologisches Organ charakteristisch sind,
    - Bestimmen des Vorhandenseins eines Urogenitalkrebses, wenn mindestens ein Unterschied eines Bestandteils zwischen dem Sekretom von Zellen, die aus einer Urinprobe des zu diagnostizierenden Patienten isoliert wurden, und dem Referenzsekretom bestimmt wird,

wobei das Sekretom und das Referenzsekretom aus allen Bestandteilen bestehen, die deren jeweilige Sekretionen bilden.

2. In-vitro-Verfahren zur Diagnose von Urogenitalkrebs nach Anspruch 1, wobei das Bestimmen mindestens eines Unterschieds eines Bestandteils zwischen dem Sekretom von Zellen, die aus einer Urinprobe eines zu diagnostizierenden Patienten isoliert wurden, und dem Referenzsekretom insbesondere entspricht

    - dem Vorhandensein eines Bestandteils in höherer oder niedrigerer Konzentration,
    - der Abwesenheit eines Bestandteils,
    in dem Sekretom von Zellen, die aus einer Urinprobe des zu diagnostizierenden Patienten isoliert wurden, in Bezug auf den Bestandteil im Referenzsekretom;
    - dem Vorhandensein eines Bestandteils in dem Sekretom von Zellen, die aus einer Urinprobe des zu diagnostizierenden Patienten isoliert wurden, der im Referenzsekretom nicht vorhanden ist.

3. In-vitro-Verfahren zur Diagnose von Urogenitalkrebs nach einem der Ansprüche 1 bis 2, wobei das analysierte Referenzsekretom aus gesunden Zellen, die aus einer Urinprobe einer gesunden Person isoliert wurden, oder aus von Nieren, Harnblase, Prostata gewonnenen abgeleiteten Standardzelllinien gewonnen wird, wobei das Diagnoseverfahren ferner einen Schritt des Bestimmens der Lage des Urogenitalkrebses in der Harnblase, den Nieren oder der Prostata umfasst, je nachdem, ob der mindestens eine Unterschied eines Bestandteils in Bezug auf ein für die Harnblase, die Nieren oder die Prostata erhaltenes Referenzsekretoms beobachtet wird.

4.  In-vitro-Verfahren zur Diagnose von Urogenitalkrebs nach einem der Ansprüche 1 bis 3, wobei die isolierten Zellen auf einem 2D-Kulturmedium kultiviert werden, umfassend eine Zellmatte konfluenter Epithelialzellen unter einer Zellmatte konfluenter Fibroblasten.

5.  In-vitro-Verfahren zur Diagnose von Urogenitalkrebs nach einem der Ansprüche 1 bis 4, wobei das 2D-Kulturmedium mindestens einen Rücksprung aufweist, so dass ein 3D-Kulturmedium gebildet wird, das die Struktur mindestens einer exokrinen Drüse nachahmt, die aus acinärer/canaliculärer Struktur zusammengesetzt ist und die Epithelialzellmatte unter der Fibroblastenzellmatte umfasst.

6.  In-vitro-Verfahren zur Diagnose von Urogenitalkrebs nach einem der Ansprüche 1 bis 5, wobei die Analyse des Sekretoms durch Massenspektrometrie ohne vorherige Trennung der Bestandteile des Sekretoms durchgeführt wird, um ein spezifisches Massenspektrum des Sekretoms von den aus der Probe isolierten Zellen zu erhalten, und insbesondere das Vergleichen des Massenspektrums eines Sekretoms von aus einer Urinprobe des zu diagnostizierenden Patienten isolierten Zellen in Bezug auf ein Massenspektrum eines Referenzsekretoms umfasst, das aus gesunden, aus einer Urinprobe einer gesunden Person isolierten Zellen erhalten wurde.

7.  In-vitro-Verfahren zur Diagnose von Urogenitalkrebs nach einem der Ansprüche 1 bis 6, wobei die Lage des Urogenitalkrebses in den Nieren, der Harnblase oder der Prostata durch das Vergleichen des Massenspektrums des Sekretoms von aus einer Urinprobe des zu diagnostizierenden Patienten isolierten Zellen in Bezug auf die Massenspektren der Referenzsekretome durchgeführt wird, die separat aus gesunden Nieren-, Harnblasen- und Prostatazellen, isoliert aus einer Urinprobe einer gesunden Person, gewonnen wurden, und dem Bestimmen von mindestens einem Unterschied zwischen dem Massenspektrum des Sekretoms von aus einer Urinprobe des zu diagnostizierenden Patienten isolierten Zellen und mindestens einem der Referenzsekretome aus Nieren, Harnblase oder Prostata.

8.  In-vitro-Verfahren zur Diagnose von Urogenitalkrebs nach einem der Ansprüche 1 bis 7, wobei die in den Sekretionen, die das Sekretom bilden, vorhandenen Bestandteile Proteine, Peptide, Aminosäuren und Biomarker-Nukleinsäuren (DNA, RNA, miRNA und RNAi) sind und insbesondere die Bestandteile, die das Sekretom bilden, die folgenden Proteine umfassen: PSA, PCA3, KLK15, SPINK1, PRSS3, Cathepsin D, Apolipoprotein A-I, PLK2, und die folgenden Biomarker-Nukleinsäure umfassen: miR-141, miR-375, Fusionstranskripte TMPRSS2-ERG, die für SFPR1 und BNC1 codierenden Gene, die für Gammaglutamylhydrolase (GGH) codierenden Gene, den Diazepam binding inhibitor (DBI) und den Transkriptionsfaktor E2F3.

9.  In-vitro-Verfahren zur Diagnose von Urogenitalkrebs nach einem der Ansprüche 1 bis 8, wobei die Bestandteile, die das Sekretom gesunder Prostatazellen bilden, die aus einer Urinprobe einer gesunden Person isoliert wurden, die folgenden Proteine umfassen: PSA, PCA3, KLK15, SPINK1 und PRSS3, und die Fusions-RNA TMPRSS2-ERG und die miRNA miR-141 und miR-375 umfassen.

10. In-vitro-Verfahren zur Diagnose von Urogenitalkrebs nach einem der Ansprüche 1 bis 8, wobei die Bestandteile, die das Sekretom gesunder Nierenzellen bilden, die aus einer Urinprobe einer gesunden Person isoliert wurden, das Protein Cathepsin D umfassen und die folgenden Biomarker-Nukleinsäuren umfassen: die für SFPR1 und BNC1 codierenden Gene.

11. In-vitro-Verfahren zur Diagnose von Urogenitalkrebs nach einem der Ansprüche 1 bis 8, wobei die Bestandteile, die das Sekretom gesunder Harnblasenzellen bilden, die aus einer Urinprobe einer gesunden Person isoliert wurden, die folgenden Proteine umfassen: Apolipoprotein A-I und PLK2, und die folgenden Biomarker-Nukleinsäuren: die für die Gammaglutamylhydrolase (GGH) codierenden Gene, den Diazepam binding inhibitor (DBI) und den Transkriptionsfaktor E2F3.

**Claims**

1.  Method for the *in vitro* diagnosis of a urological cancer comprising the following steps:

    - retrieving the secretions produced by the cells isolated from a urine sample from a patient to be diagnosed,
    - analysing the components of said secretions, referred to as the secretome, produced by said cells isolated from a urine sample from said patient to be diagnosed;
    - comparing a secretome of cells isolated from a urine sample from a patient to be diagnosed with respect, either to a reference secretome obtained from secretions of isolated healthy cells from a urine sample from a healthy subject, or to a reference secretome obtained from secretions of healthy cells derived from standard cell line cultures characteristic of a defined urological organ,

- determining the presence of a urological cancer when at least one difference of a component is determined between said secretome of cells isolated from a urine sample from said patient to be diagnosed and said reference secretome,

said secretome and said reference secretome consisting of the set of components forming the respective secretions thereof.

2. Method for the *in vitro* diagnosis of a urological cancer according to claim 1, wherein determining at least one difference of a component between the secretome of cells isolated from a urine sample from a patient to be diagnosed and the reference secretome, corresponding particularly to

- the presence of a component in a higher or lower concentration,
- the absence of a component,

in the secretome of cells isolated from a urine sample from said patient to be diagnosed, with respect to said component in the reference secretome;

- the presence of a component in the secretome of cells isolated from a urine sample from said patient to be diagnosed, which is absent in the reference secretome.

3. Method for the *in vitro* diagnosis of a urological cancer according to any one of claims 1 to 2, wherein the reference secretome analysed is obtained from healthy cells isolated from a urine sample from a healthy subject, or obtained from standard kidney-, bladder-, prostate-derived cell lines, the diagnostic method further comprising a step of determining the location of said urological cancer in the bladder, kidneys, or prostate, according to whether the at least one difference of a component is observed with respect to a reference secretome obtained for the bladder, kidneys, or prostate.

4. Method for the *in vitro* diagnosis of a urological cancer according to any one of claims 1 to 3, wherein the isolated cells are cultured on a 2D culture medium including a cell layer of confluent epithelial cells topped with a cell layer of confluent fibroblasts.

5. Method for the *in vitro* diagnosis of a urological cancer according to any one of claims 1 to 4, wherein said 2D culture medium has at least one fold so as to form a 3D culture medium, mimicking the structure of at least one compound exocrine gland of acinar/canalicular structure and including said cell layer of epithelial cells topped with said fibroblast cell layer.

6. Method for the *in vitro* diagnosis of a urological cancer according to any one of claims 1 to 5, wherein said analysis of the secretome is carried out by mass spectrometry without prior separation of the components of said secretome, to obtain a specific mass spectrum of the secretome of the cells isolated from the sample, and particularly comprising the comparison of the mass spectrum of a secretome of cells isolated from a urine sample from a patient to be diagnosed with respect to a mass spectrum of a reference secretome obtained from healthy cells isolated from a urine sample from a healthy subject.

7. Method for the *in vitro* diagnosis of a urological cancer according to any one of claims 1 to 6, wherein the location of said urological cancer is performed in the kidneys, bladder or prostate by comparing the mass spectrum of the secretome of cells isolated from a urine sample from the patient to be diagnosed with respect to the mass spectra of reference secretome obtained separately from healthy kidney, bladder and prostate cells, isolated from a urine sample from a healthy subject,
and determining at least one difference between the mass spectrum of the secretome of cells isolated from a urine sample from the patient to be diagnosed and at least one of said reference kidney, bladder or prostate secretomes.

8. Method for the *in vitro* diagnosis of a urological cancer according to any one of claims 1 to 7, wherein the components present in the secretions forming the secretome are proteins, peptides, amino acids, and nucleic biomarkers (DNA, RNA, miRNA, and RNAi) and particularly the components forming the secretome comprise the following proteins: PSA, PCA3, KLK15, SPINK1, PRSS3, cathepsin D, Apolipoprotein A-I, PLK2, and comprise the following nucleic biomarkers: miR-141, miR-375, TMPRSS2-ERG fusion transcripts, the genes coding for SFPR1 and BNC1, the genes coding for Gamma-glutamyl hydrolase (GGH), diazepam binding inhibitor (DBI), and transcription factor E2F3.

9. Method for the *in vitro* diagnosis of a urological cancer according to any one of claims 1 to 8, wherein the components forming the secretome of healthy prostate cells isolated from a urine sample from a healthy subject, comprise the following proteins: PSA, PCA3, KLK15, SPINK1, and PRSS3, and comprise TMPRSS2-ERG fusion RNA and miR-141 and miR-375 miRNA.

**10.** Method for the *in vitro* diagnosis of a urological cancer according to any one of claims 1 to 8, wherein the components forming the secretome of healthy kidney cells isolated from a urine sample from a healthy subject, comprise the protein cathepsin D, and comprise the following nucleic biomarkers: the genes coding for SFPR1 and BNC1.

**11.** Method for the *in vitro* diagnosis of a urological cancer according to any one of claims 1 to 8, wherein the components forming the secretome of healthy bladder cells isolated from a urine sample from a healthy subject, comprise the following proteins: Apolipoprotein A-I and PLK2, and the following nucleic biomarkers: the genes coding for Gamma-glutamyl hydrolase (GGH), diazepam binding inhibitor (DBI), and transcription factor E2F3.

## Figure 1

## Figure 2

## Figure 3

## Figure 4

## Figure 5

## Figure 6

## Figure 7

## Figure 8

## Figure 9

## Figure 10

## Figure 11

## Figure 12

## Figure 13

## Figure 14

## Figure 15

## Figure 16

## Figure 17

## Figure 18

## Figure 19

## Figure 20

## Figure 21

## Figure 22

## Figure 23

## Figure 24

## Figure 25

## Figure 26

## Figure 27

## Figure 28

## Figure 29

## Figure 30

## Figure 31

**Figure 32**

## Figure 33

## Figure 34

# Figure 35

**Figure 36**

## Figure 37

## Figure 38

## Figure 39

## Figure 40

## Figure 41

**107**

205

201

**109**

201

206

**108**

201

201

## Figure 42

202

**107**

**101**

**104**

205

## Figure 43

**101**

201

**103**

203

201

**102**

202

202

201

201

## Figure 44

**104**

204

204

**105**

**106**

207

204

204

## Figure 45

## Figure 46

## Figure 47

## Figure 48

## Figure 49

## Figure 50

## Figure 51

Figure 52

## Figure 53

H

208

209

## Figure 54

22

208

209

## Figure 55

210

211

212

## Figure 56

210

8

5

## Figure 57

210

211

212

G

2

3

1

4

**Figure 58**

**Figure 59**

## Figure 60

## Figure 61

## Figure 62

## Figure 63

## Figure 64

## Figure 65

## Figure 66

## Figure 67

**Figure 68**

**Figure 69**

**Figure 70**

## Figure 71

A

B

G1

F

C

D

## Figure 72

218

g1

4

106

208

218

h1

214

216

215

213

i

217

## Figure 73

## Figure 74

## Figure 75

## Figure 76

**Figure 77**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8647861 B2, Ingber

- US 20140038279 A1, Ingber

**Littérature non-brevet citée dans la description**

- **KIM et al.** Gut-on-a-Chip microenvironment induces human intestinal cells to undergo villus différenciation. *Integr. Biol.,* 2013, vol. 5, 1130
- **MARCH et al.** *Differentiating Intestinal Stem Cells in a 3D Niche,* 2014

- **R. T. BRYAN et al.** Protein shedding in urothelial bladder cancer: prognostic implications of soluble urinary EGFR and EpCAM. *Br J Cancer,* 2015, vol. 112 (6), 1052-1058